(19) Europäisches Patentamt
European Patent Office
Office européen des brevets

(11) EP 1 025 082 B1

(12) **EUROPÄISCHE PATENTSCHRIFT**

(45) Veröffentlichungstag und Bekanntmachung des Hinweises auf die Patenterteilung:
**02.05.2003 Patentblatt 2003/18**

(21) Anmeldenummer: **98954292.3**

(22) Anmeldetag: **29.09.1998**

(51) Int Cl.[7]: **C07C 401/00**, A61K 31/59

(86) Internationale Anmeldenummer:
**PCT/EP98/06159**

(87) Internationale Veröffentlichungsnummer:
**WO 99/016745 (08.04.1999 Gazette 1999/14)**

(54) **NEUE VITAMIN D-DERIVATE MIT CYCLOPROPYLRINGEN IN DEN SEITENKETTEN, VERFAHREN UND ZWISCHENPRODUKTE ZU IHRER HERSTELLUNG SOWIE IHRE VERWENDUNG ZUR HERSTELLUNG VON ARZNEIMITTELN**

NOVEL VITAMIN D DERIVATIVES WITH CYCLOPROPYL RINGS IN THE LATERAL CHAINS, A METHOD AND INTERMEDIATE PRODUCTS FOR THE PRODUCTION THEREOF AND THE UTILIZATION THEREOF FOR PRODUCING MEDICAMENTS

NOUVEAUX DERIVES DE VITAMINE D COMPRENANT DES COMPOSES CYCLIQUES CYCLOPROPYLE DANS LES CHAINES LATERALES, PROCEDE ET PRODUITS INTERMEDIAIRES PERMETTANT DE LES PREPARER, ET LEUR UTILISATION POUR PREPARER DES MEDICAMENTS

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **01.10.1997 DE 19744127**

(43) Veröffentlichungstag der Anmeldung:
**09.08.2000 Patentblatt 2000/32**

(73) Patentinhaber: **SCHERING AKTIENGESELLSCHAFT**
**13342 Berlin (DE)**

(72) Erfinder:
- **STEINMEYER, Andreas**
**D-13581 Berlin (DE)**
- **NEEF, Günter**
**D-10711 Berlin (DE)**
- **KIRSCH, Gerald**
**D-14199 Berlin (DE)**
- **SCHWARZ, Katica**
**D-10585 Berlin (DE)**
- **WIESINGER, Herbert**
**D-10781 Berlin (DE)**
- **HABEREY, Martin**
**D-12169 Berlin (DE)**
- **FÄHNRICH, Marianne**
**D-13409 Berlin (DE)**
- **LANGER, Gernot**
**D-10551 Berlin (DE)**

(56) Entgegenhaltungen:
**WO-A-87/00834**
**WO-A-89/10351**
**WO-A-97/00242**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Vitamin D-Derivate der allgemeinen Formel **I**

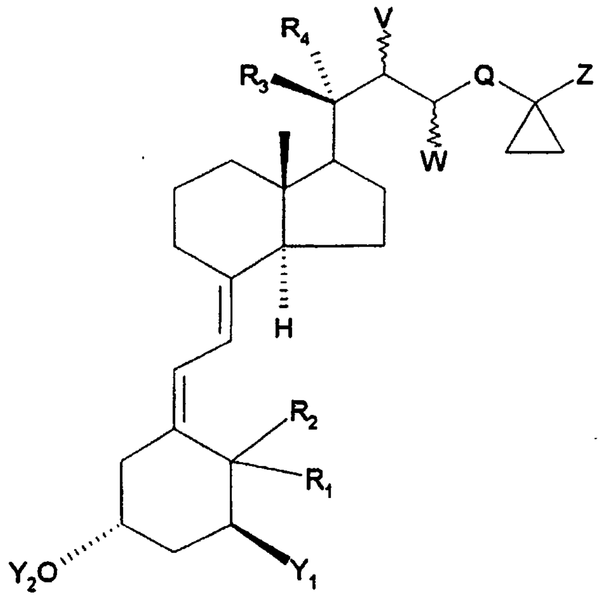

(I)

Verfahren zur ihrer Herstellung, Zwischenprodukte des Verfahrens sowie die Verwendung zur Herstellung von Arzneimitteln.

## Stand der Technik

Die natürlichen Vitamine $D_2$ und $D_3$ sind an sich biologisch inaktiv und werden erst nach Hydroxylierung am C-Atom 25 in der Leber und am C-Atom 1 in der Niere in biologisch aktive Metaboliten [1α,25-Dihydroxyvitamin $D_3$ (Calcitriol) bzw. -$D_2$] umgewandelt. Die Wirkung der aktiven Metaboliten besteht in der Regulation der Calcium- und Phosphatkonzentration im Serum; sie wirken einem Absinken der Calciumkonzentration im Serum entgegen, indem sie die Calciumabsorption im Darm erhöhen und unter bestimmten Umständen die Calciummobilisation aus dem Knochen fördern. Figur 1 zeigt bekannte Vitamin D-Derivate zusammen mit dem gebräuchlichen Nummerierungschema.

Neben ihrer ausgeprägten Wirkung auf den Calcium- und Phosphatstoffwechsel besitzen die aktiven Metaboliten von Vitamin $D_2$ und $D_3$ und seine synthetischen Abkömmlinge eine proliferationshemmende und differenzierungsstimulierende Wirkung auf Tumorzellen und normale Zellen, wie zum Bespiel Hautzellen. Weiterhin wurde eine ausgeprägte Wirkung auf Zellen des Immunsystems (Hemmung der Proliferation und Interleukin 2-Synthese von Lymphocyten, Steigerung der Cytotoxizität und Phagocytose in vitro von Monocyten) gefunden, die sich in einer immunmodulatorischen Wirkung äußert, schließlich wird infolge einer fördernden Wirkung auf knochenbildende Zellen eine vermehrte Knochenbildung bei normalen und osteoporotischen Ratten gefunden [R. Bouillon et al. "Short term course of $1,25(OH)_2D_3$ stimulates osteoblasts but not osteoclasts", *Calc. Tissue Int.* **49**, 168 (1991)].

Alle Wirkungen werden durch Bindung an den Vitamin D-Rezeptor vermittelt. Infolge der Bindung wird die Aktivität von spezifischen Genen reguliert.

Bei Anwendung der biologisch aktiven Metaboliten von Vitamin $D_2$ und $D_3$ wird eine toxische Wirkung auf den Calciumstoffwechsel hervorgerufen (Hypercalcämie).

Durch strukturelle Manipulationen der Seitenkette können therapeutisch nutzbare Wirkqualitäten von der unerwünschten hypercalcämischen Aktivität abgetrennt werden. Eine geeignete Strukturvariante ist die Einführung von 24-Hydroxy-Derivaten.

In 24-Stellung hydroxylierte 1α-Cholecalciferole gehen bereits aus der DE 25 26 981 hervor. Sie besitzen eine geringere Toxizität als das entsprechende nicht-hydroxylierte 1α-Cholecalciferol. Darüberhinaus sind 24-Hydroxy-Derivate in folgenden Patentanmeldungen beschrieben: DE 39 33 034, DE 40 03 854, DE 40 34 730, EP 0 421 561, EP 0 441 467, WO 87/00834, WO 91/12238.

Schließlich werden in der WO 94/07853 an C-24 hydroxylierte 25-Carbonsäure-Derivate von Calcitriol beschrieben, die ein günstigeres Wirkspektrum als Calcitriol aufweisen. Entsprechendes gilt auch für neue Vitamin D-Derivate mit Substituenten an C-25 (WO 97/00242). Während die Fähigkeit zur Auslösung einer Hypercalcämie deutlich

abgeschwächt ist, bleiben die proliferationshemmenden und differenzierungsstimuliernden Wirkungen erhalten. In der Regel führt die Einführung der 24-Hydroxylgruppe jedoch zur metabolischen Destabilisierung der Derivate, insbesondere wenn sich in der Nachbarstellung ein Cyclopropylring befindet. Aus diesem Grunde sind diese Verbindungen nur bedingt zur systemischen Applikation geeignet.

Es besteht daher Bedarf an neuen Vitamin D-Derivaten, die ein ähnlich günstiges Wirkspektrum, wie die im Stand der Technik beschriebenen Verbindungen (insbesondere WO 94/07853 und WO 97/00242) aufweisen, aber durch eine höhere metabolische Stabilität besser zur systemischen Applikation geeignet sind.
Der vorliegenden Patentanmeldung liegt daher die Aufgabe zugrunde, derartige Vitamin D-Derivate zur Verfügung zu stellen. Die Lösung dieser Aufgabe erfolgt durch die in den Patentansprüchen offenbarten Verbindungen.

Die vorliegende Erfindung betrifft daher Vitamin D-Derivate der allgemeinen Formel I,

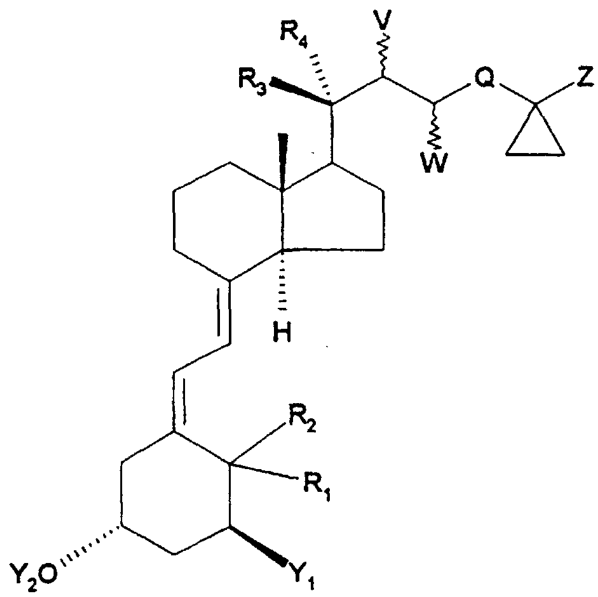

I

worin

| | |
|---|---|
| $Y_1$ | ein Wasserstoffatom, eine Hydroxylgruppe, ein Fluor-, Chloroder Bromatom oder eine Gruppe -O(CO)$R_8$, worin |
| | $R_8$ ein aliphatischer oder aromatischer Rest mit 1 bis 12 C-Atomen ist, |
| $Y_2$ | ein Wasserstoffatom oder eine Gruppe -(CO)$R_9$, worin |
| | $R_9$ ein aliphatischer oder aromatischer Rest mit 1 bis 12 C-Atomen ist, |
| $R_1$ und $R_2$ | je ein Wasserstoffatom oder gemeinsam eine exocyclische Methylengruppe, |
| $R_3$ und $R_4$ | unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring, |
| V und W | zusammen eine *E*-Doppelbindung oder V eine Hydroxylgruppe und W ein Wasserstoffatom, |
| Q | eine Gruppe ausgewählt aus |

$-CH_2-$, $-(CH_2)_2-$, $-(CH_2)_3-$, $-(CH_2)_4-$, $-(CH_2)_7$, $-CH_2-C(CH_3)_2-CH_2-$,
$-CH_2-CH(CH_3)-CH_2-CH(CH_3)-CH_2-$,
$-CH_2-CH(OH)-$, $-CH_2-CH_2-CH(OH)-$, $-CH(OH)-CH_2-$,
$-CH(OH)-CH_2-CH_2-$, $-CH_2-CH(OH)-CH_2-CH(OH)-CH_2-$,

$-CH_2-CH(OH)-CH_2-$, $-CH_2-CH(OC_2H_5)-$, $-CH_2-CH(OCOCH_3)-CH_2-CH(OCOCH_3)-CH_2-$, $-CH_2-CH(OCOC_4H_9)-CH_2-$, -CO-, $-CO-CH_2$, $-CO-CH_2-CH_2-$, $-CH_2COCH_2-$, -CH(Cl)-, $-CH(Cl)-CH_2-$, $-CH_2-CH(Cl)-$, $-CH(NH_2)-$, $-CH(NH_2)-CH_2-$, $-CH(N(CH_3)_2)-$, $-CH(N(CH_3)_2)-CH_2-$, $-CH_2-CH(N(CH_3)_2)-CH_2-CH(N(CH_3)_2)-CH_2-$, -CH(F)-, $-CH(F)-CH_2-$, $-CH_2-CH(F)-CH_2$, wobei die Hydroxysubstituenten in α- oder in β-Position stehen können,

Z einen gerad- oder verzweigtkettigen, gesättigten oder ungesättigten Kohlenwasserstoffrest mit bis zu 12 Kohlenstoffatomen, der an beliebigen Positionen Ketogruppen, α- oder β-Hydroxylgruppen, welche ihrerseits verethert oder verestert sein können, der Aminogruppen, Fluor-, Chlor-, Bromatome aufweisen kann,

bedeuten.

Die Erfindung betrifft ferner Verfahren zur Herstellung der erfindungsgemäßen Verbindungen, Zwischenprodukte des Herstellungsverfahrens sowie die Verwendung der erfindungsgemäßen Verbindungen zur Herstellung von Arzneimitteln.

Besonders vorteilhafte Ausführungsformen der Erfindung sind Gegenstand der Unteransprüche.

Bevorzugt stehen die Reste $R_1$ und $R_2$ für Wasserstoffatome.

Für $R_3$ und $R_4$ gelten die folgenden bevorzugten Kombinationen: $R_3$= H, $R_4$= Methyl oder $R_3$= Methyl, $R_4$=H; $R_3$= F, $R_4$= Methyl oder $R_3$= Methyl, $R_4$=F; $R_3$, $R_4$= Methyl; $R_3$ und $R_4$ bilden zusammen eine Methylengruppe oder gemeinsam mit dem tertiären Kohlenstoffatom 20 einen Cyclopropylring.

Die optionalen Reste $R_8$ und $R_9$ sind organische Gruppen mit 1 bis 12 C-Atomen. Diese Reste können gesättigt oder ungesättigt, verzweigt oder unverzweigt, acyclisch, carbocyclisch oder heterocyclisch sein. Beispiele für die Reste $R_8$ und $R_9$ sind Methyl-, Ethyl-, Propyl-, i-Propyl-, Butyl- oder Phenylgruppen. Möglich sind aber auch die Reste natürlich vorkommender Aminosäuren, wie z.B. $-CH_2-CH(CH_3)_2$, $-CH_2-Ph$, $-CH_2OH$, $-CH(OH)-CH_3$, $-CH_2SH$, $-CH_2-SCH_3$, $-CH_2CO_2H$, $-CH_2CH_2-CO_2H$, $-(CH_2)_4-NH_2$, $-(CH_2)_3-C(NH)NH_2$, aber auch die Reste der Aminosäuren Tryptophan, Tyrosin oder Histamin.

Die bevorzugten Reste leiten sich von $C_1$- bis $C_9$-, insbesondere $C_2$- bis $C_5$-Alkancarbonsäuren wie beispielsweise Essigsäure, Propionsäure, Buttersäure oder Pivaloylsäure ab. Unter den aromatischen Gruppen sind die Phenylgruppe und substituierte Phenylgruppen bevorzugt.

Q ist eine eine geradkettige oder verzweigte Kohlenstoffeinheit mit bis zu 10 Kohlenstoffatomen, z.B. $-CH_2-$, $-(CH_2)_2-$, $-(CH_2)_3-$, $-(CH_2)_4-$, $-(CH_2)_7-$, $-CH_2-C(CH_3)_2-CH_2-$, $-CH_2-CH(CH_3)-CH_2-CH(CH_3)-CH_2-$. Die in Q enthaltenen Kohlenstoffatome können mit Ausnahme von $-CH_2-$ an beliebigen Positionen Hydroxylgruppen aufweisen, z.B. $-CH_2-CH(OH)-$, $-CH_2-CH_2-CH(OH)-$, $-CH(OH)-CH_2-$, $-CH(OH)-CH_2-CH_2-$, $-CH_2-CH(OH)-CH_2-CH(OH)-CH_2-$, $-CH_2-CH(OH)-CH_2-$, $-CH_2-CH(OH)-CH_2-CH(OH)-CH_2-$. Die Hydroxylgruppen können ihrerseits verestert oder verethert sein, z.B. $-CH(OCH_3)-$, $-CH_2-CH(OC_2H_5)-$, $-CH_2-CH(OCOCH_3)-CH_2-CH(OCOCH_3)-CH_2-$, $-CH_2-CH(OCOC_4H_9)-CH_2-$. Die Gruppe Q kann auch Ketogruppen, Aminogruppen oder Halogenatome aufweisen, z.B. -CO-, $-CO-CH_2$, $-CO-CH_2-CH_2-$, $-CH_2COCH_2-$, -CH(Cl)-, $-CH(Cl)-CH_2-$, $-CH_2-CH(Cl)-$, $-CH(NH_2)-$, $-CH(NH_2)-CH_2-$, $-CH(N(CH_3)_2)-$, $-CH(N(CH_3)_2)-CH_2-$, $-CH_2-CH(N(CH_3)_2)-CH_2-CH(N(CH_3)_2)-CH_2-$, -CH(F)-, $-CH(F)-CH_2-$, $-CH_2-CH(F)-CH_2$.

Folgende Gruppen Q sind bevorzugt:

Q ist eine unsubstituierte, unverzweigte Alkyleneinheit mit 1, 2 oder 3 Kohlenstoffatomen oder

Q = $-CH(OH)-CH_2-$ oder $-CH(OH)-CH_2-CH_2-$ (α- oder β-ständige Hydroxylgruppen).

Der Rest Y1 steht bevorzugt für ein Wasserstoff-, ein Fluoratom oder eine Hydroxylgruppe.

Z ist ein gerad- oder verzweigtkettiger, gesättigter oder ungesättigter Kohlenwasserstoffrest mit bis zu 12 Kohlenstoffatomen, z.B. $-CH_3$, $-CH_2-CH_3$, $-(CH_2)_2-CH_3$, $-(CH_2)_3-CH_3$, $-(CH_2)_4-CH_3$, $-(CH_2)_5-CH_3$, $-(CH_2)_6-CH_3$, $-(CH_2)_7-CH_3$, $-CH_2-C(CH_3)_2-CH_2-CH_3$, $-CH_2-CH(CH_3)-CH_2-CH(CH_3)-CH_2-CH_3$. Die in Z enthaltenen Kohlenstoffatome können an beliebigen Positionen Hydroxylgruppen aulweisen, z.B. $-CH(OH)-CH_3$, $-CH_2-CH(OH)-CH_3$, $-CH_2-CH(OH)-CH_2-CH(OH)-CH_2-CH_3$, $-CH_2-CH(OH)-CH_2-CH_3$, $-CH_2-CH(OH)-CH_2-CH(OH)-CH_2-CH_3$. Die Hydroxylgruppen können ihrerseits verestert oder verethert sein, z.B. $-CH(OCH_3)-CH_3$, $-CH_2-CH(OC_2H_5)-CH_3$, $-CH_2-CH(OCOCH_3)$ $-CH_2-CH(OCOCH_3)-CH_2-CH_3$, $-CH_2-CH(OCOC_4H_9)-CH_2-CH_3$. Die Gruppe Z kann auch Ketogruppen, Aminogruppen oder Halogenatome aufweisen, z.B. $-CH_2COCH_2-CH_3$, $-CH_2-CH(Cl)-CH_3$, $-CH_2-CH(N(CH_3)_2)-CH_2-CH(N(CH_3)_2)$ $-CH_2-CH_3$, $-CH_2-CH(F)-CH_2-CH_3$.

Folgende Gruppen Z sind bevorzugt:

Z ist eine 1-Oxoalkylgruppe mit 1-12-Kohlenstoffatomen,
Z ist eine Alkylgruppe mit 1-12 Kohlenstoffatomen,
Z ist eine Alkenylgruppe mit 1-12 Kohlenstoffatomen, bei der die Doppelbindung *E*- oder *Z*-Geometrie besitzen kann und an beliebigen Positionen der Seitenkette vorliegen kann.

Die Gruppen V und W bilden entweder zusammen eine *E*-Doppelbindung oder V ist eine Hydroxylgruppe und W ein Wasserstoffatom. Die beiden Möglichkeiten für das betreffende Strukturelement sind im Folgenden abgebildet:

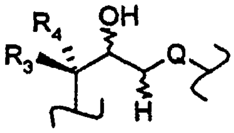

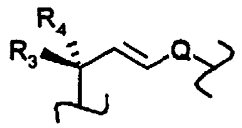

Von den erfindungsgemäßen Verbindungen der allgemeinen Formel I sind die folgenden Verbindungen ganz besonders bevorzugt:

(5*Z*,7*E*,22*E*)-(1*S*,3*R*)-25-Acetyl-26,27-cyclo-9,10-secocholesta-5,7,10(19),22-tetraen-1,3-diol,
(5*Z*,7*E*,22*E*)-(1*S*,3*R*)-25-(1-Oxopropyl)-26,27-cyclo-9,10-secocholesta-5,7,10(19),22-tetraen-1,3-diol,
(5*Z*,7*E*,22*E*)-(1*S*,3*R*)-25-(1-Oxobutyl)-26,27-cyclo-9,10-secocholesta-5,7,10(19),22-tetraen-1,3-diol,
(5*Z*,7*E*,22*E*)-(1*S*,3*R*)-25-(1-Oxopentyl)-26,27-cyclo-9,10-secocholesta-5,7,10(19),22-tetraen-1,3-diol,
(5*Z*,7*E*,22*E*)-(1*S*,3*R*)-25-(1-Oxohexyl)-26,27-cyclo-9,10-secocholesta-5,7,10(19),22-tetraen-1,3-diol,
(5*Z*,7*E*,22*E*)-(1*S*,3*R*)-25-(1-Oxoheptyl)-26,27-cyclo-9,10-secocholesta-5,7,10(19),22-tetraen-1,3-diol,
(5*Z*,7*E*,22*E*)-(1*S*,3*R*)-25-(1-Oxooctyl)-26,27-cyclo-9,10-secocholesta-5,7,10(19),22-tetraen-1,3-diol,
(5*Z*,7*E*,22*E*)-(1*S*,3*R*)-25-(1-Oxononyl)-26,27-cyclo-9,10-secocholesta-5,7,10(19),22-tetraen-1,3-diol,
(5*Z*,7*E*,22*E*)-(1*S*,3*R*)-25-( 1 -Oxodecyl)-26,27-cyclo-9,10-secocholesta-5,7,10(19),22-tetraen-1,3-diol,
(5*Z*,7*E*)-(1*S*,3*R*,22*S*)-25-Acetyl-26,27-cyclo-9,10-secocholesta-5,7,10(19)-trien-1,3,22-triol,
(5*Z*,7*E*)-(1*S*,3*R*,22*R*)-25-Acetyl-26,27-cyclo-9,10-secocholesta-5,7,10(19)-trien-1,3,22-triol,
(5*Z*,7*E*)-(1*S*,3*R*,22*S*)-25-(1-Oxopropyl)-26,27-cyclo-9,10-secocholesta-5, 7,10(19)-trien-1,3,22-triol,
(5*Z*,7*E*)-(1*S*,3*R*,22*R*)-25-(1-Oxopropyl)-26,27-cyclo-9,10-secocholesta-5,7,10(19)-trien-1,3,22-triol,
(5*Z*,7*E*)-(1*S*,3*R*,22*S*)-25-(1-Oxobutyl)-26,27-cyclo-9,10-secocholesta-5,7,10(19)-trien-1,3,22-triol,
(5*Z*,7*E*)-(1*S*,3*R*,22*R*)-25-(1-Oxobutyl)-26,27-cyclo-9,10-secocholesta-5,7,10(19)-trien-1,3,22-triol,
(5*Z*,7*E*)-(1*S*,3*R*,22*S*)-25-(1-Oxopentyl)-26,27-cyclo-9,10-secocholesta-5,7,10(19)-trien-1,3,22-triol,
(5*Z*,7*E*)-(1*S*,3*R*,22*R*)-25-(1-Oxopentyl)-26,27-cyclo-9,10-secocholesta-5,7,10(19)-trien-1,3,22-triol
(5*Z*,7*E*)-(1*S*,3*R*,22*S*)-25-(1-Oxohexyl)-26,27-cyclo-9,10-secocholesta-5,7,10(19)-trien-1,3,22-triol
(5*Z*,7*E*)-(1*S*,3*R*,22*R*)-25-(1-Oxohexyl)-26,27-cyclo-9,10-secocholesta-5,7,10(19)-trien-1,3,22-triol
(5*Z*,7*E*)-(1*S*,3*R*,22*S*)-25-(1-Oxoheptyl)-26,27-cyclo-9,10-secocholesta-5,7,10(19)-trien-1,3,22-triol
(5*Z*,7*E*)-(1*S*,3*R*,22*R*)-25-(1-Oxoheptyl)-26,27-cyclo-9,10-secocholesta-5,7,10(19)-trien-1,3,22-triol
(5*Z*,7*E*)-(1*S*,3*R*,22*S*)-25-(1-Oxooctyl)-26,27-cyclo-9,10-secocholesta-5,7,10(19)-trien-1,3,22-triol
(5*Z*,7*E*)-(1*S*,3*R*,22*R*)-25-(1-Oxooctyl)-26,27-cyclo-9,10-secocholesta-5,7,10(19)-trien-1,3,22-triol
(5*Z*,7*E*)-(1*S*,3*R*,22*S*)-25-(1-Oxononyl)-26,27-cyclo-9,10-secocholesta-5,7,10(19)-trien-1,3,22-triol
(5*Z*,7*E*)-(1*S*,3*R*,22*R*)-25-(1-Oxononyl)-26,27-cyclo-9,10-secocholesta-5,7,10(19)-trien-1,3,22-triol
(5*Z*,7*E*)-(1*S*,3*R*,22*S*)-25-(1-Oxodecyl)-26,27-cyclo-9,10-secocholesta-5,7,10(19)-trien-1,3,22-triol
(5*Z*,7*E*)-(1*S*,3*R*,22*R*)-25-(1-Oxodecyl)-26,27-cyclo-9,10-secocholesta-5,7,10(19)-trien-1,3,22-triol
(5*Z*,7*E*,22*E*)-(1*S*,3*R*)-25-Acetyl-26,27-cyclo-24a-homo-9,10-secocholesta-5,7,10(19),22-tetraen-1,3-diol,
(5*Z*,7*E*,22*E*)-(1*S*,3*R*)-25-(1-Oxopropyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxobutyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxopentyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxohexyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxoheptyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxooctyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxononyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxodecyl)-26,27-cyclo-24a-homo-9,10-secocholesta-5,7,10(19),22-tetraen-1,3-diol,
(5*Z*,7*E*)-(1*S*,3*R*,22*S*)-25-Acetyl-26,27-cyclo-24a-homo-9,10-secocholesta-5,7,10(19)-trien-1,3,22-triol,
(5*Z*,7*E*)-(1*S*,3*R*,22*R*)-25-Acetyl-26,27-cyclo-24a-homo-9,10-secocholesta-5,7,10(19)-trien-1,3,22-triol,
(5*Z*,7*E*)-(1*S*,3*R*,22*S*)-25-(1-Oxopropyl)-26,27-cyclo-24a-homo-9,10-secocholesta-5,7,10(19)-trien-1,3,22-triol,
(5*Z*,7*E*)-(1*S*,3*R*,22*R*)-25-(1-Oxopropyl)-26,27-cyclo-24a-homo-9,10-secocholesta-5,7,10(19)-trien-1,3,22-triol,

(5*Z*,7*E*)-(1*S*,3*R*,22*S*)-25-(1-Oxobutyl)-26,27-cyclo-24a-homo-9,10-secocholesta-5,7,10(19)-trien-1,3,22-triol,
(5*Z*,7*E*)-(1*S*,3*R*,22*R*)-25-(1-Oxobutyl)-26,27-cyclo-24a-homo-9,10-secocholesta-5,7,10(19)-trien-1,3,22-triol,
(5*Z*,7*E*)-(1*S*,3*R*,22*S*)-25-(1-Oxopentyl)-26,27-cyclo-24a-homo-9,10-secocholesta-5,7,10(19)-trien-1,3,22-triol,
(5*Z*,7*E*)-(1*S*,3*R*,22*R*)-25-(1-Oxopentyl)-26,27-cyclo-24a-homo-9,10-secocholesta-5,7,10(19)-trien-1,3,22-triol,
(5*Z*,7*E*)-(1*S*,3*R*,22*S*)-25-(1-Oxohexyl)-26,27-cyclo-24a-homo-9,10-secocholesta-5,7,10(19)-trien-1,3,22-triol,
(5*Z*,7*E*)-(1*S*,3*R*,22*R*)-25-(1-Oxohexyl)-26,27-cyclo-24a-homo-9,10-secocholesta-5,7,10(19)-trien-1,3,22-triol,
(5*Z*,7*E*)-(1*S*,3*R*,22*S*)-25-(1-Oxoheptyl)-26,27-cyclo-24a-homo-9,10-secocholesta-5,7,10(19)-trien-1,3,22-triol,
(5*Z*,7*E*)-(1*S*,3*R*,22*R*)-25-(1-Oxoheptyl)-26,27-cyclo-24a-homo-9,10-secocholesta-5,7,10(19)-trien-1,3,22-triol,
(5*Z*,7*E*)-(1*S*,3*R*,22*S*)-25-(1-Oxooctyl)-26,27-cyclo-24a-homo-9,10-secocholesta-5,7,10(19)-trien-1,3,22-triol,
(5*Z*,7*E*)-(1*S*,3*R*,22*R*)-25-(1-Oxooctyl)-26,27-cyclo-24a-homo-9,10-secocholesta-5,7,10(19)-trien-1,3,22-triol,
(5*Z*,7*E*)-(1*S*,3*R*,22*S*)-25-(1-Oxononyl)-26,27-cyclo-24a-homo-9,10-secocholesta-5,7,10(19)-trien-1,3,22-triol,
(5*Z*,7*E*)-(1*S*,3*R*,22*R*)-25-(1-Oxononyl)-26,27-cyclo-24a-homo-9,10-secocholesta-5,7,10(19)-trien-1,3,22-triol,
(5*Z*,7*E*)-(1*S*,3*R*,22*S*)-25-(1-Oxodecyl)-26,27-cyclo-24a-homo-9,10-secocholesta-5,7,10(19)-trien-1,3,22-triol,
(5*Z*,7*E*)-(1*S*,3*R*,22*R*)-25-(1-Oxodecyl)-26,27-cyclo-24a-homo-9,10-secocholesta-5,7,10(19)-trien-1,3,22-triol,
(5*Z*,7*E*,22*E*)-(1*S*,3*R*,24*R*)-25-Acetyl-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-Acetyl-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxopropyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxopropyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxobutyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxobutyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxopentyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxopentyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxohexyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxohexyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxoheptyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxoheptyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxooctyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxooctyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxononyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxononyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxodecyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-(1-Oxodecyl)-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-Acetyl-24-methoxy-26,27-cyclo-24a,24b-dihomo-9,10-secocholesta-5,7,10(19),22-tetraen-1,3-diol,
(5*Z*,7*E*,22*E*)-(1*S*,3*R*,24*S*)-25-Acetyl-24-methoxy-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxopropyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxopropyl)-26,27-cyclo-24a,24bdihomo-9,10-secocholesta-5,7,10(19),22-tetraen-1,3-diol,

(5*Z*,7*E*,22*E*)-(1*S*,3*R*,24*R*)-24-Methoxy-25-(1-oxobutyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxobutyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxopentyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxopentyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxohexyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxohexyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxoheptyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxoheptyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxooctyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxooctyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxononyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxononyl)-26,27-cyclo-24a,24bdihomo-9,10-secocholesta-5,7,10(19),22-tetraen-1,3-diol,
(5*Z*,7*E*,22*E*)-(1*S*,3*R*,24*R*)-24-Methoxy-25-(1-oxodecyl)-26,27-cyclo-24a,24bdihomo-9,10-secocholesta-5,7,10(19),22-tetraen-1,3-diol,
(5*Z*,7*E*,22*E*)-(1*S*,3*R*,24*S*)-24-Methoxy-25-(1-oxodecyl)-26,27-cyclo-24a,24bdihomo-9,10-secocholesta-5,7,10(19),22-tetraen-1,3-diol,

außerdem sind die folgenden Verbindungen Gegenstand der Erfindung:

(5*Z*,7*E*,22*E*)-(1*S*,3*R*,24*S*)-25-Methyl-26,27-cyclo-9,10-secocholesta-5,7,10(19),22-tetraen-1,3,24-triol,
(5*Z*,7*E*,22*E*)-(1*S*,3*R*,24*R*)-25-Methyl-26,27-cyclo-9,10-secocholesta-5,7,10(19),22-tetraen-1,3,24-triol,
(5*Z*,7*E*,22*E*)-(1*S*,3*R*,24*S*)-25-Ethyl-26,27-cyclo-9,10-secocholesta-5,7,10(19),22-tetraen-1,3,24-triol,
(5*Z*,7*E*,22*E*)-(1*S*,3*R*,24*R*)-25-Ethyl-26,27-cyclo-9,10-secocholesta-5,7,10(19),22-tetraen-1,3,24-triol,
(5*Z*,7*E*,22*E*)-(1*S*,3*R*,24*S*)-25-Propyl-26,27-cyclo-9,10-secocholesta-5,7,10(19),22-tetraen-1,3,24-triol,
(5*Z*,7*E*,22*E*)-(1*S*,3*R*,24*R*)-25-Propyl-26,27-cyclo-9,10-secocholesta-5,7,10(19),22-tetraen-1,3,24-triol,
(5*Z*,7*E*,22*E*)-(1*S*,3*R*,24*S*)-25-Butyl-26,27-cyclo-9,10-secocholesta-5,7,10(19),22-tetraen-1,3,24-triol,
(5*Z*,7*E*,22*E*)-(1*S*,3*R*,24*R*)-25-Butyl-26,27-cyclo-9,10-secocholesta-5,7,10(19),22-tetraen-1,3,24-triol,
(5*Z*,7*E*,22*E*)-(1*S*,3*R*,24*S*)-25-Pentyl-26,27-cyclo-9,10-secocholesta-5,7,10(19),22-tetraen-1,3,24-triol,
(5*Z*,7*E*,22*E*)-(1*S*,3*R*,24*R*)-25-Pentyl-26,27-cyclo-9,10-secocholesta-5,7,10(19),22-tetraen-1,3,24-triol,
(5*Z*,7*E*,22*E*)-(1*S*,3*R*,24*S*)-25-Hexyl-26,27-cyclo-9,10-secocholesta-5,7,10(19),22-tetraen-1,3,24-triol,
(5*Z*,7*E*,22*E*)-(1*S*,3*R*,24*R*)-25-Hexyl-26,27-cyclo-9,10-secocholesta-5,7,10(19),22-tetraen-1,3,24-triol,
(5*Z*,7*E*,22*E*)-(1*S*,3*R*,24*S*)-25-Heptyl-26,27-cyclo-9,10-secocholesta-5,7,10(19),22-tetraen-1,3,24-triol,
(5*Z*,7*E*,22*E*)-(1*S*,3*R*,24*R*)-25-Heptyl-26,27-cyclo-9,10-secocholesta-5,7,10(19),22-tetraen-1,3,24-triol,
(5*Z*,7*E*,22*E*)-(1*S*,3*R*,24*S*)-25-Octyl-26,27-cyclo-9,10-secocholesta-5,7,10(19),22-tetraen-1,3,24-triol,
(5*Z*,7*E*,22*E*)-(1*S*,3*R*,24*R*)-25-Octyl-26,27-cyclo-9,10-secocholesta-5,7,10(19),22-tetraen-1,3,24-triol,
(5*Z*,7*E*,22*E*)-(1*S*,3*R*,24*S*)-25-Nonyl-26,27-cyclo-9,10-secocholesta-5,7,10(19),22-tetraen-1,3,24-triol,
(5*Z*,7*E*,22*E*)-(1*S*,3*R*,24*R*)-25-Nonyl-26,27-cyclo-9,10-secocholesta-5,7,10(19),22-tetraen-1,3,24-triol,
(5*Z*,7*E*,22*E*)-(1*S*,3*R*,24*S*)-25-Decyl-26,27-cyclo-9,10-secocholesta-5,7,10(19),22-tetraen-1,3,24-triol,
(5*Z*,7*E*,22*E*)-(1*S*,3*R*,24*R*)-25-Decyl-26,27-cyclo-9,10-secocholesta-5,7,10(19),22-tetraen-1,3,24-triol,
(5*Z*,7*E*,22*E*)-(1*S*,3*R*,24*S*)-25-Ethylen-26,27-cyclo-9,10-secocholesta-5,7,10(19),22-tetraen-1,3,24-triol,
(5*Z*,7*E*,22*E*)-(1*S*,3*R*,24*R*)-25-Ethylen-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Propenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Propenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Butenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Butenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Pentenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Pentenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Hexenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Hexenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1 -Heptenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Heptenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Octenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Octenyl)-26,27-cyclo-9,10-secocholesta-5,7,10(19),22-tetraen-1,3,24-triol,
[5*Z*,7*E*,22*E*,25(*E*)]-(1*S*,3*R*,24*S*)-25-(1-Nonenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Nonenyl)-26,27-cyclo-9,10-secocholesta-5,7,10(19),22-tetraen-1,3,24-triol,
[5*Z*,7*E*,22*E*,25(*E*)]-(1 *S*,3*R*,24*S*)-25-(1-Decenyl)-26,27-cyclo-9,10-secocholesta-5,7,10(19),22-tetraen-1,3,24-triol,
[5*Z*,7*E*,22*E*,25(*E*)]-(1*S*,3*R*,24*R*)-25-(1-Decenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Propenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1-Propenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Butenyl)-26,27-cyclo-9,10-secocholesta-5,7,10(19),22-tetraen-1,3,24-triol,
[5*Z*,7*E*,22*E*,25(Z)]-(1*S*,3*R*,24*R*)-25-(1-Butenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Pentenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1-Pentenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Hexenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1 -Hexenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Heptenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1-Heptenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Octenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1-Octenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Nonenyl)-26,27-cycl-9,10-secocholesta-5,7,10(19),22-tetraen-1,3,24-triol,
[5*Z*,7*E*,22*E*,25(*Z*)]-(1*S*,3*R*,24*R*)-25-(1-Nonenyl)-26,27-cyclo-9,10-secocholesta-5,7,10(19),22-tetraen-1,3,24-triol,
[5*Z*,7*E*,22*E*,25(*Z*)]-(1*S*,3*R*,24*S*)-25-(1-Decenyl)-26,27-cyclo-9,10-secocholesta-5,7,10(19),22-tetraen-1,3,24-triol,
[5*Z*,7*E*,22*E*,25(*Z*)]-(1*S*,3*R*,24*R*)-25-(1-Decenyl)-26,27-cyclo-9,10-secocholesta-5,7,10(19),22-tetraen-1,3,24-triol.

Die erfindungsgemäßen Substanzen besitzen eine deutlich höhere metabolische Stabilität als die strukturell

verwandten Verbindungen des Standes der Technik und eignen sich daher in besonderer Weise für systemische Applikationen.

Gegenüber den strukturell verwandten Verbindungen des Standes der Technik zeichnen sich einige der erfindungsgemäßen Substanzen ferner dadurch aus, daß sie eine stärkere Wirkung auf die Zelldifferenzierung zeigen, wobei die Wirkung auf den Calciumhaushalt nicht zunimmt. Andere der erfindungsgemäßen Substanzen dagegen weisen ein antagonistisches oder partialagonistisches Wirkprofil auf, das neue Anwendungen ermöglicht.

**Bestimmung der biologischen Aktivität**

Die Vitamin D-Aktivität der erfindungsgemäßen Substanzen wird mittels des Calcitriol-Rezeptortests bestimmt. Er wird unter Verwendung eines Proteinextraktes aus dem Darm von jungen Schweinen durchgeführt.

Rezeptorhaltiger Proteinextrakt wird mit $^3$H-Calcitriol ($5 \times 10^{-10}$ mol/l) in einem Reaktionsvolumen von 0,270 ml in Abwesenheit und in Anwesenheit der Prüfsubstanzen für zwei Stunden bei 4°C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4°C für 20 Min. inkubiert. Anschließend werden die Proben bei 10 000 g 5 Minuten bei 4°C zentrifugiert. Der Überstand wird dekantiert und nach 1stündiger Äquilibrierung in Picofluor 15 ™ in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Den erfindungsgemäßen Verbindungen ist gemein, daß sie alle über eine beträchtliche Affinität zum Calcitriol-Rezeptor verfügen.

Zur Bestimmung der akuten hypercalcämischen Wirkung verschiedener Calcitriolderivate wird nachfolgend beschriebener Test durchgeführt:

Die Wirkung von Kontrolle (Lösungsgrundlage), Referenzsubstanz (1,25-Dihydroxyvitamin $D_3$ = Calcitriol) und Testsubstanz wird jeweils nach einmaliger subcutaner Applikation in Gruppen von 10 gesunden männlichen Ratten (140-170g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22 h) gesammelt. Eine orale Calciumgabe (0.1 mM Calcium in 6,5% Alpha-Hydroxypropylcellulose, 5 ml/Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich zur Kontrolle erhöhten Serum-Calciumspiegel-Werten.

Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt.

Es ist literaturbekannt [Mangelsdorf, D.J. et al., *J. Cell. Biol.* **98**, 391 (1984)], daß die Behandlung humaner Leukämiezellen (Promyelozytenzellinie HL 60) in vitro mit Calcitriol die Differenzierung der Zellen zu Makrophagen induziert.

HL 60-Zellen werden in Gewebekulturmedium (RPMI 10% fetales Kälberserum) bei 37°C in einer Atmosphäre 5% $CO_2$ in Luft kultiviert.

Zur Substanztestung werden die Zellen abzentrifugiert und $2{,}0 \times 10^5$ Zellen/ml in phenol-rotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5% $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz $2 \times 10^{-7}$ mol/l) pipettiert.

Durch Inkubation über 2 Stunden bei 37°C und 5% $CO_2$ in Luft wird infolge der intrazel-lulären Sauerstoffradikalfreisetzung, stimuliert durch TPA, in den zu Makrophagen diffe-renzierten Zellen NBT zu unlöslichem Formazan reduziert.

Zur Beendigung der Reaktion werden die Vertiefungen der 96-Loch-Platte abgesaugt und die Zellen durch Zugabe von Methanol am Plattenboden fixiert und nach Fixation getrocknet. Zur Lösung der gebildeten intrazellulären Formazankristalle werden in jede Vertiefung 100 µl Kaliumhydroxid (2 mol/l) und 100 µl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem Formazan. Das Ergebnis wird als Dosisrelation (DR = Faktor TestsubstanzdosislReferenzsubstanzdosis für vergleichbare halbmaximale Wirkungen) angegeben.

Zur Ermittlung der metabolischen Stabilität wird die Prüfsubstanz mit Gewebehomogenat (aus Rattenleber) in Gegenwart von Puffersystemen inkubiert. Zeitabhängig wird der Abfall der Ausgangskonzentration verfolgt. Nach bestimmten Zeiten wird die Inkubation gestoppt und die unveränderte Prüfsubstanz aus dem Homogenat extrahiert (Diethylether), unter Stickstoff eingedampft, über HPLC isoliert (Laufmittel: Acetonitril/Wasser) und mittels UV-Absorption detektiert.

Die Ergebnisse des Calcitriol-Rezeptortests sowie der Bestimmung der Dosisrelation der Differenzierungsinduktion von HL 60-Zellen und der Dosisrelation für Hypercalcämie sowie die Halbwertszeit im Leberhomogenat sind nachfolgend zusammengefaßt:

Beispiele für Testverbindungen:

(5*Z*,7*E*,22*E*)-(1*S*,3*R*)-25-Acetyl-26,27-cyclo-9,10-secocholesta-5,7,10(19),22-tetraen-1,3-diol **66**
(5*Z*,7*E*,22*E*)-(1*S*,3*R*)-25-(1-Oxobutyl)-26,27-cyclo-9,10-secocholesta-5,7,10(19),22-tetraen-1,3-diol **71**
(5*Z*,7*E*,22*E*)-(1*S*,3*R*)-25-(1-Oxopentyl)-26,27-cyclo-9,10-secocholesta-5,7,10(19),22-tetraen-1,3-diol **76**
(5*Z*,7*E*,22*E*)-(1*S*,3*R*)-25-Acetyl-26,27-cyclo-24a-homo-9,10-secocholesta-5,7,10(19),22-tetraen-1,3-diol **126**
(5*Z*,7*E*)-(1*S*,3*R*,22*S*)-25-Acetyl-26,27-cyclo-24a-homo-9,10-secocholesta-5,7,10(19)-trien-1,3,22-triol **127**
(5*Z*,7*E*,22*E*)-(1*S*,3*R*)-25-(1-Oxobutyl)-26,27-cyclo-24a-homo-9,10-secocholesta-5,7,10(19),22-tetraen-1,3-diol **131**
(5*Z*,7*E*,22*E*)-(1*S*,3*R*)-25-(1-Oxopentyl)-26,27-cyclo-24a-homo-9,10-secocholesta-5,7,10(19),22-tetraen-1,3-diol **136**
(5*Z*,7*E*,22*E*)-(1*S*,3*R*,24*S*)-25-Acetyl-26,27-cyclo-24a,24b-dihomo-9,10-secocholesta-5,7,10(19),22-tetraen-1,3,24-triol **168b**
(5*Z*,7*E*,22*E*)-(1*S*,3*R*,24*S*)-25-(1-Oxobutyl)-26,27-cyclo-24a,24b-dihomo-9,10-secocholesta-5,7,10(19), 22-tetraen-1,3,24-triol **175b**
(5*Z*,7*E*,22*E*)-(1*S*,3*R*,24*S*)-25-(1-Oxopentyl)-26,27-cyclo-24a,24b-dihomo-9,10-secocholesta-5,7,10(19), 22-tetraen-1,3,24-triol **182b**
(5*Z*,7*E*,22*E*)-(1*S*,3*R*,24*S*)-25-Ethyl-26,27-cyclo-9,10-secocholesta-5,7,10(19),22-tetraen-1,3,24-triol **214a**
(5*Z*,7*E*,22*E*)-(1*S*,3*R*,24*R*)-25-Ethyl-26,27-cyclo-9,10-secocholesta-5,7,10(19),22-tetraen-1,3,24-triol **214b**
[5*Z*,7*E*,22*E*,25(*E*)]-(1*S*,3*R*,24*R*)-25-(1-Butenyl)-26,27-cyclo-9,10-secocholesta-5,7,10(19),22-tetraen-1,3,24-triol **221b**
(5*Z*,7*E*,22*E*)-(1*S*,3*R*,24*S*)-25-Butyl-26,27-cyclo-9,10-secocholesta-5,7,10(19),22-tetraen-1,3,24-triol **226a**
(5*Z*,7*E*,22*E*)-(1*S*,3*R*,24*R*)-25-Butyl-26,27-cyclo-9,10-secocholesta-5,7,10(19),22-tetraen-1,3,24-triol **226b**
(5*Z*,7*E*,22*E*)-(1*S*,3*R*,24*R*)-25-Hexyl-26,27-cyclo-9,10-secocholesta-5,7,10(19),22-tetraen-1,3,24-triol **231b**
(5*Z*,7*E*,22*E*)-(1*S*,3*R*,24*R*)-25-Heptyl-26,27-cyclo-9,10-secocholesta-5,7,10(19),22-tetraen-1,3,24-triol **236b**
[5*Z*,7*E*,22*E*,25(*Z*)]-(1*S*,3*R*,24*S*)-25-(1-Octenyl)-26,27-cyclo-9,10-secocholesta-5,7,10(19),22-tetraen-1,3,24-triol **246b**

Vergleichsverbindungen:

Calcitriol
(5*Z*,7*E*,22*E*)-(1*S*,3*R*,24*S*)-26,27-Cyclo-9,10-secocholesta-5,7,10(19),22-tetraen-1,3,24-triol (Calcipotriol)

| Verbindung | KF | DR HL 60 | DR Ca | T1/2 (min) |
|---|---|---|---|---|
| **66** | 10 | 0,5 | 30 | 110 |

(fortgesetzt)

| Verbindung | KF | DR HL 60 | DR Ca | T1/2 (min) |
|---|---|---|---|---|
| **71** | 9 | 1,7 | 100 | 120 |
| **76** | 7 | 19 | >100 | >120 |
| **81** | 40 | 9 | >>300 | >120 |
| **126** | 5 | 0,5 | 20 | 120 |
| **127** | 30 | 4 | >100 | 120 |
| **131** | 10 | 0,2 | 30 | 120 |
| **132** | 20 | 2 | >100 | 100 |
| **136** | 10 | 17 | >100 | >120 |
| **168b** | 3 | 7 | >100 | 120 |
| **175b** | 1 | 3 | >100 | 90 |
| **182b** | 2 | 18 | >100 | 100 |
| **214a** | 3 | 1,5 | >>1 | 80 |
| **214b** | 1 | 0,1 | >1 | 100 |
| **221b** | 2 | 0,1 | 10 | 90 |
| **226a** | 4 | 2 | >>1 | 80 |
| **226b** | 3 | 0,4 | >5 | 100 |
| **231b** | 9 | 2 | >100 | 75 |
| **236b** | 20 | 4 | >>30 | 100 |
| **246b** | 10 | 2 | >>10 | 100 |
| **Calcitriol** | 1 | 1 | 1 | 120 |
| **Calcipotriol** | 1 | 1 | 50 | 40 |

Die aufgeführten Verbindungen zeigen neben einer Affinität zum Vitamin D-Rezeptor, die der von Calcitriol vergleichbar ist, zum Teil eine ebenso vergleichbare oder bessere zelldifferenzierende Aktivität.

Die Induktion einer Hypercalcämie erfolgt dagegen durchweg erst bei sehr viel höheren Dosen als bei Calcitriol.

Die metabolische Stabilität der Verbindungen gleicht der von Calcitriol und ist deutlich höher als die des strukturell verwandten Calcipotriols.

**Verwendung der erfindungsgemäßen Verbindungen**

Durch die verminderte Eigenschaft, eine Hypercalcämie auszulösen sowie die hohe metabolische Stabilität, eignen sich die erfindungsgemäßen Substanzen in besonderer Weise zur Herstellung von Arzneimitteln für die Behandlung von Erkrankungen, die durch eine Hyperproliferation und fehlende Zelldifferenzierung gekennzeichnet sind. Dazu zählen zum Beispiel hyperproliferative Erkrankungen der Haut (Psoriasis, Pituriasis subia pilasis, Akne, Ichthyosis) und Pruritus sowie Tumorerkrankungen und Präkanzerosen (zum Beispiel Darmtumoren, Mammakarzinom, Lungentumoren, Prostatakarzinom, Leukämien, T-Zell-Lymphome, Melanome, Batazell Larzin, Squamous Carcinoma, aktinische Keratosen, Cervixdysplasien, metastasierende Tumore jeglicher Art).

Auch zur Behandlung und Prophylaxe von Erkrankungen, die durch eine Störung des Gleichgewichts des Immunsystems gekennzeichnet sind, eignen sich die erfindungsgemäßen Substanzen. Hierzu zählen Ekzeme und Erkrankungen des atopischen Formenkreises und entzündliche Erkrankungen (rheumatoide Arthritis, Asthma) sowie Autoimmunerkrankungen wie zum Beispiel Multiple Sklerose, Diabetes mellitus Typ I, Myasthenia gravis, Lupus erythematodes, Sklerodermie, bullöse Hauterkrankungen (Pemphigus, Pemphigoid), weiterhin Abstoßungsreaktionen bei autologen, allogenen oder xenogenen Transplantaten sowie AIDS. Bei all diesen Erkrankungen können die neuen

Verbindung der allgemeinen Formel **I** vorteilhaft mit anderen immunsuppressiv wirksamen Stoffen wie Cyclosporin A, FK 506, Rapamycin und Anti-CD 4-Antikörpern kombiniert werden.

Ebenso sind die Substanzen geeignet zur Therapie von sekundärem Hyperparathyreoidismus und renaler Osteodystrophie infolge der Eigenschaft von Calcitriolen, die Parathormonsynthese zu senken.

Aufgrund der Präsenz des Vitamin D-Rezeptor in den insulinproduzierenden Zellen der Bauchspeicheldrüse eignen sich die Substanzen durch Erhöhung der Insulinsekretion zur Therapie des Diabetes mellitus Typ II.

Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise um den Faktor 6 erhöht.

Diese Eigenschaften der erfindungsgemäßen Derivate in der Vitamin D-Reihe läßt sie zum therapeutischen Einsatz bei atrophischer Haut, wie sie bei natürlicher Hautalterung infolge erhöhter Lichtexposition oder medikamentös induzierter Hautatrophie durch Behandlung mit Glucocorticoiden auftritt, geeignet erscheinen.

Darüber hinaus kann die Wundheilung durch topische Applikation mit den neuen Verbindungen beschleunigt werden.

In Zellpopulationen das Haarfollikels, die entscheidend zum Haarwachstum bzw. der Haarzyklusregulation beitragen, konnten Vitamin $D_3$-Rezeptorproteine nachgewiesen werden [Stumpf, W. E. et al., *Cell Tissue Res*. **238**, 489 (1984); Milde, P. et al., *J. Invest. Dermatol*. **97**, 230 (1991)]. Außerdem zeigen in vitro-Befunde an isolierten Haarfollikelkeratinozyten einen proliferationsinhibierenden und differenzierungsstimulierenden Einfluß von 1,25-$(OH)_2$-$D_3$.

Aus klinischen Beobachtungen ist bekannt, daß die Vitamin $D_3$-resistente Rachitis häufig mit einer Alopezie einhergeht, die sich im frühen Kindesalter ausprägt. Experimentelle Befunde zeigen, daß die Vitamin $D_3$-Bindungsstelle des VDR bei dieser Erkrankung mutiert, d. h. defekt ist [Kristjansson. K et al., *J. Clin. Invest.* **92**, 12 (1993)]. Keratinozyten, die aus den Haarfollikeln dieser Patienten isoliert wurden, reagieren in vitro nicht auf die Zugabe von 1.25-$(OH)_2$-$D_3$ [Arase, S. et al., *J. Dermatol. Science* **2**, 353 1991)].

Aus diesen Befunden läßt sich eine entscheidende Rolle von 1,25-$(OH)_2$-$D_3$ auf die Regulation des Haarwuchstums ableiten.

Daher eignen sich diese Analoga besonders zur Herstellung von Arzneimitteln zur Behandlung von Erkrankungen, die mit einem gestörten Haarwachstum einhergehen (androgenetische Alopezie, Alopezia areata/totalis, chemotherapieinduzierte Alopezie), oder zur Unterstützung des physiologischen Haarwachstums ohne die Nebenwirkungen des Calcitriols (insbesondere Hypercalcämie) hervorzurufen.

Die senile und postmenopausale Osteoporose ist gekennzeichnet durch einen erhöhten Knochenumsatz mit einer insgesamt negativen Bilanz. Aufgrund des Knochenschwundes insbesondere von trabekulärem Knochen kommt es in verstärktem Maße zu Knochenbrüchen. Aufgrund der fördernden Wirkung von Calcitriol sowohl auf die Anzahl als auch die Syntheseleistung von knochenneubildenden Zellen (Osteoblasten) eignen sich die erfindungsgemäßen Substanzen zur Therapie und Prophylaxe der senilen und postmenopausalen Osteoporose (EP 0 634 173 A1), der steroidinduzierten Osteoporose sowie zur beschleunigten Einheilung von Gelenkplastiken ohne die Nebenwirkungen des Calcitriols (insbesondere Hypercalcämie) hervorzurufen. Für die Therapie der verschiedenen Formen der Osteoporose können sie vorteilhaft mit Estradiol oder anderen Abkömmlingen des Östrogens kombiniert werden.

Schließlich konnte gezeigt werden, daß Calcitriol die Synthese eines Wuchsstoffes für Nervenzellen (nerve growth factor) steigert [M.S. Saporito et al. *Brain Res.* **633,** 189 (1994)]. Daher eignen sich die erfindungsgemäßen Verbindungen auch zur Behandlung von degenerativen Erkrankungen des peripheren und zentralen Nervensystems, wie der Alzheimerschen Erkrankung und der amyotrophen Lateralsklerose.

Es wurde außerdem gefunden, daß bestimmte Verbindungen der allgemeinen Formel **I** in HL 60-Zellen überraschenderweise die Wirkung von Calcitriol antagonisieren (siehe auch WO 94/07853, WO 97/00242).

Solche Verbindungen können bei der Therapie von Hypercalcämien eingesetzt werden, wie zum Beispiel bei Hypervitaminose D oder Intoxikation mit Calcitriol und calcitriolartig wirksamen Substanzen, oder bei erhöhter extrarenaler Calcitriolsynthese bei granulomatösen Erkrankungen (Sarkoidose, Tuberkulose). Auch paraneoplastische Hypercalcämien (zum Beispiel bei osteolytischen Metastasen und Tumoren mit erhöhter Synthese von Parathormon-related peptide) sowie bei Hypercalcämie bei Hyperparathyreoidismus.

Weiterhin sind Calcitriolantagonisten zur Fertilitätskontrolle einzusetzen. Im Reproduktionstrakt weiblicher und männlicher Tiere wird der Vitamin D-Rezeptor exprimiert. Es ist bekannt, daß die weibliche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z. B. bei Infektabwehr-schwäche, einzusetzen.

Von Calcitriol ist bekannt, daß es das Haarwachstum modulieren kann. Calcitriolantagonisten können daher bei unerwünschtem Haarwachstum, z. B. beim Hirsutismus, therapeutische Verwendung finden.

Eine fördernde Rolle von Vitamin D auf die Bildung von arteriosklerotischen Plaques ist seit langem bekannt. In solchen Gefäßläsionen wird ein Calcitriolreguliertes Protein, das Osteopontin, vermehrt gefunden, dem eine Rolle bei der Gefäßverkalkung zugeschrieben wird [R. Eisenstein et al. *Arch. Path.* **77**, 27 (1964), L.A. Fitzpatrick et al. *J. Clin. Invest*. **94**, 1597 (1994)]. Deshalb eignen sich Calcitriolantagonisten zur Therapie und Prophylaxe aller Erscheinungsformen der Arteriosklerose.

Schließlich eignen sich Calcitriolantagonisten infolge der Eigenschaft von Calcitriol, unspezifische Immunreaktionen von monocytären Zellen zu steigern, zur Therapie von entzündlichen Erkrankungen insbesondere chronischer Natur, wie rheumatoide Arthritis, Morbus Crohn, Colitis ulcerosa, und granulomatösen Erkrankungen wie Sarkoidose und anderen Fremdkörperreaktionen.

Für alle aufgezählten therapeutischen Anwendungen gilt, daß die erfindungsgemäßen Verbindungen in der Lage sind, in den genannten Krankheitsbildern eine therapeutische Wirkung zu erreichen, ohne die Nebenwirkungen des Calcitriols (insbesondere Hypercalcämie) hervorzurufen.

Die vorliegende Erfindung bezieht sich somit auf pharmazeutische Präparate, die mindestens eine Verbindung gemäß der allgemeinen Formel **I** zusammen mit einem pharmazeutisch verträglichen Träger enthalten.

Die Verbindungen können als Lösungen in pharmazeutisch verträglichen Solventien oder als Emulsionen, Suspensionen, oder Dispersionen in geeigneten pharmazeutischen Solventien oder Trägern oder als Pillen, Tabletten oder Kapseln, die in an sich bekannter Weise feste Trägerstoffe enthalten, formuliert werden. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien. Die Verbindungen werden vorteilhafterweise durch Injektion, intravenöse Infusion in geeigneter steriler Lösungen, als Aerosol über Bronchien und Lunge oder als orale Dosierung über den Ernährungstrakt oder topisch in der Form von Cremes, Salben, Lotions oder geeigneter transdermaler Pflaster appliziert, wie in der EP-A 0 387 077 beschrieben ist.

| | |
|---|---|
| Die tägliche Dosis liegt bei | 0,1 µg/Patient/Tag -1000 µg/Patient/Tag, |
| vorzugsweise | 1,0 µg/Patient/Tag - 500 µg/Patient/Tag. |

**Verfahren zur Herstellung der erfindungsgemäßen Verbindungen**

Die Herstellung der Vitamin D-Derivate der allgemeinen Formel **I** erfolgt erfindungsgemäß aus einer Verbindung der allgemeinen Formel **II**,

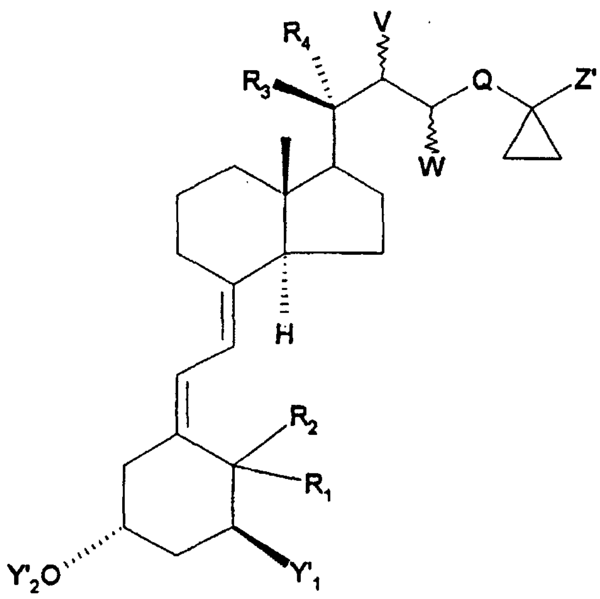

(II)

worin **Y'$_1$** ein Wasserstoffatom, ein Halogenatom oder eine geschützte Hydroxylgruppe und **Y'$_2$** eine Hydroxyschutzgruppe bedeuten.

**Z'** unterscheidet sich von **Z** dadurch, daß eventuell vorliegende Hydroxylgruppen oder Ketogruppen in geschützter Form vorliegen können.

Bei den Schutzgruppen handelt es sich vorzugsweise um alkyl-, aryl- oder gemischt alkylarylsubstituierte Silylgruppen, z.B. die Trimethylsilyl- (TMS), Triethylsilyl- (TES), *tert.*-Butyldimethylsilyl- (TBDMS), *tert.*-Butyldiphenylsilyl-(TBDPS) oder Triisopropylsilylgruppen (TIPS) oder eine andere gängige Hydroxyschutzgruppe (Methoxymethyl-, Methoxyethoxymethyl-, Ethoxyethyl-, Tetrahydrofuranyl- Tetrahydropyranyl-Gruppen) für die Ketogruppen handelt es sich vorzugsweise um Ketale (1,3-Dioxolane, 1,3-Dioxane, Dialkoxyketale) (siehe T.W. Greene, P.G.M. Wuts "Protective Groups in Organic Synthesis", 2nd Edition, John Wiley & Sons, 1991).

Durch gleichzeitige oder sukzessive Abspaltung der Hydroxy- sowie Ketoschutzgruppen und gegebenenfalls durch partielle, sukzessive oder vollständige Veresterung der freien Hydroxylgruppen wird **II** in eine Verbindung der allgemeinen Formel **I** überführt.

Im Falle der Silylschutzgruppen oder der Trimethylsilylethoxymethylgruppe verwendet man zu deren Abspaltung Tetrabutylammoniumfluorid, Fluorwasserstoffsäure oder Fluorwasserstoffsäure/Pyridin oder saure Ionentauscher; im Falle von Ethergruppen (Methoxymethyl-, Methoxyethoxymethyl-, Ethoxyethyl-, Tetrahydropyranylether) und Ketalen werden diese unter katalytischer Einwirkung von Säure, beispielsweise p-Toluolsulfonsäure, Pyridinium-p-toluolsulfonat, Essigsäure, Salzsäure, Phosphorsäure oder einem sauren Ionenaustauscher abgespalten.

Die Veresterung der freien Hydroxygruppen kann nach gängigen Verfahren mit den entsprechenden Carbonsäurechloriden, -bromiden oder -anhydriden erfolgen.

Die Herstellung der Ausgangsverbindungen für die allgemeine Formel **II** geht je nach letztendlich gewünschtem Substitutionsmuster in 10- und 20-Position von verschiedenen Startverbindungen aus.

Für die Herstellung von Verbindungen der allgemeinen Formel **II,** worin $R_1$ und $R_2$ gemeinsam eine exocyclische Methylengruppe und $Y'_1$ ein Wasserstoffatom oder eine geschützte Hydroxylgruppe und $Y'_2$ eine Hydroxyschutzgruppe bedeuten, wird von dem bekannten Aldehyd **III** ausgegangen [M. Calverley Tetrahedron **43**, 4609 (1987), WO 87/00834].

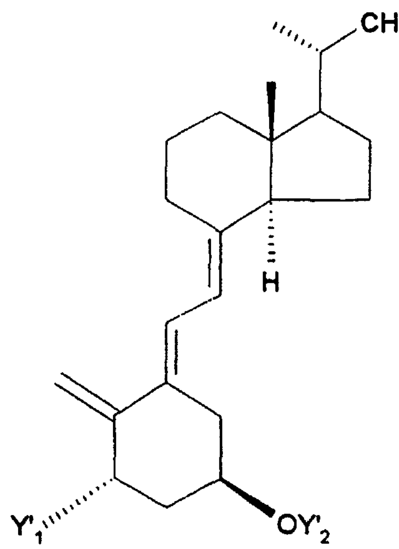

(III)

Andere Schutzgruppen für $Y'_1$ und $Y'_2$ als die in den Literaturstellen erwähnten lassen sich durch analoge Vorgehensweise unter Verwendung entsprechend modifizierter Silylchloride (z.B. *tert*.-Butyldiphenylsilylchlorid anstelle von *tert.*-Butyldimethylsilylchlorid) erhalten. Durch Verzicht auf die entsprechenden Stufen zur 1α-Hydroxylierung lassen sich Derivate vom Typ $Y'_1$=H erhalten.

Die Verbindungen der allgemeinen Formel **III** werden nun analog bekannter Verfahren in Aldehyde der allgemeinen Formel **IV** überführt [EP 647 219, WO 94/07853, M.J. Calverley, L. Binderup *Bioorg. Med. Chem. Lett.* 3, 1845-1848 (1993)].

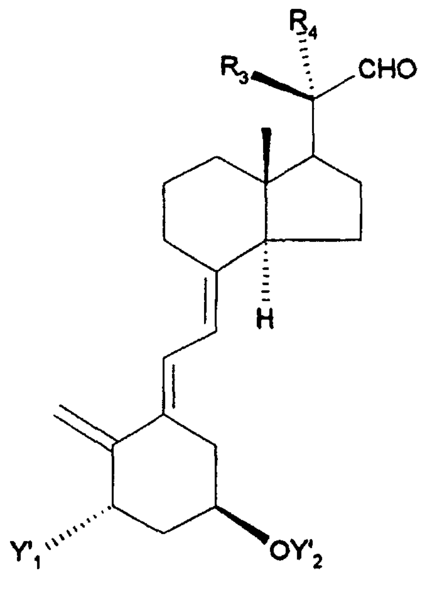

(IV)

Für $R_3$ und $R_4$ gelten die schon eingangs erwähnten Definitionen.

Zur Etablierung des natürlichen Vitamin D-Triensystems wird eine photochemische Isomerisierung von Verbindungen der allgemeinen Formel **IV** vorgenommen. Bestrahlung mit ultraviolettem Licht erfolgt in Gegenwart eines sogenannten Triplettsensibilisators. Im Rahmen der vorliegenden Erfindung wird dafür Anthracen verwendet. Durch Spaltung der π-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt, wobei Verbindungen der allgemeinen Formel **V** anfallen,

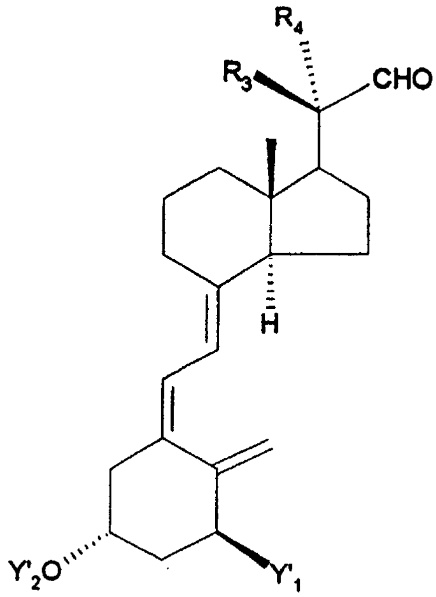

(V)

Grundsätzlich ist diese Isomerisierungsreaktion auch auf einer späteren Stufe möglich. Exemplarisch werden im Folgenden die Umsetzungen des Aldehydes **VI** mit natürlicher Konfiguration an C-20 beschrieben.

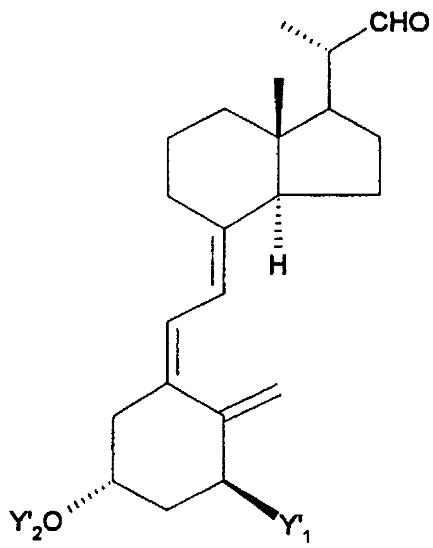

(VI)

Prinzipiell sind die folgenden Umsetzungen aber auch mit den genannten Substitutionsmustern an C-20 möglich.

Zunächst wird die Synthese von Verbindungen, die einen Spezialfall der allgemeinen Formel **II** darstellen, beschrieben. So bilden $R_1$ und $R_2$ gemeinsam eine Methylengruppe, $R_3$ ist ein Wasserstoffatom und $R_4$ ist eine Methylgruppe, Q ist eine Methylengruppe und Z' bedeutet eine geradkettige 1-Oxoalkylgruppe mit 1-12 Kohlenstoffatomen, in der die Ketogruppe geschützt vorliegt (z.B. Ketal: Dialkoxyketal, 1,3-Dioxolan, 1,3-Dioxan, 5,5-Dimethyl-1,3-dioxan).

Startmaterial für die Seitenkettenfragmente ist ein Acetessigsäureester der allgemeinen Formel **VII**.

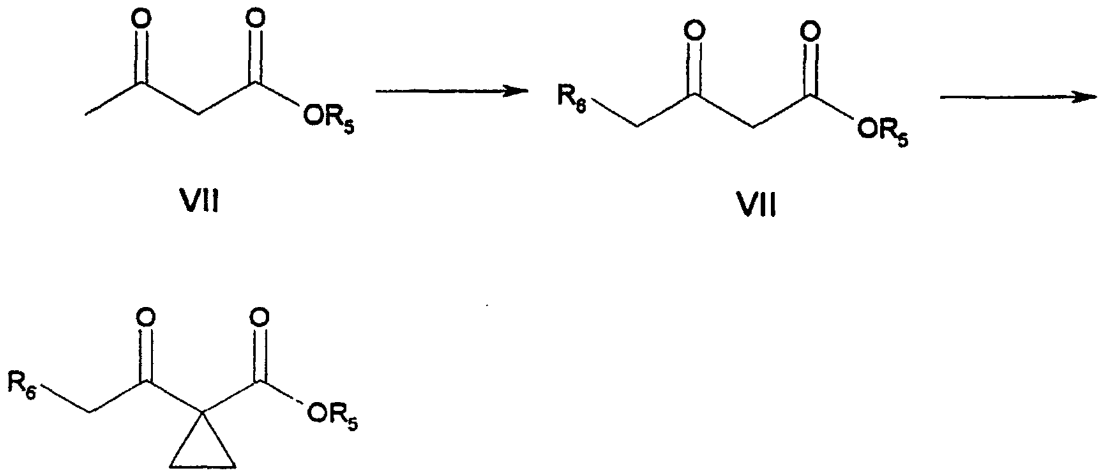

VII VII

IX

$R_5$ kann eine geradkettige oder verzweigte Alkylgruppe mit bis zu 6 Kohlenstoffatomen sein (vorzugsweise Methyl- oder Ethylgruppe). Zum Aufbau längerer Ketten wird die Verbindung **VII** mit zwei Äquivalenten einer oder zwei verschiedener Basen (z.B. Lithiumdiisopropylamid, *n*-Butyllithium, Natriumhydrid, Kaliumhydrid) doppelt deprotoniert und anschließend mit einem Äquivalent eines Alkylhalogenides (Bromid oder lodid) an der reaktiveren Position alkyliert, wobei die Verbindung der allgemeinen Formel **VIII** anfällt [L. Weiler et al. *J. Am. Chem. Soc.* **96**, 1082-1087 (1974), N. Haddad et al. *J. Org. Chem.* **60**, 6883-6887 (1995), D.F. Taber et al. *J. Org. Chem.* **60**, 2283-2285 (1995)]. $R_6$ bedeutet eine geradkettige oder verzweigte Alkylgruppe mit bis zu 11 Kohlenstoffatomen.

Die Verbindung **VII** oder **VIII** werden nun mit einer Base (z.B. Kaliumcarbonat, Natriumcarbonat, Kaliuma (koholat) und 1,2-Dibromethan umgesetzt, wobei Verbindungen der allgemeinen Formel **IX** entstehen. $R_6$ hat die schon genannte Bedeutung oder ist ein Wasserstoffatom [D.F. Taber et al. *J. Org. Chem.* **57**, 436-441 (1992)].

Die Ketogruppe der Verbindung **IX** wird nun unter Standardbedingungen in ein Ketal überführt, wobei die Verbindung **X** entsteht, in der K eine Ketoschutzgruppe bedeutet. Als Ketoschutzgruppen kommen alle in "Protective Groups in Organic Synthesis", 2nd Edition, John Wiley & Sons, 1991 (T.W. Greene, P.G.M. Wuts) infrage (z. B. 1,3-Dioxolan, 1,3-Dioxan, 5,5-Dimethyl-1,3-dioxan, Dialkoxyketale). Exemplarisch wird im Folgenden das 1,3-Dioxolan-Derivat **X** (K= $-O-CH_2-CH_2-O-$) beschrieben. Die Verwendung anderer Ketalgruppen ist aber grundsätzlich möglich.

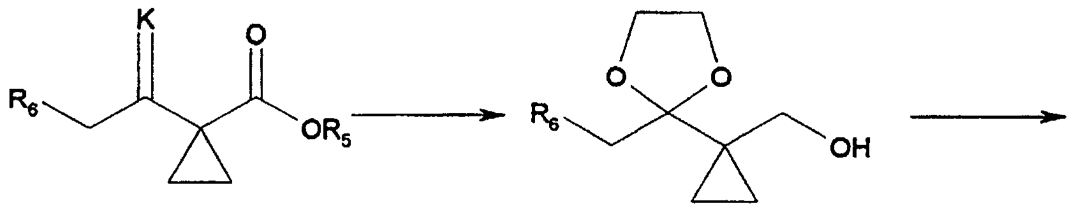

**X** **XI**

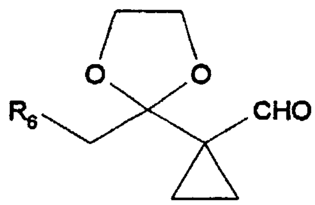

**XII**

Reduktion der Estereinheit zum Alkohol **XI** kann mit einem gängigen Reduktionsmittel erfolgen (z.B. Lithiumaluminumhydrid, Diisobutylaluminiumhydrid). Anschließende Oxidation unter den üblichen Bedingungen (Mangandioxid, Pyridiniumchlorochromat, Pyridiniumdichromat, Swern-Bedingungen) ergibt als wichtiges Zwischenprodukt den Aldehyd **XII** (zeichnerisch dargestellt als 1,3-Dioxolan-Derivat, aber nicht auf diese Schutzgruppe beschränkt, sondern auch mit anderen Ketoschutzgruppen herstellbar, s.o.). In einer Wittig-Reaktion mit dem Ylid aus Methyltriphenylphosphoniumiodid oder -bromid (Deprotonierung z.B. mit *n*-Butyllithium) wird das Olefin **XIII** generiert.

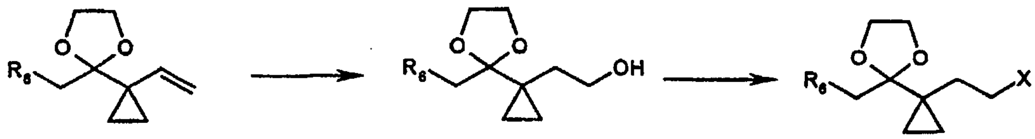

**XIII** **XIV** **XV**

Die Doppelbindung kann nun unter den Standardbedingungen hydroboriert und nach oxidativer Aufarbeitung in den Alkohol **XIV** überführt werden [Pelter, Smith, Brown *Borane Reagents;* Academic Press: New York, 1988; H.C. Browm et al. *Heterocycles* **25,** 641-567 (1987).] Die Alkoholfunktion wird nun tosyliert (**XV**, X=OTos) und mit dem Thiophenolatanion zum Thioether **XV** (X=SPh) umgesetzt. Oxidation (z.B. mit Wasserstoffperoxid, Metachlorperbenzoesäure, *tert.*-Butylhydroperoxid, Natriumperiodat) ergibt dann das Sulfon **XV** (X=$SO_2$Ph) [P.C. Bulman Page et al. *Synth. Comm.* **23,** 1507-1514 (1993)].

Das Sulfon **XV** wird nun mit einer Base (z.B. *n*-Butyllithium, Lithiumdiisopropylamid, Natriumhydrid, Kaliumhydrid) deprotoniert und bei tiefer Temperatur (zwischen -100°C und -20°C) mit dem Aldehyd **VI** umgesetzt, wobei Hydroxysulfone der allgemeinen Formel **XVI** (X=$SO_2$Ph) anfallen.

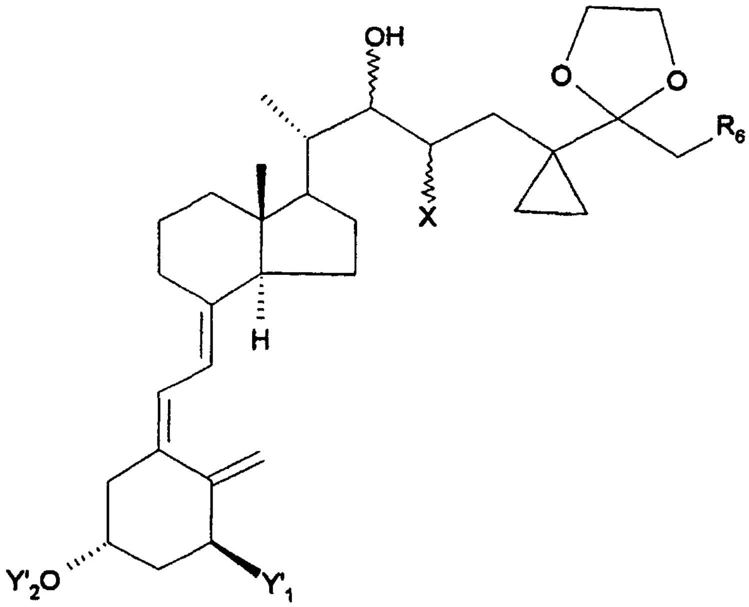

(XVI)

Überführung der Hydroxysulfone in Verbindungen, die eine Doppelbindung in der Seitenkette tragen gelingt im Fall von Vitamin D-Derivaten vorzugsweise durch Reaktion mit Natriumamalgam (H.F. DeLuca et al. *Bioorg. Chem.* **15**, 152-166 (1987), H.F. DeLuca et al. *Biochemistry* **29**, 190-196 (1990)].

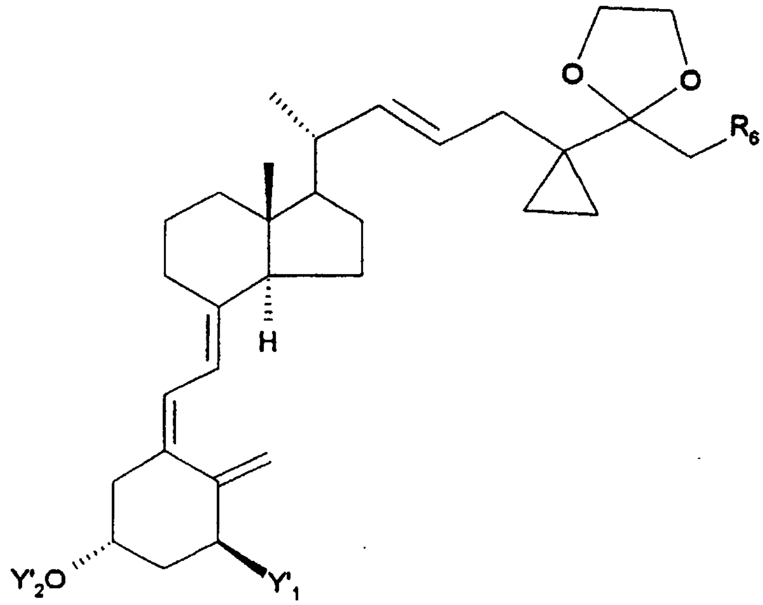

(XVII)

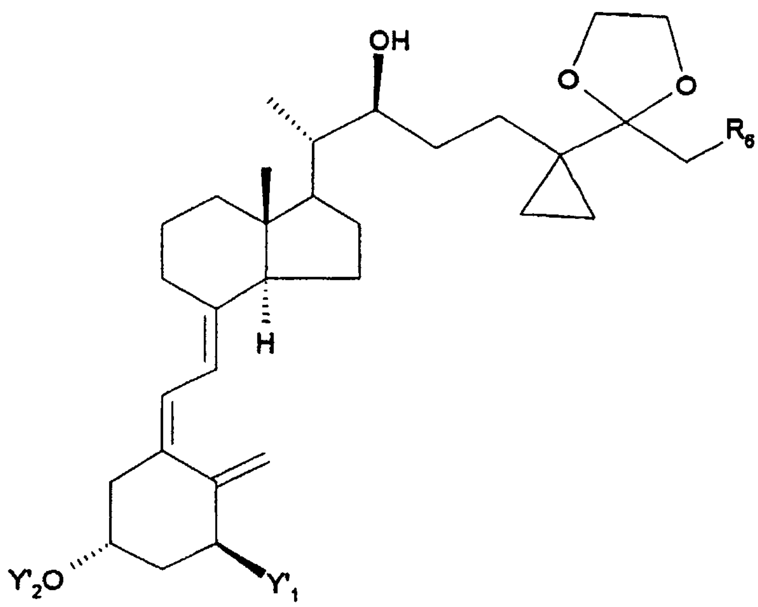

(XVIII)

Neben den Olefinen der allgemeinen Formel **XVII** fallen auch die Alkohole der allgemeinen Formel **XVIII** an, die chromatographisch abgetrennt werden.

Die Verbindungen der allgemeinen Formeln **XVII** und **XVIII** können als Sonderfälle der allgemeinen Formel **II** angesehen werden und können wie vorstehend beschrieben in die Titelverbindungen der allgemeine Formel **I** überführt werden.

Im Folgenden wird die Synthese von Verbindungen, die einen weiteren Spezialfall der allgemeinen Formel **II** darstellen, beschrieben. So bilden $R_1$ und $R_2$ gemeinsam eine Methylengruppe, $R_3$ ist ein Wasserstoffatom und $R_4$ ist eine Methylgruppe, Q ist eine Ethylengruppe und Z' hat die bereits vorstehend erwähnte Definition.

Zum Aufbau der Seitenkettenfragmente werden Aldehyde der allgemeinen Formel **XII** in Horner-Wadsworth-Emmons-Reaktionen mit deprotonierten Phosphonoessigsäureestern (Base: z.B. Lithiumdiisopropylamid, *n*-Butyllithium, Natriumhydrid, Kaliumhydrid) umgesetzt, wobei Ester der allgemeinen Formel **XIX** anfallen.

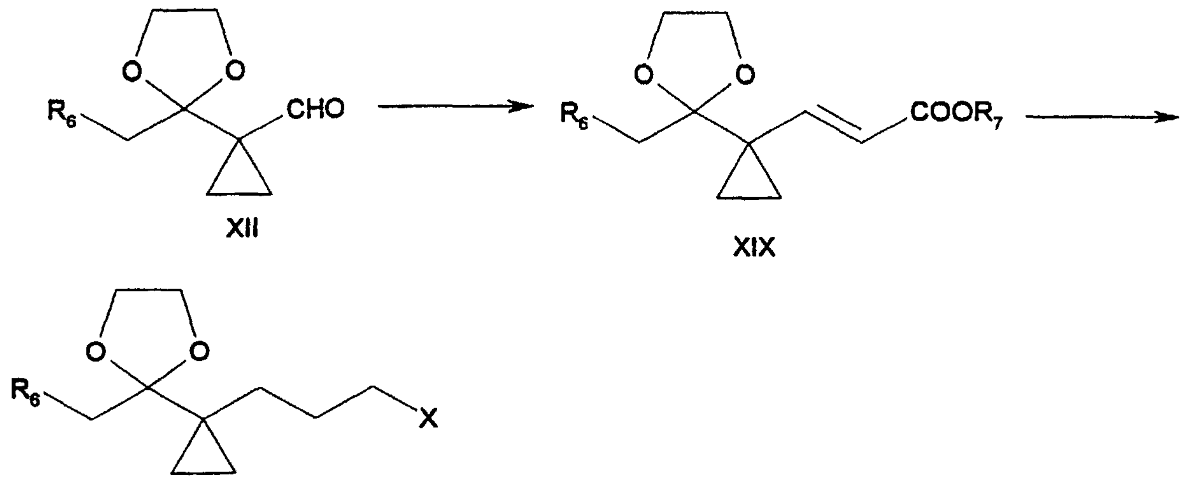

XII

XIX

XX

Reduktion unter Birch-Bedingungen (Lithium, Natrium oder Calcium in flüssigem Ammoniak oder Aminen) liefert die Alkohole der allgemeinen Formel **XX** (X=OH).

Die Alkoholfunktion wird nun tosyliert (**XX**, X=OTos) und mit dem Thiophenolatanion zum Thioether **XX** (X=SPh) umgesetzt. Oxidation (z.B. mit Wasserstoffperoxid, Metachlorperbenzoesäure, *tert.*-Butylhydroperoxid, Natriumperiodat) ergibt dann das Sulfon **XX** (X=$SO_2$Ph) [P.C. Bulman Page et al. *Synth. Comm.* 23, 1507-1514 (1993)].

Das Sulfon **XX** wird nun mit einer Base (z.B. *n*-Butyllithium, Litiumdiisopropylamid, Natriumhydrid, Kaliumhydrid) deprotoniert und bei tiefer Temperatur (zwischen -100°C und -20°C) mit dem Aldehyd **VI** umgesetzt, wobei Hydroxysulfone der allgemeinen Formel **XXI** (X=$SO_2$Ph) anfallen.

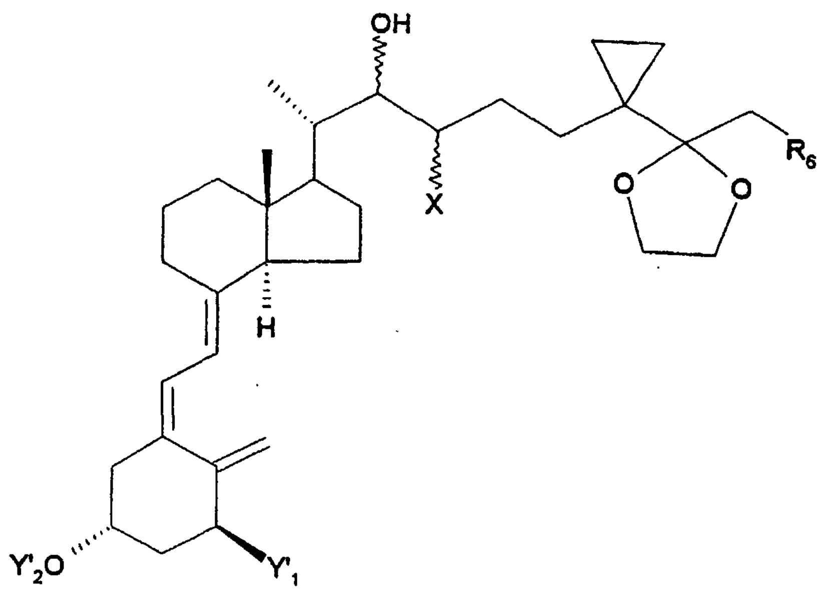

(XXI)

Überführung der Hydroxysulfone in Verbindungen, die eine Doppelbindung in der Seitenkette tragen gelingt im Fall von Vitamin D-Derivaten vorzugsweise durch Reaktion mit Natriumamalgam (H.F. DeLuca et al. *Bioorg. Chem.* 15, 152-166 (1987), H.F. DeLuca et al. *Biochemistry* 29, 190-196 (1990)].

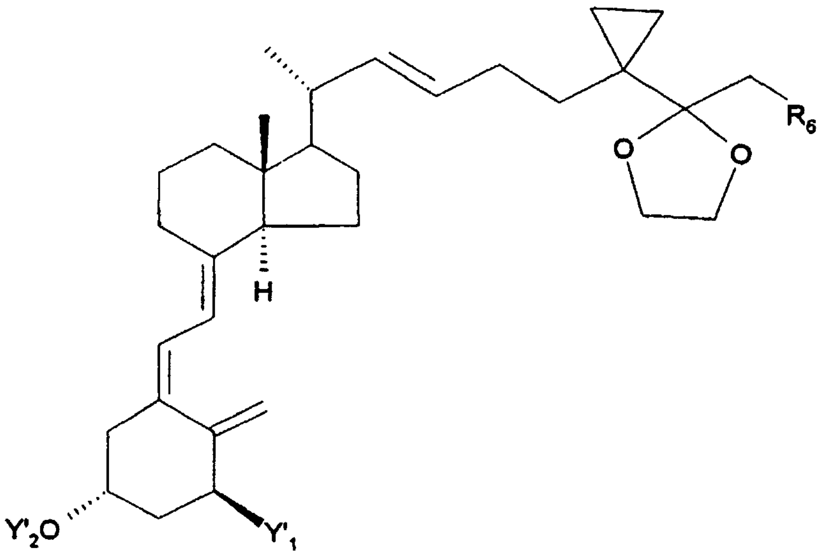

(XXII)

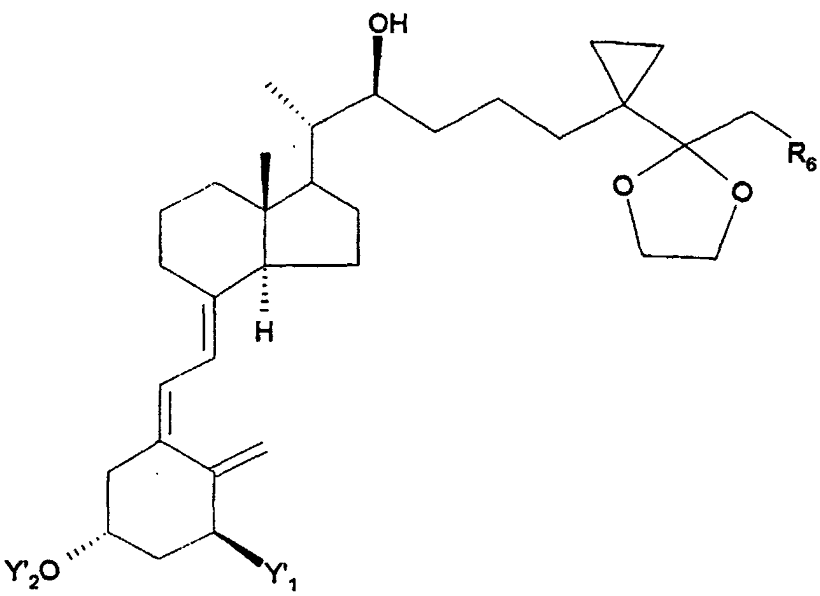

(XXIII)

Neben den Olefinen der allgemeinen Formel **XXII** fallen auch die Alkohole der allgemeinen Formel **XXIII** an, die chromatographisch abgetrennt werden.

Die Verbindungen der allgemeinen Formeln **XXII** und **XXIII** können als Sonderfälle der allgemeinen Formel **II** angesehen werden und können wie vorstehend beschrieben in die Titelverbindungen der allgemeine Formel **I** überführt werden.

Im Folgenden wird die Synthese von Verbindungen, die einen weiteren Spezialfall der allgemeinen Formel **II** darstellen, beschrieben. So bilden $R_1$ und $R_2$ gemeinsam eine Methylengruppe, $R_3$ ist ein Wasserstoffatom und $R_4$ ist eine Methylgruppe, Q bedeutet $-CH(OH)-CH_2-CH_2-$ und Z' hat die schon genannte Bedeutung.

In diesem Fall wird der Aldehyd der allgemeinen Formel **XII** mit Oxopropylphosphonsäuredialkylester in Gegenwart einer Base (Triethylamin, Ethyldiisopropylamin, Triisopropylamin, Diazabicyclononan, Diazabicycloundecan, Natriumhydrid) unter eventuellem Zusatz von Lithiumchlorid in das Keton **XXIV** überführt [S. Masamune et al. *Tetrahedron Lett.* __**25,**__ 2183-2186 (1984), **B.** Resul et al. *J. Med. Chem.* __**36,**__ 243-248 (1993)].

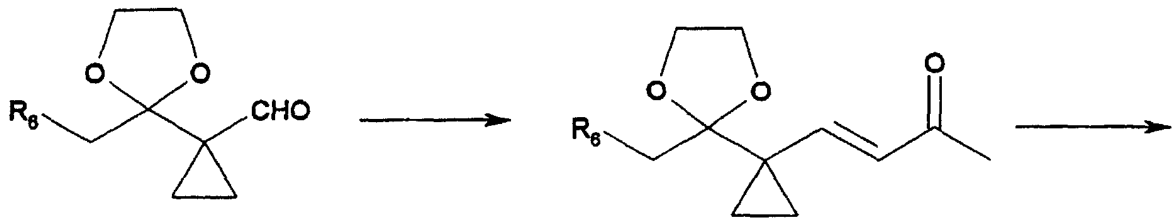

XII

XXIV

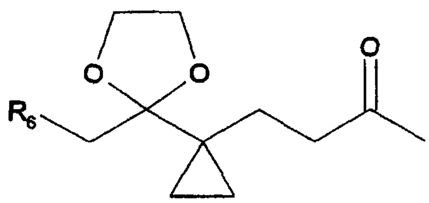

XXV

Reaktion zu gesättigten Ketonen der allgemeinen Formel **XXV** kann durch Birch-Reduktion (vorstehend beschrieben) eventuell gefolgt von Reoxidation (z.B. mit Pyridiniumdichromat, Pyridiniumchlorochromat, Swem-Bedingungen) oder durch Hydrierung der Doppelbindung erfolgen. Um eine hydrogenolytische Spaltung des Cyclopropylringes zu vermeiden, sollte als Katalysator Platin(VI)oxid oder ein löslicher Rhodium-Katalysator (z.B. Wilkinson-Katalysator) verwendet werden.

Die Ketone **XXV** werden mit einer Base regioselektiv deprotoniert (z.B. Lithiumdiisopropylamid, Lithium- oder Natriumhexamethyldisilazid) und bei tiefer Temperatur mit dem Aldehyd der allgemeinen Formel **VI** umgesetzt, wobei Verbindungen der allgemeinen Formel **XXVI** (X=OH) anfallen.

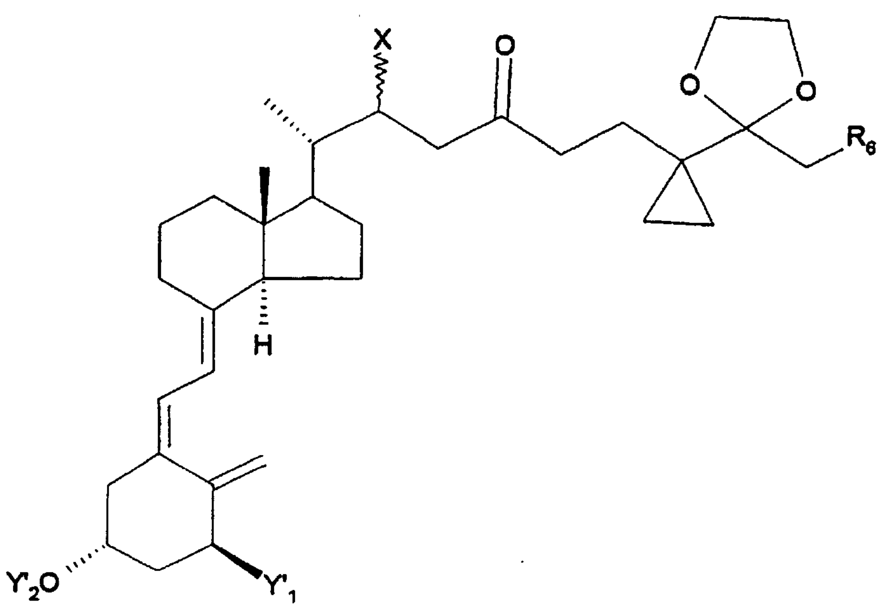

(XXVI)

Die Hydroxylgruppe wird anschließend unter Standardbedingungen in eine Fluchtgruppe überführt, wobei Verbindungen der allgemeinen Formel **XXVI** (X= z.B. Acetat, Trifluoracetat, Tosylat, Mesylat oder Triflat) entstehen.

Eliminierung unter Einsatz von Basen (z.B. Diazabicyclononan, Diazabicycloundecan, Triethylamin, Diisopropylamin, Ethyldiisopropylamin) bei gegebenfalls erhöhten Reaktionstemperaturen liefert die Ketone der allgemeinen Formel **XXVII.**

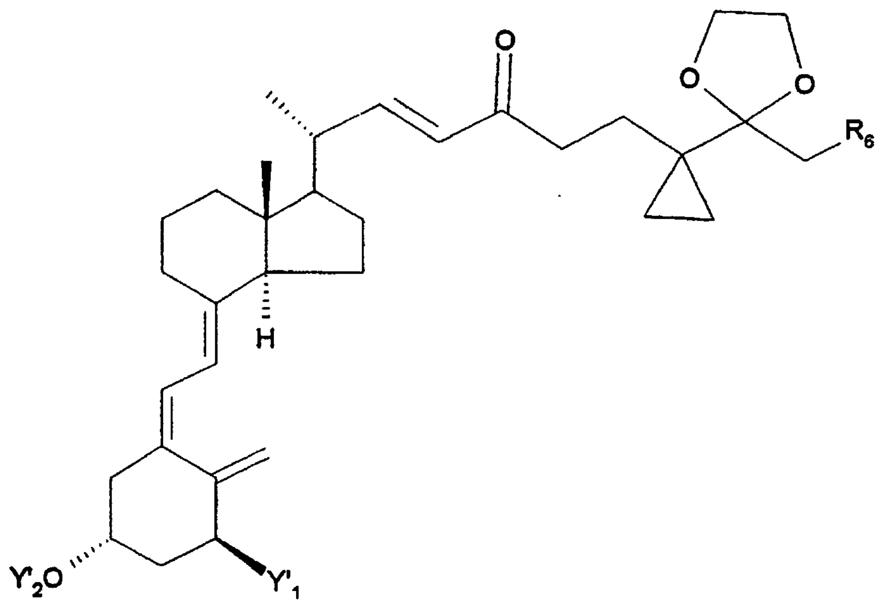

(XXVII)

Die Carbonylgruppen in **XXVII** können nun zu den diastereomeren Alkoholen **XXVIII** reduziert werden (Reduktionsmittel: z.B. Natriumborhydrid/Certrichlorid, Lithium-aluminiumhydrid, Diisobutylaluminiumhydrid, Lithiumborhydrid) und chromatographisch getrennt werden.

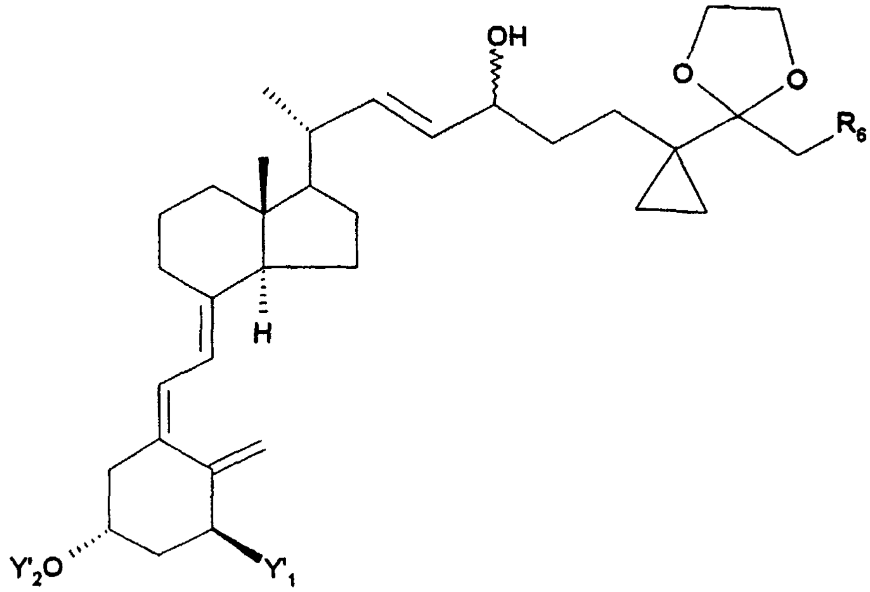

(XXVIII)

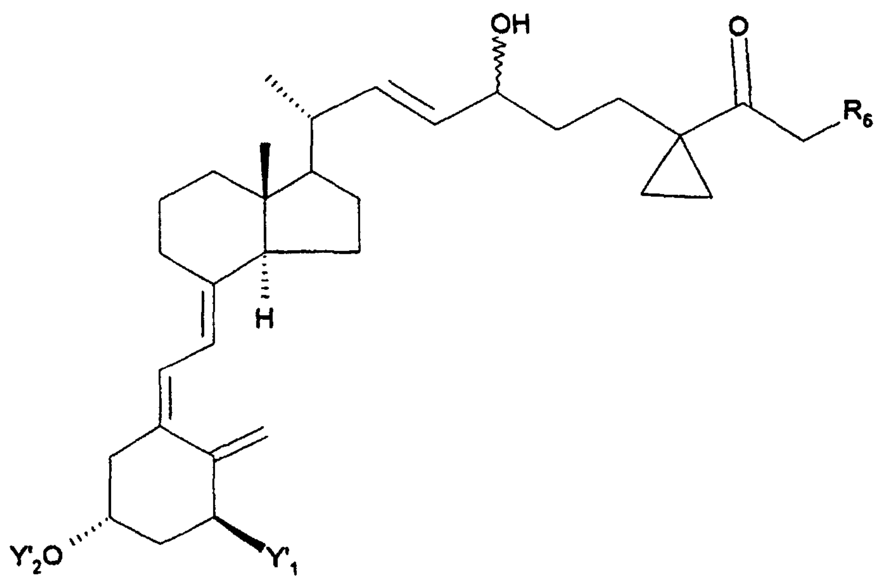

(XXIX)

Die Schutzgruppenabspaltung an der Verbindung der allgemeinen Formel **XXVIII**, welche als Sonderfall der allgemeinen Formel **II** aufzufassen ist, sollte sukzessive erfolgen. Das Ketal wird durch mild saure Reaktionsbedingungen (z.B. Pyridinium-p-Toluolsulfonat, Oxalsäure/Silicagel) abgespalten, wobei Verbindungen der allgemeinen Formel **XXIX** anfallen. Wie beschrieben gelangt man dann zu Verbindungen der allgemeinen Formel **I**.

Im Folgenden wird die Synthese von Verbindungen, die einen weiteren Spezialfall der allgemeinen Formel **II** darstellen, beschrieben. So bilden $R_1$ und $R_2$ gemeinsam eine Methylengruppe, $R_3$ ist ein Wasserstoffatom und $R_4$ ist eine Methylgruppe, Q bedeutet -CH(OH)- und Z' bedeutet eine Alkyl- oder Alkenylgruppe mit 1-12 Kohlenstoffatomen.

Man setzt den Aldehyd der allgemeinen Formel **XII** für den Fall, daß $R_6$ ein Wasserstoffatom ist, mit Alkyltriphenylphosphoniumsalzen in Gegenwart von Basen (z.B. n-Butyllithium, Natriumhydrid, Kaliumhydrid) in Wittig-Reaktionen um, wobei Verbindungen der allgemeinen Formel **XXX** anfallen. $R_7$ bedeutet eine geradkettige oder verzweigte Alkyl- oder Alkenylgruppe mit 1-10 Kohlenstoffatomen. Üblicherweise entstehen *E,Z*-Gemische der Doppelbindung. Eine chromatographische Isomerentrennung wird erst auf einer späteren Stufe durchgeführt.

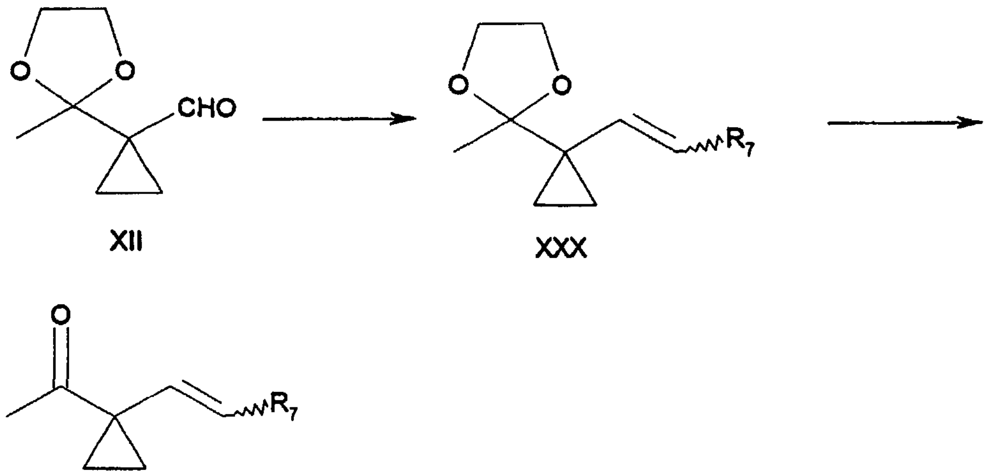

XII

XXX

XXXI

Die Spaltung der Ketaleinheit erfolgt unter sauren Reaktionsbedingungen (z.B. Salzsäure, Aceton; p-Toluolsulfonsäure, Methanol; Oxalsäure, Silicagel), wobei Verbindungen der allgemeinen Formel **XXXI** erhalten werden.
Die Ketone **XXXI** werden mit einer Base deprotoniert (z.B. Lithiumdiisopropylamid, Lithium- oder Natriumhexamethyldisilazid) und bei tiefer Temperatur mit dem Aldehyd der allgemeinen Formel **VI** umgesetzt, wobei Verbindungen der allgemeinen Formel **XXXII** (X=OH) anfallen.

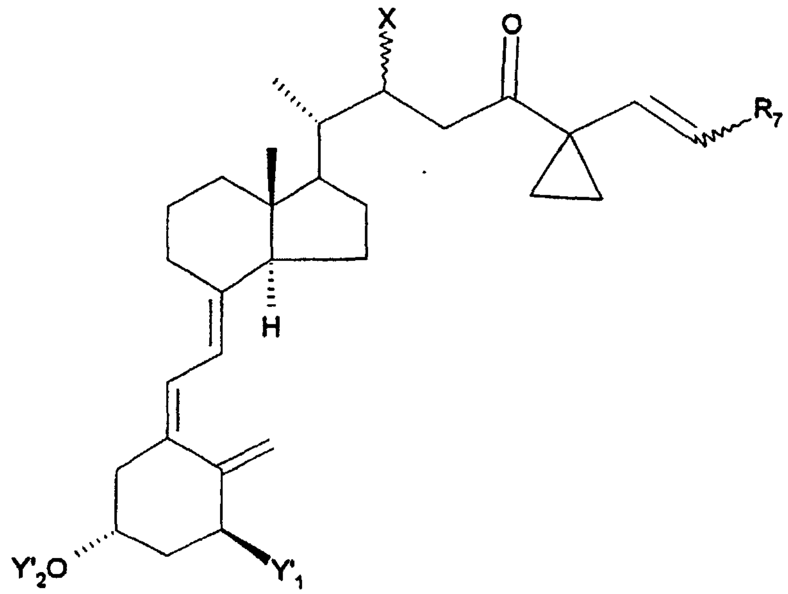

(XXXII)

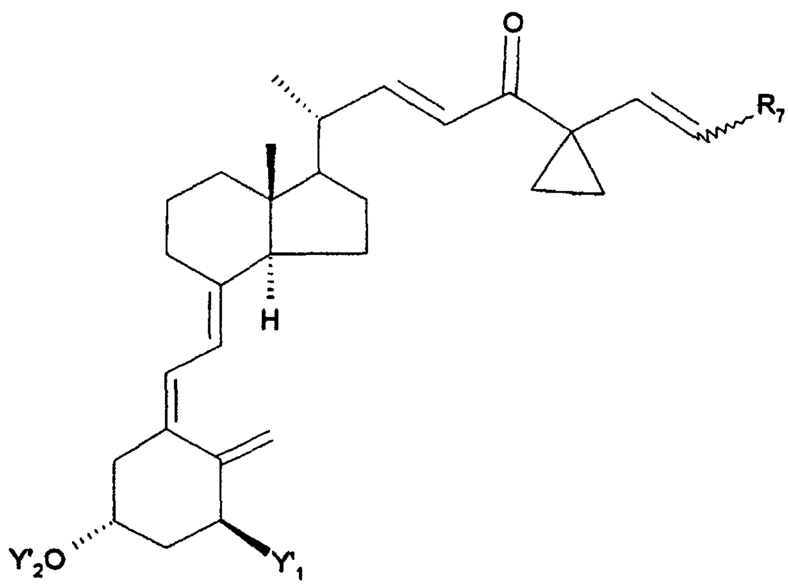

(XXXIII)

Die Hydroxylgruppe wird anschließend unter Standardbedingungen in eine Fluchtgruppe überführt, wobei Verbindungen der allgemeinen Formel **XXXII** (X= z.B. Acetat, Trifluoracetat, Tosylat, Mesylat oder Triflat) entstehen.

Eliminierung unter Einsatz von Basen (z.B. Diazabicyclononan, Diazabicycloundecan, Triethylamin, Diisopropylamin, Ethyldiisopropylamin) bei gegebenfalls erhöhten Reaktionstemperaturen liefert die Ketone der allgemeinen Formel **XXXIII**.

Die Carbonylgruppen in **XXXIII** können nun zu den diastereomeren Alkoholen **XXXIV** reduziert (Reduktionsmittel: z.B. Natriumborhydrid/Certrichlorid, Lithiumaluminium-hydrid, Diisobutylaluminiumhydrid, Lithiumborhydrid) und chromatographisch getrennt werden.

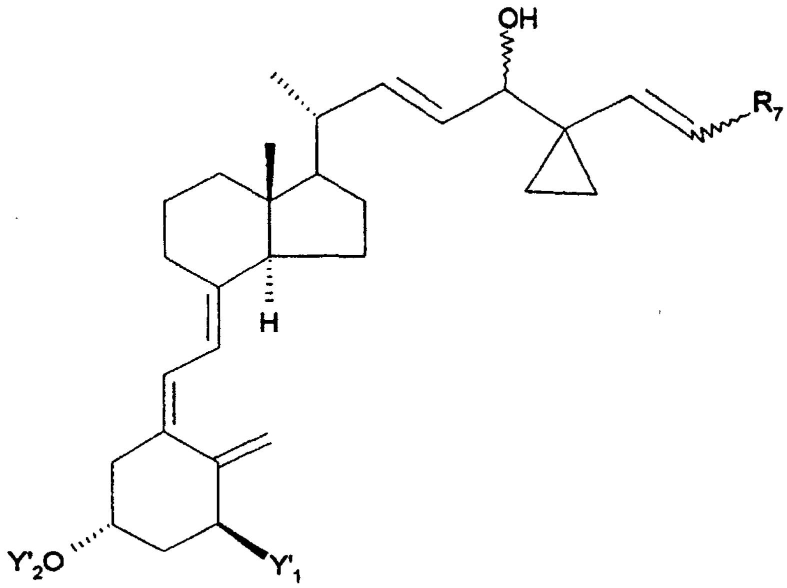

(XXXIV)

Die Verbindung der allgemeinen Formel **XXXIV** kann als Spezialfall der allgemeinen Formel **II** betrachtet werden und wird wie beschrieben in die Verbindung der allgemeinen Formel **I** überführt.

Daneben kann die Doppelbindung der Verbindung der allgemeinen Formel **XXX** hydriert werden, wobei eine Verbindung der allgemeinen Formel **XXXV** anfällt. Als Katalysatoren sind hier lösliche Rhodium-Katalysatoren (Wilkinson-Katalysator) oder Platin(VI)oxid vorzuziehen.

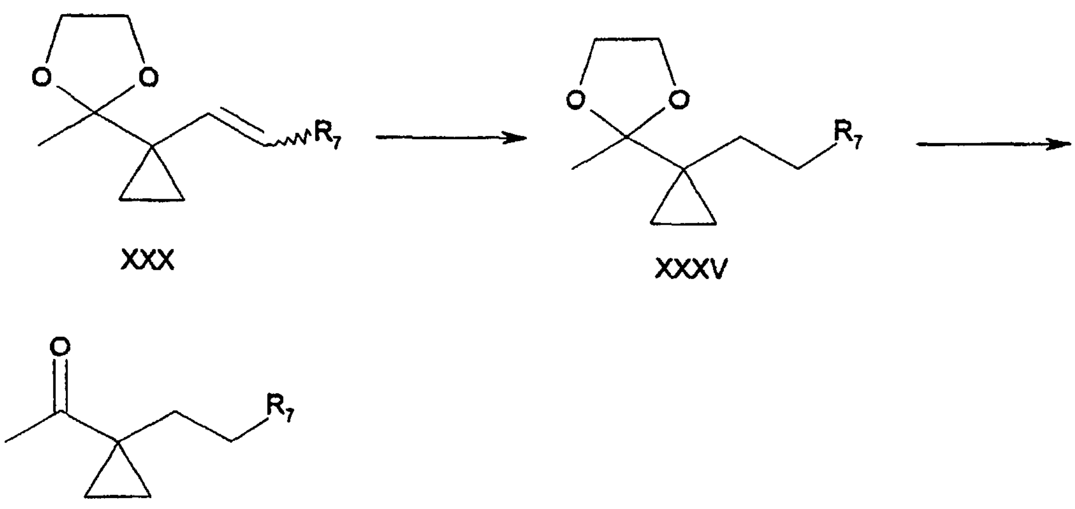

XXX

XXXV

XXXVI

Die Spaltung des Ketals zum Keton **XXXVI** und die Anknüpfung an das Vitamin D-Gerüst erfolgen wie vorstehend beschrieben, so daß letztlich eine Verbindung der allgemeinen Formel **XXXVII**, welche als Spezialfall der allgemeinen Formel **II** zu betrachten ist, entsteht.

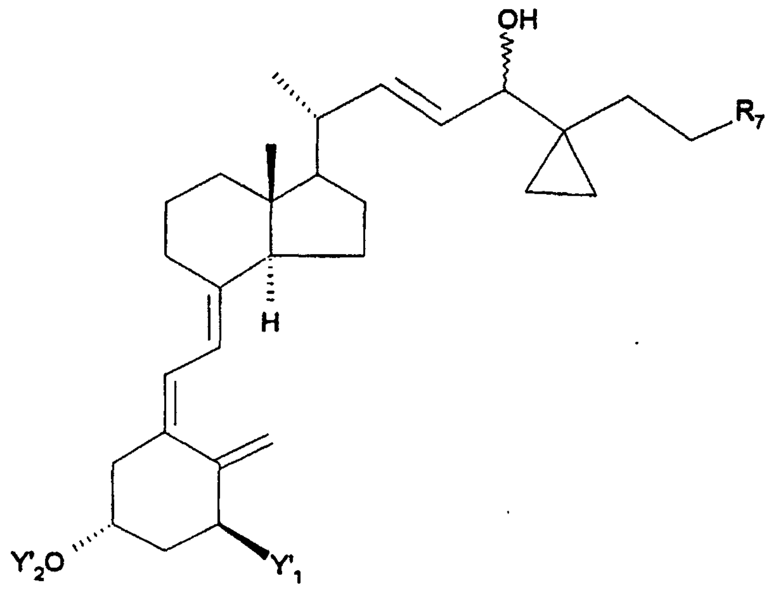

(XXXVII)

Die Überführung in eine Verbindung der allgemeinen Formel **I** erfolgt wie beschrieben.

Zur Synthese von Verbindungen der allgemeinen Formel **II** für die $R_1$ und $R_2$ Wasserstoffatome sind, muß ein konvergenter Syntheseweg beschritten werden, bei dem CD- und A-Ring-Fragmente separat aufgebaut werden. Zur Synthese der CD-Fragmente wird der literaturbekannte Aldehyd **XXXVIII** [H.H. Inhoffen et al. *Chem. Ber.* **92,** 781-791 (1958); H.H. Inhoffen et al. *Chem. Ber.* **92,** 1772-1788 (1959); W.G. Dauben et al. *Tetrahedron Lett.* **30,** 677-680 (1989)] verwendet,

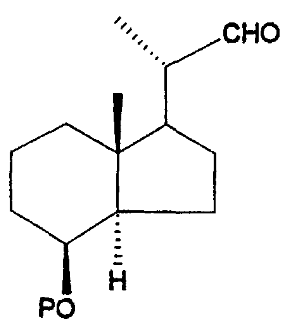

(XXXVIII)

worin P eine acyl-, alkyl- oder arylsubstituierte Silyl- oder eine Tetrahydropyranyl-, Tetrahydrofuranyl-, Methoxymethyl-, Ethoxyethyl-, eine Acylgruppe (z.B. Acetyl-, Benzoylgruppe) oder eine andere Hydroxyschutzgruppe bedeutet (siehe T.W. Greene, P.G.M. Wuts *Protective Groups in Organic Synthesis*, 2nd Edition, John Wiley and Sons, Inc. 1991).

Nach den bekannten Verfahren können hier die schon beschriebenen Modifikationen an C-20 eingeführt werden (WO 94/07853), wobei eine Verbindung der allgemeinen Formel **XXXIX** anfällt.

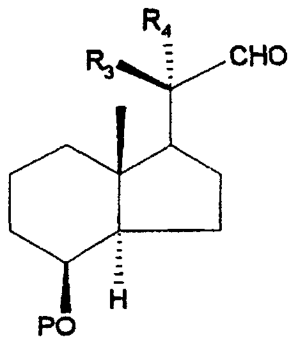

(XXXIX)

Die Einführung der Seitenketten erfolgt hier in Analogie zum Fall des Vitamin D-Aldehydes **XII,** wobei man Verbindungen der allgemeinen Formel **XL** erhält.

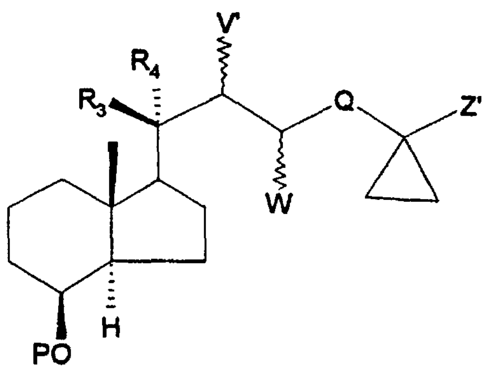

(XL)

V' bedeutet eine geschützte Hydroxylgruppe oder zusammen mit W eine *E*-Doppelbindung. Die übrigen Variablen wurden vorher bereits definiert.

Bei der Wahl geeigneter Schutzgruppen (z.B. P=Triethylsilyl, V'=Tetrahydropyranoxy) wird P selektiv gespalten (z.B. mit Tetrabutylammoniumfluorid), wobei die Verbindung der allgemeinen Formel **XLI** anfällt.

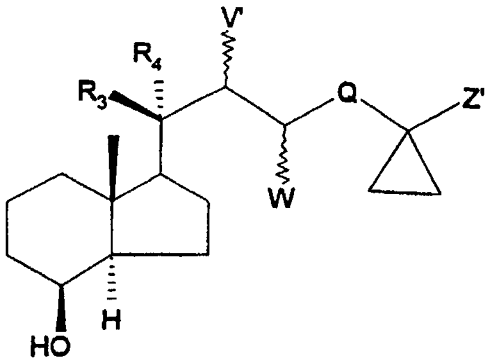

XLI

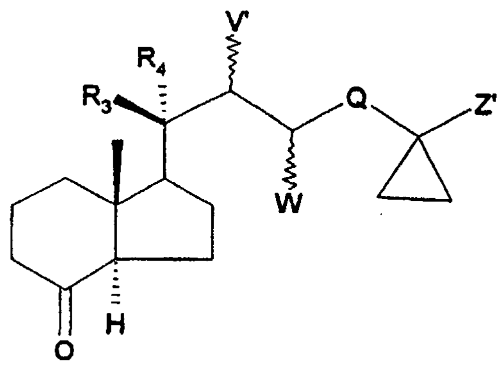

XLII

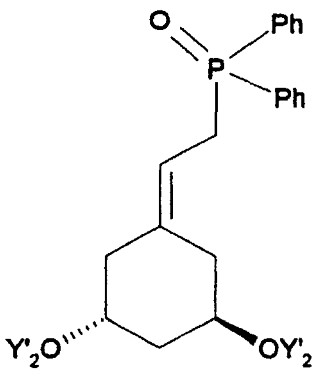

XLIII

Oxidation nach bekannter Methode (z.B. Pyridiniumchlorochromat, Pyridiniumdichromat, Swem-Bedingungen) ergeben eine Verbindung der allgemeinen Formel **XLII**, die durch Reaktion mit dem durch eine Base (z.B. Lithiumdiisopropylamid, *n*-Butyllithium) erzeugten Anion des literaturbekannten Phosphinoxides der allgemeinen Formel **XLIII** [H.F. DeLuca et al. *Tetrahedron Lett.* 32, 7663-7666 (1991)], worin $Y'_2$ die schon beschriebene Bedeutung hat, in entsprechende Verbindungen der allgemeinen Formel **II** für die gilt: $Y'_1$=O$Y'_2$ überführt wird. Die weitere Umsetzung in die Verbindung der allgemeinen Formel **I** erfolgt wie schon beschrieben.

Die nachstehenden Beispiele dienen der näheren Erläuterung der Erfindung.

**Synthese der Ausgangsverbindungen in der 24-Methylen-Reihe**

1. (5*Z*,7*E*)-(1*S*,3*R*)-1,3-Bis[[dimethyl(1,1-dimethylethyl)silyl]oxy]-9,10-secopregna-5,7,10(19)-trien-20-carbaldehyd **2**

Man löst 7,5 g (5*E*,7*E*)-(1*S*,3*R*)-1,3-Bis[[dimethyl(1,1-dimethylethyl)silyl]oxy]-9,10-secopregna-5,7,10(19)-trien-20-carbaldehyd **1** [M.J. Calverley *Tetrahedron* 43, 4609-4619 (1987)] in 200 ml Toluol, fügt 2 g Anthracen und 0,5 ml Triethylamin hinzu und bestrahlt unter Stickstoffdurchleitung in einer Pyrex-Apparatur mit einer Quecksilber-hochdrucklampe für 30 min. Anschließend filtriert man, engt ein und chromatographiert den Rückstand an Silicagel mit Essigester/Hexan, wobei man 7,1 g der Titelverbindung **2** als farblosen Schaum erhält.

$^1$H-NMR (300 MHz, $CDCl_3$): $\delta$= 0,05 ppm (s, 12H); 0,55 (s, 3H); 0,88 (s, 18H); 1,11 (d, 3H); 2,37 (m, 1H); 4,18 (m, 1H); 4,37 (m, 1H); 4,84 (brs, 1H); 5,17 (brs, 1H); 6,00 (d, 1H); 6,22 (d, 1H); 9,58 (d, 1H)

2. 1-Acetylcyclopropancarbonsäuremethylester **4**

Man löst 56 g Acetessigsäuremethylester **3** in 500 ml Aceton und fügt unter Eiskühlung 276,2 g Kaliumcarbonat sowie 86 ml Dibromethan hinzu. Man erhitzt unter Stickstoff auf 50°C und rührt 72 h bei dieser Temperatur. Nach dem Abkühlen wird das Gemisch mit Essigester verdünnt, mit Wasser und gesättigter Natriumchlorid-Lösung gewaschen, über Natriumsulfat getrocknet und eingeengt. Der Rückstand wird im Vakuum destilliert, wobei 66 g der Titelverbindung als farbloses Öl erhalten werden.
$^1$H-NMR (300 MHz, $CDCl_3$): δ= 1,48 ppm (s, 4H); 2,47 (s, 3H); 3,74 (s, 3H)

3. 1-(2-Methyl-1,3-dioxolan-2-yl)cyclopropancarbonsäuremethylester **5**

Man löst 18,7 g **4** in 500 ml Benzol, gibt 30 ml Ethylenglykol und 1 g p-Toluolsulfonsäure hinzu und erhitzt unter Stickstoff am Wasserabscheider für 12 h. Nach dem Abkühlen wird Natriumhydrogencarbonat-Lösung zugegeben, mit Essigester extrahiert, über Natriumsulfat getrocknet und eingeengt, wobei 23g der Titelverbindung **5** als farbloses Öl anfallen.
$^1$H-NMR (300 MHz, $CDCl_3$): δ= 1,02 ppm (m, 2H); 1,16 (m, 2H); 1,61 (s, 3H); 3,69 (s, 3H); 3,92 (m, 4H)

4. 1-(2-Methyl-1,3-dioxolan-2-yl)cyclopropanmethanol **6**

Man löst 11,2 g **5** in 150 ml Toluol und kühlt unter Stickstoff auf 0°C ab. Nun tropft man langsam 125 ml DIBAH-Lösung (1.2 M in Toluol) hinzu und rührt 2 h nach. Anschließend werden 1,25 ml Isopropanol und 25 ml Wasser zugetropft und über Nacht gerührt. Der Niederschlag wird abfiltriert, das Filtrat eingeengt und an Silicagel mit Essigester/Hexan chromatographiert, wobei 9,1 g der Titelverbinbdung **6** als farbloses Öl anfallen.
$^1$H-NMR (300 MHz, $CDCl_3$): δ= 0,47 ppm (m, 2H); 0,72 (m, 2H); 1,41 (s, 3H); 2,92 (t, OH); 3,53 (d, 2H); 3,97 (m, 4H)

5. 1-(2-Methyl-1,3-dioxolan-2-yl)cyclopropancarbaldehyd **7**

Man löst 10 g **6** in 500 ml Dichlormethan und gibt bei Raumtemperatur unter Stickstoff 3,7 g Natriumacetat und 19,3 g Pyridiniumchlorochromat zu. Nach 3 h bei Raumtemperatur wird über Silicagel filtriert, eingeengt, mit Diethylether verdünnt, erneut filtriert und das Solvens entfernt, wobei man 7,8 g der Titelverbindung **7** als farbloses Öl erhält.
$^1$H-NMR (300 MHz, $CDCl_3$): δ= 1,16 ppm (m, 4H); 1,57 (s, 3H); 3,97 (m, 3H); 9,49 (s, 1H)

6. 2-(1-Ethenylcyclopropyl)-2-methyl-1,3-dioxolan **8**

Es werden 44,6 g Methyltriphenylphosphoniumbromid in 700 ml Diethylether vorgelegt und bei 0°C unter Stickstoff werden 50 ml *n*-Butyiiithium-Lösung (2.5 M in Hexan) zugetropft. Man rührt 1 h bei Raumtemperatur nach und gibt dann 9,5 g **7** in 10 ml Diethylether hinzu. Nach 1 h quencht man die Reaktionslösung mit Natriumchlorid-Lösung, extrahiert mit Essigester, wäscht mit Natriumchlorid-Lösung, trocknet über Natriumsulfat und entfernt das Solvens. Der Rückstand wird an Silicagel mit Essigester/Hexan chromatographiert, wobei man 5,4 g der Titelverbindung **8** als farbloses Öl erhält.
$^1$H-NMR (300 MHz, $CDCl_3$): δ= 0,59 ppm (m, 2H); 0,86 (m, 2H); 1,41 (s, 3H); 3,95 (m, 4H); 4,98 (d, 1H); 4,99 (d, 1H); 6,21 (dd, 1H)

7. 1-(2-Methyl-1,3-dioxolan-2-yl)cyclopropanethanol **9**

Man löst 5,4 g **8** in 200 ml Tetrahydrofuran (THF) und tropft bei 0°C unter Stickstoff 14 ml Boran-THF-Lösung (1.0 M in THF) zu. Man läßt das Reaktionsgemisch dann auf Raumtemperatur kommen und rührt 2 h nach. Nach erneuter Kühlung auf 0°C werden nacheinander 17 ml Wasser, 17 ml wässrige Natriumhydroxid-Lösung (25%) und 2 ml Wasserstoffperoxid (30%) zugegeben und 1 h nachgerührt. Anschließend gibt man Natriumthiosulfat-Lösung zu, extrahiert mit Essigester, wäscht mit Natriumchlorid-Lösung, trocknet über Natriumsulfat und engt ein. Der Rückstand wird an Silicagel mit Essigester/Hexan chromatographiert, wobei man 3,9 g der Titelverbindung **9** als farbloses Öl erhält.
$^1$H-NMR (300 MHz, $CDCl_3$): δ= 0,29 ppm (m, 2H); 0,73 (m, 2H); 1,43 (s, 3H); 1,63 (t, 2H); 3,52 (t, OH); 3,79 (q, 2H); 3,93 (m, 4H)

8. 4-Methylbenzolsulfonsäure 2-[1-(2-methyl-1,3-dioxolan-2-yl)cyclopropyl]ethylester **10**

Es werden 470 mg **9** in 10 ml Pyridin gelöst und bei 0°C wird unter Stickstoff p-Toluolsulfonylchlorid zugegeben. Man rührt 1 h bei 0°C und gibt dann Natriumhydrogencarbonat-Lösung hinzu. Es wird mit Essigester extrahiert, mit verdünnter Salzsäure und anschließend mit Natriumhydrogencarbonat-Lösung gewaschen, über Natriumsulfat getrocknet und eingeengt. Nach Chromatographie des Rückstandes an Silicagel mit Essigester/Hexan verbleiben 670 mg der Titelverbindung als farbloses Öl.
$^1$H-NMR (300 MHz, $CDCl_3$): δ= 0,27 ppm (m, 2H); 0,61 (m, 2H); 1,29 (s, 3H); 1,78 (t, 2H); 2,46 (s, 3H); 3,80 (m, 4H); 4,23 (t, 2H); 7,35 (d, 2H); 7,81 (d, 2H)

9. 2-Methyl-2-[1-[2-(phenylsulfanyl)ethyl]cyclopropyl]-1,3-dioxolan **11**

590 mg **10** werden in 2 ml Dimethylformamid (DMF) gelöst und unter Stickstoff zu einer Mischung aus 300 mg Kalium-*t*-Butylat und 0,27 ml Thiophenol in 5 ml DMF gegeben. Man rührt 1 h bei Raumtemperatur und quencht dann mit Natriumchlorid-Lösung. Es wird mit Esigester extrahiert, mit Natriumhydrogencarbonat-Lösung und Natriumchlorid-Lösung gewaschen, über Natriumsulfat getrocknet, eingeengt und der Rückstand an Silicagel mit Essigester/Hexan chromatographiert, wobei 630 mg der Titelverbindung **11** als gelbliches Öl anfallen.
$^1$H-NMR (300 MHz, $CDCl_3$): δ= 0,29 ppm (m, 2H); 0,68 (m, 2H); 1,38 (s, 3H); 1,75 (t, 2H); 3,10 (m, 2H); 3,92 (m, 4H); 7,28 (d, 5H)

10. 2-Methyl-2-[1-[2-(phenylsulfonyl)ethyl]cyclopropyl]-1,3-dioxolan **12**

Man löst 480 mg **11** in 18 ml Methanol und gibt unter Stickstoff 180 mg Kaliumcarbonat, 221 mg Acetonitril und 612 mg Wasserstoffperoxid zu und rührt 6 h bei Raumtemperatur. Nun wird Natriumthiosulfat-Lösung zugegeben, mit Essigester extrahiert, mit Natriumchlorid-Lösung gewaschen, über Natriumsulfat getrocknet, eingeengt und der Rückstand über Silicagel mit Essigester/Hexan chromatographiert, wobei man 380 mg der Titelverbindung **12** als farbloses Öl erhält.
$^1$H-NMR (300 MHz, $CDCl_3$): δ= 0,25 ppm (m, 2H); 0,68 (m, 2H); 1,23 (s, 3H); 1,78 (m, 2H); 3,39 (m, 2H); 3,82 (m, 4H); 7,57 (t, 2H); 7,66 (t, 1H); 7,90 (d, 2H)

11. 3-Oxohexansäuremethylester **13**

Man legt 44,4 g Natriumhydrid-Suspension (60% in Paraffinöl) in 1500 ml THF vor und gibt unter Stickstoff bei 0°C 107,5 ml Acetessigsäuremethylester **3** hinzu. Nach 10 min tropft man dann 440 ml *n*-Butyllithium-Lösung (2.5 M in Hexan) zu und rührt weitere 30 min bei 0°C. Nun werden 88,9 ml lodethan zugegeben und es wird über Nacht bei Raumtemperatur nachgerührt. Zur Aufarbeitung wird wieder auf 0°C gekühlt und mit 4 N Salzsäure neutralisiert. Die organische Phase wird mit Essigester verdünnt, mit Thiosulfat-Lösung und Natriumchlorid-Lösung gewaschen, über Natriumsulfat getrocknet und eingeengt. Der Rückstand wird dann an Silicagel mit Essigester/Hexan chromatographiert, wobei 95,13 g der Titelverbindung **13** als farbloses Öl erhalten werden.
$^1$H-NMR (300 MHz, $CDCl_3$): δ= 0,93 ppm (t, 3H); 1,64 (quint, 2H); 2,52 (t, 2H); 3,46 (s, 2H); 3,75 (s, 3H)

12. 1-Oxobutylcyclopropancarbonsäuremethylester **14**

Man setzt 95 g **13** analog 2, um und erhält 110 g der Titelverbindung **14** als farbloses Öl.
$^1$H-NMR (300 MHz, $CDCl_3$): δ= 0,91 ppm (t, 3H); 1,43 (s, 4H); 1,61 (quint, 2H); 2,81 (t, 2H); 3,75 (s, 3H)

13. 1-(2-Propyl-1,3-dioxolan-2-yl)cyclopropancarbonsäuremethylester **15**

Man setzt 113 g **14** analog 3. um und erhält 78 g der Titelverbindung **15** als farbloses Öl.
$^1$H-NMR (300 MHz, $CDCl_3$): δ= 0,91 ppm (t, 3H); 1,00 (m, 2H); 1,04 (m, 2H); 1,40 (m, 2H); 2,04 (m, 2H); 3,68 (s, 3H); 3,93-3,98 (m, 4H)

14. 1-(2-Propyl-1,3-dioxolan-2-yl)cyclopropanmethanol **16**

Man setzt 52 g **15** analog 4. um und erhält 41,50 g der Titelverbindung **16** als farbloses Öl.
$^1$H-NMR (300 MHz, $CDCl_3$): δ= 0,40 ppm (m, 2H); 0,68 (m, 2H); 0,93 (t, 3H); 1,40 (m, 2H); 1,84 (m, 2H); 2,98 (t, OH); 3,50 (d, 2H); 3,98 (m, 4H)

15. 1-(2-Propyl-1,3-dioxolan-2-yl)cyclopropancarbaldehyd **17**

Man setzt 25,65 g **16** analog 5. um und erhält 19,62 g der Titelverbindung **17** als farbloses Öl.
$^1$H-NMR (300 MHz, $CDCl_3$): δ= 0,92 ppm (t, 3H); 1,10 (s, 4H); 1,42 (m, 2H); 1,90 (m, 2H); 3,98 (m, 4H); 9,58 (s, 1H)

16. 2-(1-Ethenylcyclopropyl)-2-propyl-1,3-dioxolan **18**

Man setzt 4,2 g **17** analog 6. um und erhält 4,15 g der Titelverbindung **18** als farbloses Öl.
$^1$H-NMR (300 MHz, $CDCl_3$): δ= 0,54 ppm (m, 2H); 0,80 (m, 2H); 0,90 (t, 3H); 1,40 (m, 2H); 1,75 (m, 2H); 3,96 (m, 4H); 4,94 (d, 1H); 4,95 (d, 1H); 6,23 (dd, 1H)

17. 1-(2-Propyl-1,3-dioxolan-2-yl)cyclopropanethanol **19**

Man setzt 4,15 g **18** analog 7. um und erhält 2,71 g der Titelverbindung **19** als farbloses Öl. $^1$H-NMR (300 MHz, $CDCl_3$): δ= 0,22 ppm (m, 2H); 0,69 (m, 2H); 0,94 (t, 3H); 3,65 (t, OH); 3,70 (m, 2H); 3,94 (m, 4H)

18. 4-Methylbenzolsulfonsäure 2-[1-(2-propyl-1,3-dioxolan-2-yl)cyclopropyl]ethylester **20**

Man setzt 1,85 g **19** analog 8. um und erhält 1,41 g der Titelverbindung **20** als farbloses Öl. $^1$H-NMR (300 MHz, $CDCl_3$): δ= 0,20 ppm (m, 2H); 0,59 (m, 2H); 0,87 (t, 3H); 1,70 (t, 2H); 2,45 (s, 3H); 3,80 (m, 4H); 4,26 (t, 2H); 7,35 (d, 2H); 7,80 (d, 2H)

19. 2-[1-[2-(Phenylsulfanyl)ethyl]cyclopropyl]-2-propyl-1,3-dioxolan **21**

Man setzt 1,41 g **20** analog 9. um und erhält 980 mg der Titelverbindung **21** als farbloses Öl. $^1$H-NMR (300 MHz, $CDCl_3$): δ= 0,23 ppm (m, 2H); 0,65 (m, 2H); 0,92 (t, 3H); 1,75 (t, 2H); 3,12 (t, 2H); 3,94 (m, 4H); 7,30 (m, 5H)

20. 2-[1-[2-(Phenylsulfonyl)ethyl]cyclopropyl]-2-propyl-1,3-dioxolan **22**

Man setzt 910 mg **21** analog 10. um und erhält 722 mg der Titelverbindung **22** als farbloses Öl. $^1$H-NMR (300 MHz, $CDCl_3$): δ= 0,18 ppm (m, 2H); 0,62 (m, 2H); 0,86 (t, 3H); 1,32 (m, 2H); 1,58 (m, 2H); 1,72 (m, 2H); 3,40 (m, 2H); 3,81 (m, 4H); 7,57 (t, 2H); 7,65 (t, 1H); 7,90 (d, 2H)

21. 3-Oxoheptansäuremethylester **23**

Man setzt 118 ml Acetessigsäuremethylester **3** mit *n*-Iodpropan analog 11. um und erhält 135 g der Titelverbindung **23** als farbloses Öl.
$^1$H-NMR (300 MHz, $CDCl_3$): δ= 0,91 ppm (t, 3H); 1,32 (hex, 2H); 1,59 (quint, 2H); 2,53 (t, 2H); 3,47 (s, 2H); 3,75 (s, 3H)

22. 1-Oxopentylcyclopropancarbonsäuremethylester **24**

Man setzt 135 g **23** analog 2. um und erhält 123 g der Titelverbindung **24** als farbloses Öl. $^1$H-NMR (300 MHz, $CDCl_3$): δ= 0,90 ppm (t, 3H); 1,30 (hex, 2H); 1,42 (s, 4H); 1,58 (quint, 2H); 2,83 (t, 2H); 3,72 (s, 3H)

23. 1-(2-Butyl-1,3-dioxolan-2-yl)cyclopropancarbonsäuremethylester **25**

Man setzt 59 g **24** analog 3. um und erhält 45 g der Titelverbindung **25** als farbloses Öl. $^1$H-NMR (300 MHz, $CDCl_3$): δ= 0,89 ppm (t, 3H); 1,00 (m, 2H); 1,12 (m, 2H); 1,30 (m, 4H); 2,05 (m, 2H); 3,68 (s, 3H); 3,96 (m, 4H)

24. 1-(2-Butyl-1,3-dioxolan-2-yl)cyclopropanmethanol **26**

Man setzt 25 g **25** analog 4. um und erhält 16,8 g der Titelverbindung **26** als farbloses Öl. $^1$H-NMR (300 MHz, $CDCl_3$): δ= 0,40 ppm (m, 2H); 0,69 (m, 2H); 0,89 (t, 3H); 1,32 (m, 4H); 1,84 (m, 2H); 3,02 (brs, OH); 3,50 (brs, 2H); 3,97 (m, 4H)

25.1-(2-Butyl-1,3-dioxolan-2-yl)cyclopropancarbaldehyd **27**

Man setzt 16,3 g **26** analog 5. um und erhält 16,0 g der Titelverbindung **27** als farbloses Öl. $^1$H-NMR (300 MHz, $CDCl_3$): δ= 0,90 ppm (t, 3H); 1,10 (s, 4H); 1,33 (m, 4H); 1,90 (m, 2H); 3,98 (m, 4H); 9,58 (s, 1H)

26. 2-Butyl-2-(1-ethenylcyclopropyl)-1,3-dioxolan **28**

Man setzt 5,0 g **27** analog 6. um und erhält 3,89 g der Titelverbindung **28** als farbloses Öl. $^1$H-NMR (300 MHz, $CDCl_3$): δ= 0,54 ppm (m, 2H); 0,80 (m, 2H); 0,90 (t, 3H); 1,30 (m, 4H); 1,79 (m, 2H); 3,94 (m, 4H); 4,94 (d, 1H); 4,95 (d, 1H); 6,22 (dd, 1H)

27. 1-(2-Butyl-1,3-dioxolan-2-yl)cyclopropanethanol **29**

Man setzt 1,51 g **28** analog 7. um und erhält 1,10 g der Titelverbindung **29** als farbloses Öl. $^1$H-NMR (300 MHz, $CDCl_3$): δ= 0,22 ppm (m, 2H); 0,69 (m, 2H); 0,90 (t, 3H); 1,31 (m, 4H); 1,60 (t, 2H); 1,83 (m, 2H); 3,63 (brs, OH); 3,70 (m, 2H); 3,94 (m, 4H)

28. 4-Methylbenzolsulfonsäure 2-[1-(2-butyl-1,3-dioxolan-2-yl)cyclopropyl]ethylester **30**

Man setzt 1,10 g **29** analog 8. um und erhält 1,05 g der Titelverbindung **30** als farbloses Öl. $^1$H-NMR (300 MHz, $CDCl_3$): δ= 0,20 ppm (m, 2H); 0,60 (m, 2H); 0,89 (t, 3H); 1,70 (t, 2H); 2,47 (s, 3H); 3,80 (m, 4H); 4,24 (t, 2H); 7,33 (d, 2H); 7,79 (d, 2H)

29. 2-Butyl-2-[1-[2-(phenylsulfanyl)ethyl]cyclopropyl]-1,3-dioxolan **31**

Man setzt 1,05 g **30** analog 9. um und erhält 675 mg der Titelverbindung **31** als farbloses Öl. $^1$H-NMR (300 MHz, $CDCl_3$): δ= 0,23 ppm (m, 2H); 0,64 (m, 2H); 0,90 (t, 3H); 1,30 (m, 4H); 1,72 (m, 4H); 3,11 (m, 2H); 3,91 (m, 4H); 7,28 (m, 5H)

30. 2-Butyl-2-[1-[2-(phenylsulfonyl)ethyl]cyclopropyl]-1,3-dioxolan **32**

Man setzt 1,15 g **31** analog 10. um und erhält 913 mg der Titelverbindung **32** als farbloses Öl. $^1$H-NMR (300 MHz, $CDCl_3$): δ= 0.19 ppm (m, 2H); 0,64 (m, 2H); 0,87 (t, 3H); 1,28 (m, 6H); 1,72 (m, 2H); 3,40 (m, 2H); 3,82 (m, 4H); 7,58 (t, 2H); 7,68 (t, 1H); 7,92 (d, 2H)

31. 3-Oxooctansäuremethylester **33**

Man setzt 23 g Acetessigsäuremethylester **3** mit *n*-Iodbutan analog 11. um und erhält 29,4 g der Titelverbindung **33** als farbloses Öl.
$^1$H-NMR (300 MHz, $CDCl_3$): δ= 0,90 ppm (t, 3H); 1,29 (m, 4H); 1,60 (m, 2H); 2,52 (t, 2H); 3,46 (s, 2H); 3,73 (s, 3H)

32. 1-Oxohexylcyclopropancarbonsäuremethylester **34**

Man setzt 18,3 g **33** analog 2. um und erhält 15,2 g der Titelverbindung **34** als farbloses Öl. $^1$H-NMR (300 MHz, $CDCl_3$): δ= 0,89 ppm (t, 3H); 1,30 (m, 4H); 1,45 (s, 4H); 1,60 (m, 2H); 2,81 (t, 2H); 3,75 (s, 3H)

33. 1-(2-Pentyl-1,3-dioxolan-2-yl)cyclopropancarbonsäuremethylester **35**

Man setzt 15,1 g **34** analog 3. um und erhält 13,2 g der Titelverbindung **35** als farbloses Öl. $^1$H-NMR (300 MHz, $CDCl_3$): δ= 0,89 ppm (t, 3H); 1,00 (m, 2H); 1,14 (m, 2H); 1,30 (m, 6H); 2,05 (m, 2H); 3,69 (s, 3H); 3,92 (m, 4H)

34. 1-(2-Pentyl-1,3-dioxolan-2-yl)cyclopropanmethanol **36**

Man setzt 10,0 g **35** analog 4. um und erhält 7,3 g der Titelverbindung **36** als farbloses Öl. $^1$H-NMR (300 MHz, $CDCl_3$): δ= 0,40 ppm (m, 2H); 0,69 (m, 2H); 0,90 (t, 3H); 1,33 (m, 6H); 1,85 (m, 2H); 3,01 (t, OH); 3,50 (d, 2H); 3,97 (m, 4H)

35. 1-(2-Pentyl-1,3-dioxolan-2-yl)cyclopropancarbaldehyd **37**

Man setzt 7,3 g **36** analog 5. um und erhält 5,9 g der Titelverbindung **37** als farbloses Öl. $^1$H-NMR (300 MHz, $CDCl_3$): δ= 0,89 ppm (t, 3H); 1,10 (s, 4H); 1,24-1,47 (m, 6H); 1,90 (m, 2H); 3,98 (m, 4H); 9,58 (s, 1H)

36. 2-(1-Ethenylcyclopropyl)-2-pentyl-1,3-dioxolan **38**

Man setzt 5,3 g **37** analog 6. um und erhält 1,98 g der Titelverbindung **38** als farbloses Öl. $^1$H-NMR (300 MHz, $CDCl_3$): δ= 0,53 ppm (m, 2H); 0,80 (m, 2H); 0,89 (t, 3H); 1,20-1,45 (m, 6H); 1,77 (m, 2H); 3,96 (m, 4H); 4,96 (d, 1H); 4,97 (d, 1H); 6,23 (dd, 1H)

37. 1-(2-Pentyl-1,3-dioxolan-2-yl)cyclopropanethanol **39**

Man setzt 1,70 g **38** analog 7. um und erhält 1,20 g der Titelverbindung **39** als farbloses Öl.
$^1$H-NMR (300 MHz, $CDCl_3$): δ= 0,23 ppm (m, 2H); 0,70 (m, 2H); 0.90 (t, 3H); 1,22-1,43 (m, 6H); 1,62 (m, 2H); 1,85 (m, 2H); 3,57 (brs, OH); 3,70 (m, 2H); 3,94 (m, 4H)

38. 4-Methylbenzolsulfonsäure 2-[1-(2-pentyl-1,3-dioxolan-2-yl)cyclopropyl]ethylester **40**

Man setzt 456 mg **39** analog 8. um und erhält 620 mg der Titelverbindung **40** als farbloses Öl.
$^1$H-NMR (300 MHz, $CDCl_3$): δ= 0,20 ppm (m, 2H); 0,60 (m, 2H); 0,90 (t, 3H); 1,29 (m, 6H); 1,63 (m, 2H); 1,72 (t, 2H); 2,47 (s, 3H); 3,80 (m, 4H); 4,25 (t, 2H); 7,35 (d, 2H); 7,80 (d, 2H)

39. 2-Pentyl-2-[1-[2-(phenylsulfanyl)ethyl]cyclopropyl]-1,3-dioxolan **41**

Man setzt 610 mg **40** analog 9. um und erhält 499 mg der Titelverbindung **41** als farbloses Öl.
$^1$H-NMR (300 MHz, $CDCl_3$): δ= 0,21 ppm (m, 2H); 0,63 (m, 2H); 0,89 (t, 3H); 1,19-1,42 (m, 6H); 1,72 (m, 4H); 3,10 (m, 2H); 3,90 (m, 4H); 7,30 (m, 5H)

40. 2-Pentyl-2-[1-[2-(phenylsulfonyl)ethyl]cyclopropyl]-1,3-dioxolan **42**

Man setzt 318 mg **41** analog 10. um und erhält 287 mg der Titelverbindung **42** als farbloses Öl.
$^1$H-NMR (300 MHz, $CDCl_3$): δ= 0,17 ppm (m, 2H); 0,62 (m, 2H); 0,88 (t, 3H); 1,23 (m, 6H); 1,58 (m, 2H); 1,70 (m, 2H); 3,39 (m, 2H); 3,83 (m, 4H); 7,59 (t, 2H); 7,67 (t, 1H); 7,92 (d, 2H)

41. 3-Oxononansäuremethylester **43**

Man setzt 53,5 ml Acetessigsäuremethylester **3** mit *n*-Iodpentan analog 11. um und erhält 87,9 g der Titelverbindung **43** als farbloses Öl.
$^1$H-NMR (300 MHz, $CDCl_3$): δ= 0,88 ppm (t, 3H); 1,29 (m, 6H); 1,60 (m, 2H); 2,53 (t, 2H); 3,45 (s, 2H); 3,74 (s, 3H)

42. 1-Oxoheptylcyclopropancarbonsäuremethylester **44**

Man setzt 87,9 g **43** analog 2. um und erhält 99,6 g der Titelverbindung **44** als farbloses Öl.
$^1$H-NMR (300 MHz, $CDCl_3$): δ= 0,88 ppm (t, 3H); 1,29 (m, 6H); 1,45 (s, 4H); 1,58 (m, 2H); 2,82 (t, 2H); 3,72 (s, 3H)

43. 1-(2-Hexyl-1,3-dioxolan-2-yl)cyclopropancarbonsäuremethylester **45**

Man setzt 102,4 g **44** analog 3. um und erhält 85,86 g der Titelverbindung **45** als farbloses Öl.
$^1$H-NMR (300 MHz, $CDCl_3$): δ= 0,89 ppm (t, 3H); 1,00 (m, 2H); 1,15 (m, 2H); 1,30 (m, 8H); 2,05 (m, 2H); 3,67 (s, 3H); 3,94 (m, 4H)

44. 1-(2-Hexyl-1,3-dioxolan-2-yl)cyclopropanmethanol **46**

Man setzt 63,45 g **45** analog **4.** um und erhält 51,92 g der Titelverbindung **46** als farbloses Öl.
$^1$H-NMR (300 MHz, $CDCl_3$): δ= 0,40 ppm (m, 2H); 0,70 (m, 2H); 0,90 (t, 3H); 1,32 (m, 8H); 1,87 (m, 2H); 3,01 (brs, OH); 3,51 (d, 2H); 3,97 (m, 4H)

45. 1-(2-Hexyl-1,3-dioxolan-2-yl)cyclopropancarbaldehyd **47**

Man setzt 10 g **46** analog 5. um und erhält 7,9 g der Titelverbindung **47** als farbloses Öl.
$^1$H-NMR (300 MHz, $CDCl_3$): δ= 0,89 ppm (t, 3H); 1,10 (s, 4H); 1,29 (m, 8H); 1,90 (m, 2H); 3,97 (m, 4H); 9,60 (s, 1H)

46. 2-(1-Ethenylcyclopropyl)-2-hexyl-1,3-dioxolan **48**

Man setzt 5,65 g **47** analog 6. um und erhält 4,9 g der Titelverbindung **48** als farbloses Öl.
$^1$H-NMR (300 MHz, $CDCl_3$): δ= 0,57 ppm (m, 2H); 0,80 (m, 2H); 0,89 (t, 3H); 1,32 (m, 8H); 1,78 (m, 2H); 3,95 (m, 4H); 4,98 (d, 1H); 4,99 (d, 1H); 6,23 (dd, 1H)

47. 1-(2-Hexyl-1,3-dioxolan-2-yl)cyclopropanethanol **49**

Man setzt 3,6 g **48** analog 7. um und erhält 2,9 g der Titelverbindung **49** als farbloses Öl.
$^1$H-NMR (300 MHz, $CDCl_3$): δ= 0,22 ppm (m, 2H); 0,69 (m, 2H); 0,89 (t, 3H); 1,30 (m, 8H); 1,60 (t, 2H); 1,85 (m, 2H); 3,57 (brs, OH); 3,69 (m, 2H); 3,95 (m, 4H)

48. 4-Methylbenzolsulfonsäure 2-[1-(2-hexyl-1,3-dioxolan-2-yl)cyclopropyl]ethylester **50**

Man setzt 2,1 g **49** analog 8. um und erhält 2,3 g der Titelverbindung **50** als farbloses Öl.
$^1$H-NMR (300 MHz, $CDCl_3$): δ= 0,22 ppm (m, 2H); 0,60 (m, 2H); 0,90 (t, 3H); 1,29 (m, 8H); 1,68 (m, 2H); 1,73 (t, 2H); 2,48 (s, 3H); 3,84 (m, 4H); 4,29 (t, 2H); 7,38 (d, 2H); 7,82 (d, 2H)

49. 2-Hexyl-2-[1-[2-(phenylsulfanyl)ethyl]cyclopropyl]-1,3-dioxolan **51**

Man setzt 2,3 g **50** analog 9. um und erhält 1,98 g der Titelverbindung **51** als farbloses Öl.
$^1$H-NMR (300 MHz, $CDCl_3$): δ= 0,20 ppm (m, 2H); 0,61 (m, 2H); 0,89 (t, 3H); 1,19-1,42 (m, 8H); 1,73 (m, 4H); 3,10 (m, 2H); 3,91 (m, 4H); 7,30 (m, 5H)

50. 2-Hexyl-2-[1-[2-(phenylsulfonyl)ethyl]cyclopropyl]-1,3-dioxolan **52**

Man setzt 1,98 g **51** analog 10. um und erhält 1,50 g der Titelverbindung **52** als farbloses Öl.
$^1$H-NMR (300 MHz, $CDCl_3$): δ= 0,18 ppm (m, 2H); 0,63 (m, 2H); 0,89 (t, 3H); 1,28 (m, 8H); 1,58 (m, 2H); 1,72 (m, 2H); 3,40 (m, 2H); 3,82 (m, 4H); 7,58 (t, 2H); 7,67 (t, 1H); 7,92 (d, 2H)

51. 3-Oxodecansäuremethylester **53**

Man setzt 30 ml Acetessigsäuremethylester **3** mit *n*-Iodhexan analog 11. um und erhält 57,8 g der Titelverbindung **53** als farbloses Öl.
$^1$H-NMR (300 MHz, $CDCl_3$): δ= 0,88 ppm (t, 3H); 1,29 (m, 6H); 1,60 (m, 2H); 2,53 (t, 2H); 3,45 (s, 2H); 3,74 (s, 3H)

52. 1-Oxooctylcyclopropancarbonsäuremethylester **54**

Man setzt 57,8 g **53** analog 2. um und erhält 62,6 g der Titelverbindung **54** als farbloses Öl.
$^1$H-NMR (300 MHz, $CDCl_3$): δ= 0,88 ppm (t, 3H); 1,30 (m, 10H); 1,40 (s, 4H); 2,82 (t, 2H); 3,68 (s, 3H)

53. 1-(2-Heptyl-1,3-dioxolan-2-yl)cyclopropancarbonsäuremethylester **55**

Man setzt 16,2 g **54** analog 3. um und erhält 13,9 g der Titelverbindung **55** als farbloses Öl.
$^1$H-NMR (300 MHz, $CDCl_3$): δ= 0,89 ppm (t, 3H); 1,00 (m, 2H); 1,12 (m, 2H); 1,30 (m, 10H); 2,05 (m, 2H); 3,68 (s, 3H); 3,93 (m, 4H)

54. 1-(2-Heptyl-1,3-dioxolan-2-yl)cyclopropanmethanol **56**

Man setzt 9,6 g **55** analog 4. um und erhält 7,9 g der Titelverbindung **56** als farbloses Öl.
$^1$H-NMR (300 MHz, $CDCl_3$): δ= 0,40 ppm (m, 2H); 0,70 (m, 2H); 0,90 (t, 3H); 1,30 (m, 10H); 1,87 (m, 2H); 3,00 (d, OH); 3,51 (d, 2H); 3,98 (m, 4H)

55. 1-(2-Heptyl-1,3-dioxolan-2-yl)cyclopropancarbaldehyd **57**

Man setzt 11,4 g **56** analog 5. um und erhält 7,6 g der Titelverbindung **57** als farbloses Öl.
$^1$H-NMR (300 MHz, $CDCl_3$): δ= 0,89 ppm (t, 3H); 1,10 (s, 4H); 1,30 (m, 10H); 1,90 (m, 2H); 3,98 (m, 4H); 9,58 (s, 1H)

56. 2-(1-Ethenylcyclopropyl)-2-heptyl-1,3-dioxolan **58**

Man setzt 3,2 g **57** analog 6. um und erhält 2,4 g der Titelverbindung **58** als farbloses Öl.
$^1$H-NMR (300 MHz, $CDCl_3$): δ= 0,54 ppm (m, 2H); 0,80 (m, 2H); 0,90 (t, 3H); 1,30 (m, 10H); 1,78 (m, 2H); 3,95 (m, 4H); 4,95 (d, 1H); 4,96 (d, 1H); 6,23 (dd, 1H)

57. 1-(2-Heptyl-1,3-dioxolan-2-yl)cyclopropanethanol **59**

Man setzt 2,4 g **58** analog 7. um und erhält 1,8 g der Titelverbindung **59** als farbloses Öl.
$^1$H-NMR (300 MHz, $CDCl_3$): δ= 0,23 ppm (m, 2H); 0,70 (m, 2H); 0,90 (t, 3H); 1,30 (m, 10H); 1,61 (t, 2H); 1,84 (m, 2H); 3,57 (brs, OH); 3,70 (m, 2H); 3,95 (m, 4H)

58. 4-Methylbenzolsulfonsäure 2-[1-(2-heptyl-1,3-dioxolan-2-yl)cyclopropyl]ethylester **60**

Man setzt 1,6 g **59** analog 8. um und erhält 1,34 g der Titelverbindung **60** als farbloses Öl.
$^1$H-NMR (300 MHz, $CDCl_3$): δ= 0,23 ppm (m, 2H); 0,59 (m, 2H); 0,89 (t, 3H); 1,30 (m, 10H); 1,68 (m, 2H); 1,75 (t, 2H); 2,47 (s, 3H); 3,86 (m, 4H); 4,30 (t, 3H); 7,37 (d, 2H); 7,79 (d, 2H)

59. 2-Heptyl-2-[1-[2-(phenylsulfanyl)ethyl]cyclopropyl]-1,3-dioxolan **61**

Man setzt 920 mg **60** analog 9. um und erhält 743 mg der Titelverbindung **61** als farbloses Öl.
$^1$H-NMR (300 MHz, $CDCl_3$): δ= 0,20 ppm (m, 2H); 0,58 (m, 2H); 0,89 (t, 3H); 1,30 (m, 10H); 1,72 (m, 4H); 3,08 (m, 2H); 3,91 (m, 4H); 7,30 (m, 5H)

60. 2-Heptyl-2-[1-[2-(phenylsulfonyl)ethyl]cyclopropyl]-1,3-dioxolan **62**

Man setzt 450 mg **61** analog 10. um und erhält 356 mg der Titelverbindung **62** als farbloses Öl.
$^1$H-NMR (300 MHz, $CDCl_3$): δ= 0,18 ppm (m, 2H); 0,63 (m, 2H); 0,90 (t, 3H); 1,27 (m, 10H); 1,58 (m, 2H); 1,71 (m, 2H); 3,39 (m, 2H); 3,82 (m, 4H); 7,60 (t, 2H); 7,68 (t, 1H); 7,92 (d, 2H)

**Beispiel 1**

(5*Z*,7*E*,22*E*)-(1*S*,3*R*)-25-Acetyl-26,27-cyclo-9,10-secocholesta-5,7,10(19),22-tetraen-1,3-diol **66**

61. Aus 0,18 ml Diisopropylamin und 0,57 ml *n*-Butyllithium-Lösung (2.5 M in Hexan) wird in 20 ml THF bei -78°C unter Stickstoff Lithiumdiisopropylamid bereitet. Dazu tropft man 380 mg des Sulfons **12** in 1 ml THF und rührt 30 min bei -78°C nach. Anschließend werden 200 mg des Aldehydes **2** in 0,5 ml THF zugegeben und es wird weitere 30 min nachgerührt. Man quencht mit Natriumchlorid-Lösung, extrahiert mit Diethylether, wäscht mit Natriumchlorid-Lösung, trocknet über Natriumsulfat und engt ein. Der Rückstand wird an Silicagel mit Essigester/Hexan chromatographiert, wobei 189 mg (5*Z*,7*E*)-(1*S*,3*R*)-1,3-Bis[[dimethyl(1,1-dimethylethyl)silyl]oxy]-25-(2-methyl-1,3-dioxolan-2-yl)-23-phenylsulfonyl-26,27-cyclo-9,10-secocholesta-5,7,10(19)-trien-22-ol **63** als farbloser Schaum anfallen.

62. Man legt 150 mg **63** in 5 ml Methanol unter Stickstoff vor und gibt 10 ml gesättigter, methanolischer Dinatriumhydrogenphosphat-Lösung zu und rührt 15 min bei Raumtemperatur. Anschließend werden 650 mg Natriumamalgam (5%) zugegeben und es wird 1 h nachgerührt. Man dekantiert vom entstandenen Quecksilber ab und extrahiert das Reaktionsgemisch mit Dichlormethan. Nach Waschen der organischen Phase mit Natriumchlorid-Lösung wird über Natriumsulfat getrocknet, das Solvens entfernt und der Rückstand an Silicagel mit Essigester/Hexan chromatographiert, wobei nacheinander 65 mg (5*Z*,7*E*,22*E*)-(1 *S*,3*R*)-1,3-Bis[[dimethyl(1,1-dimethylethyl)silyl]oxy]-25-(2-methyl-1,3-dioxolan-2-yl)-26,27-cyclo-9,10-secocholesta-5,7,10(19),22-tetraen **64** und 70 mg (5*Z*,7*E*)-(1 *S*,3*R*,22*S*)-1,3-Bis[[dimethyl(1,1-dimethylethyl)silyl]oxy]-25-(2-methyl-1,3-dioxolan-2-yl)-26,27-cyclo-9,10-secocholesta-5,7,10(19)-trien-22-ol **65** als farblose Schäume anfallen.
$^1$H-NMR (300 MHz, $CDCl_3$): **64:** δ= 0,03 ppm (s, 12H); 0,25 (m, 2H); 0,48 (m, 2H); 0,52 (s, 3H); 0,85 (s, 18H); 0,98 (d, 3H); 1,32 (s, 3H); 3,80 (m, 4H); 4,16 (m, 1H); 4,36 (m, 1H); 4,82 (s, 1H); 5,15 (s, 1H); 5,20 (m, 2H); 6,00 (d, 1H); 6,23 (d, 1H) **65:** δ= 0,04 ppm (s, 12H); 0,23 (m, 2H); 0,52 (s, 3H); 0,58 (m, 2H); 0,85 (d, 3H); 0,85 (s, 18H); 1,32 (s, 3H); 3,55 (m, 1H); 3,82 (m, 4H); 4,16 (m, 1H); 4,36 (m, 1H); 4,83 (s, 1H); 5,16 (s, 1H); 6,01 (d, 1H); 6,23 (d, 1H)

63. Man löst 35 mg **64** in 10 ml Dichlormethan/Methanol (1:9) und rührt unter Stickstoff bei Raumtemperatur mit 350 mg Dowex-Ionentauscher (aktiviert) für 48 h. Anschließend filtriert man, wäscht das Filtrat mit Natriumhydrogencarbonat-Lösung und Natriumchlorid-Lösung, trocknet über Natriumsulfat, engt ein und chromatographiert den Rückstand an Silicagel mit Essigester/Hexan, wobei 13 mg der Titelverbindung **66** als farbloser Schaum anfallen.
$^1$H-NMR (300 MHz, $CD_2Cl_2$): δ= 0,53 ppm (s, 3H); 0,78 (m, 2H); 1,01 (d, 3H); 1,20 (m, 2H); 2,10 (s, 3H); 4,19 (m, 1H); 4,39 (m, 1H); 4,98 (s, 1H); 5,30 (s, 1H); 5,32 (m, 2H); 6,01 (d, 1H); 6,38 (d, 1H)

**Beispiel 2**

64. (5*Z*,7*E*)-(1*S*,3*R*,22*S*)-25-Acetyl-26,27-cyclo-9,10-secocholesta-5,7,10(19)-trien-1,3,22-triol **67**

Man setzt 11 mg **65** in Analogie zu 63. um und erhält 4,4 mg der Titelverbindung als farblosen Schaum. $^1$H-NMR (300 MHz, $CD_2Cl_2$): δ= 0,55 ppm (s, 3H); 0,88 (d, 3H); 0,89 (t, 3H); 0,90 (m, 4H); 2,08 (s, 3H); 3,57 (m, 1H); 4,17 (m, 1H); 4,38 (m, 1H); 4,97 (s, 1H); 5,30 (s, 1H); 6,01 (d, 1H); 6,38 (d, 1H)

**Beispiel 3**

(5*Z*,7*E*,22*E*)-(1*S*,3*R*)-25-(1-Oxobutyl)-26,27-cyclo-9,10-secocholesta-5,7,10(19),22-tetraen-1,3-diol **71**

65. Man setzt 573 mg des Aldehydes **2** mit 700 mg des Sulfons **22** in Analogie zu 61. um und erhält 687 mg (5*Z*,7*E*)-(1*S*,3*R*)-1,3-Bis[[dimethyl(1,1-dimethylethyl)silyl]oxy]-23-phenylsulfonyl-25-(2-propyl-1,3-dioxolan-2-yl)-26,27-cyclo-9,10-secocholesta-5,7,10(19)-trien-22-ol **68** als farblosen Schaum.

66. 500 mg **68** werden analog 62. umgesetzt, wobei nacheinander 189 mg (5*Z*,7*E*,22*E*)-(1*S*,3*R*)-1,3-Bis[[dimethyl(1,1-dimethylethyl)silyl]oxy]-25-(2-propyl-1,3-dioxolan-2-yl)-26,27-cyclo-9,10-secocholesta-5,7,10(19),22-tetraen **69** und 156 mg (5*Z*,7*E*)-(1*S*,3*R*,22*S*)-1,3-Bis[[dimethyl(1,1-dimethylethyl)silyl]oxy]-25-(2-propyl-1,3-dioxolan-2-yl)-26,27-cyclo-9,10-secocholesta-5,7,10(19)-trien-22-ol **70** als farblose Schäume anfallen.
$^1$H-NMR (300 MHz, $CDCl_3$): **69:** δ= 0,03 ppm (s, 12H); 0,23 (m, 2H); 0,48 (m, 2H); 0,51 (s, 3H); 0,86 (s, 18H); 0,89 (t, 3H); 0,99 (d, 3H); 3,89 (m, 4H); 4,17 (m, 1H); 4,37 (m, 1H); 4,83 (s, 1H); 5,15 (s, 1H); 5,20 (m, 2H); 5,99 (d, 1H); 6,22 (d, 1H) **70:** δ= 0,04 ppm (s, 12H); 0,23 (m, 2H); 0,54 (s, 3H); 0,60 (m, 2H); 0,83 (t, 3H); 0,89 (d, 3H); 0,90 (s, 18H); 3,61 (m, 1H); 3,88 (m, 4H); 4,18 (m, 1H); 4,38 (m, 1H); 4,85 (s, 1H); 5,18 (s, 1H); 6,01 (d, 1H); 6,23 (d, 1H)

67. Man behandelt 180 mg **69** analog 63. und erhält 85 mg der Titelverbindung **71** als farblosen Schaum. $^1$H-NMR (300 MHz, $CD_2Cl_2$): δ= 0,52 ppm (s, 3H); 0,72 (m, 2H); 0,87 (t, 3H); 1,00 (d, 3H); 1,15 (m, 2H); 1,51 (hex, 2H); 2,37 (t, 2H); 4,18 (m, 1H); 4,38 (m, 1H); 4,95 (s, 1H); 5,29 (s, 1H); 5,30 (m, 2H); 6,00 (d, 1H); 6,35 (d, 1H)

**Beispiel 4**

68. (5*Z*,7*E*)-(1*S*,3*R*,22*S*)-25-(1-Oxobutyl)-26,27-cyclo-9,10-secocholesta-5,7,10(19)-trien-1,3,22-triol **72**

Man setzt 140 mg **70** in Analogie zu 63. um und erhält 39 mg der Titelverbindung als farblosen Schaum. $^1$H-NMR (300 MHz, $CD_2Cl_2$): δ= 0,53 ppm (s, 3H); 0,88 (d, 3H); 0,89 (t, 3H); 0,90 (m, 4H); 2,38 (t, 2H); 3,56 (m, 1H); 4,17 (m, 1H); 4,38 (m, 1H); 4,96 (s, 1H); 5,28 (s, 1H); 6,01 (d, 1H); 6,38 (d, 1H)

**Beispiel 5**

(5*Z*,7*E*,22*E*)-(1*S*,3*R*)-25-(1-Oxopentyl)-26,27-cyclo-9,10-secocholesta-5,7,10(19),22-tetraen-1,3-diol **76**

69. Man setzt 573 mg des Aldehydes **2** mit 300 mg des Sulfons **32** in Analogie zu 61. um und erhält 420 mg (5*Z*,7*E*)-(1*S*,3*R*)-1,3-Bis[[dimethyl(1,1-dimethylethyl)silyl]oxy]-23-phenylsulfonyl-25-(2-butyl-1,3-dioxolan-2-yl)-26,27-cyclo-9,10-secocholesta-5,7,10(19)-trien-22-ol **73** als farblosen Schaum.

70. 250 mg **73** werden analog 62. umgesetzt, wobei nacheinander 98 mg (5*Z*,7*E*,22*E*)-(1*S*,3*R*)-1,3-Bis[[dimethyl(1,1-dimethylethyl)silyl]oxy]-25-(2-butyl-1,3-dioxolan-2-yl)-26,27-cyclo-9,10-secocholesta-5,7,10(19),22-tetraen **74** und 82 mg (5*Z*,7*E*)-(1*S*,3*R*,22*S*)-1,3-Bis[[dimethyl(1,1-dimethylethyl)silyl]oxy]-25-(2-butyl-1,3-dioxolan-2-yl)-26,27-cyclo-9,10-secocholesta-5,7,10(19)-trien-22-ol **75** als farblose Schäume anfallen.
$^1$H-NMR (300 MHz, $CDCl_3$): **74:** δ= 0,03 ppm (s, 12H); 0,21 (m, 2H); 0,47 (m, 2H); 0,51 (s, 3H); 0,86 (s, 18H); 0,88 (t, 3H); 0,98 (d, 3H); 3,86 (m, 4H); 4,17 (m, 1H); 4,37 (m, 1H); 4,82 (s, 1H); 5,15 (s, 1H); 5,18 (m, 2H); 5,99 (d, 1H); 6,22 (d, 1H) 75: δ= 0,03 ppm (s, 12H); 0,21 (m, 2H); 0,54 (s, 3H); 0,58 (m, 2H); 0,88 (t, 3H); 0,89 (d, 3H); 0,89 (s, 18H); 3,57 (m, 1H); 3,86 (m, 4H); 4,17 (m, 1H); 4,37 (m, 1H); 4,82 (s, 1H); 5,17 (s, 1H); 6,00 (d, 1H); 6,22 (d, 1H)

71. Man behandelt 65 mg **74** analog 63. und erhält 28 mg der Titelverbindung **76** als farblosen Schaum. $^1$H-NMR (300 MHz, $CD_2Cl_2$): δ= 0,53 ppm (s, 3H); 0,71 (m, 2H); 0,89 (t, 3H); 1,00 (d, 3H); 1,10 (m, 2H); 2,38 (t, 2H); 4,17 (m, 1H); 4,38 (m, 1H); 4,95 (s, 1H); 5,29 (s, 1H); 5,29 (m, 2H); 6,00 (d, 1H); 6,37 (d, 1H)

**Beispiel 6**

72. (5*Z*,7*E*)-(1S,3*R*,22*S*)-25-(1-Oxopentyl)-26,27-cyclo-9,10-secocholesta-5,7,10(19)-trien-1,3,22-triol **77**

Man setzt 82 mg **75** in Analogie zu 63. um und erhält 34 mg der Titelverbindung als farblosen Schaum. $^1$H-NMR (300 MHz, $CD_2Cl_2$): δ= 0,54 ppm (s, 3H); 0,89 (d, 3H); 0,89 (t, 3H); 0,90 (m, 4H); 2,36 (t, 2H); 3,55 (m, 1H); 4,17 (m, 1H); 4,37 (m, 1H); 4,96 (s, 1H); 5,28 (s, 1H); 6,01 (d, 1H); 6,37 (d, 1H)

**Beispiel 7**

(5*Z*,7*E*,22*E*)-(1*S*,3*R*)-25-(1-Oxohexyl)-26,27-cyclo-9,10-secocholesta-5,7,10(19),22-tetraen-1,3-diol **81**

73. Man setzt 171 mg des Aldehydes **2** mit 210 mg des Sulfons **42** in Analogie zu 61. um und erhält 194 mg (5*Z*,7*E*)-(1*S*,3*R*)-1,3-Bis[[dimethyl(1,1-dimethylethyl)silyl]oxy]-25-(2-pentyl-1,3-dioxolan-2-yl)-23-phenylsulfonyl-26,27-cyclo-9,10-secocholesta-5,7,10(19)-trien-22-ol **78** als farblosen Schaum.

74. 175 mg **78** werden analog 62. umgesetzt, wobei nacheinander 65 mg (5*Z*,7*E*,22*E*)-(1*S*,3*R*)-1,3-Bis[[dimethyl(1,1-dimethylethyl)silyl]oxy]-25-(2-pentyl-1,3-dioxolan-2-yl)-26,27-cyclo-9,10-secocholesta-5,7,10(19),22-tetraen **79** und 40 mg (5*Z*,7*E*)-(1*S*,3*R*,22*S*)-1,3-Bis[[dimethyl(1,1 -dimethylethyl)silyl]oxy]-25-(2-pentyl-1,3-dioxolan-2-yl)-26,27-cyclo-9,10-secocholesta-5,7,10(19)-trien-22-ol **80** als farblose Schäume anfallen.
$^1$H-NMR (300 MHz, $CDCl_3$): **79**: δ= 0,03 ppm (s, 12H); 0,22 (m, 2H); 0,45 (m, 2H); 0,52 (s, 3H); 0,85 (s, 18H); 0,85 (t, 3H); 0,98 (d, 3H); 3,85 (m, 4H); 4,17 (m, 1H); 4,37 (m, 1H); 4,83 (s, 1H); 5,16 (s, 1H); 5,18 (m, 2H); 6,00 (d, 1H); 6,23 (d, 1H) **80**: δ= 0,03 ppm (s, 12H); 0,22 (m, 2H); 0,53 (s, 3H); 0,58 (m, 2H); 0,88 (t, 3H); 0,89 (d, 3H); 0,89 (s, 18H); 3,56 (m, 1H); 3,86 (m, 4H); 4,16 (m, 1H); 4,36 (m, 1H); 4,82 (s, 1H); 5,17 (s, 1H); 6,00 (d, 1H); 6,22 (d, 1H)

75. Man behandelt 64 mg **79** analog 63. und erhält 26 mg der Titelverbindung **81** als farblosen Schaum.

$^1$H-NMR (300 MHz, $CD_2Cl_2$): δ= 0,53 ppm (s, 3H); 0,70 (m, 2H); 0,89 (t, 3H); 0,99 (d, 3H); 1,10 (m, 2H); 2,37 (t, 2H); 4,17 (m, 1H); 4,38 (m, 1H); 4,96 (s, 1H); 5,30 (s, 1H); 5,31 (m, 2H); 6,00 (d, 1H); 6,36 (d, 1H)

**Beispiel 8**

76. (5*Z*,7*E*)-(1*S*,3*R*,22*S*)-25-(1-Oxohexyl)-26,27-cyclo-9,10-secocholesta-5,7,10(19)-trien-1,3,22-triol **82**

Man setzt 35 mg **80** in Analogie zu 63. um und erhält 174 mg der Titelverbindung als farblosen Schaum. $^1$H-NMR (300 MHz, $CD_2Cl_2$): δ= 0,55 ppm (s, 3H); 0,89 (d, 3H); 0,90 (t, 3H); 0,91 (m, 4H); 2,37 (t, 2H); 3,56 (m, 1H); 4,17 (m, 1H); 4,38 (m, 1H); 4,96 (s, 1H); 5,28 (s, 1H); 6,01 (d, 1H); 6,38 (d, 1H)

**Beispiel 9**

(5*Z*,7*E*,22*E*)-(1*S*,3*R*)-25-(1-Oxoheptyl)-26,27-cyclo-9,10-secocholesta-5,7,10(19),22-tetraen-1,3-diol **86**

77. Man setzt 573 mg des Aldehydes **2** mit 1,07 g des Sulfons **52** in Analogie zu 61. um und erhält 432 mg (5*Z*,7*E*)-(1*S*,3*R*)-1,3-Bis[[dimethyl(1,1-dimethylethyl)silyl]oxy]-25-(2-hexyl-1,3-dioxolan-2-yl)-23-phenylsulfonyl-26,27-cyclo-9,10-secocholesta-5,7,10(19)-trien-22-ol **83** als farblosen Schaum.

78. 432 mg **83** werden analog 62, umgesetzt, wobei nacheinander 93 mg (5*Z*,7*E*,22*E*)-(1*S*,3*R*)-1,3-Bis[[dimethyl(1,1-dimethylethyl)silyl]oxy]-25-(2-hexyl-1,3-dioxolan-2-yl)-26,27-cyclo-9,10-secocholesta-5,7,10(19),22-tetraen **84** und 48 mg (5*Z*,7*E*)-(1*S*,3*R*,22*S*)-1,3-Bis[[dimethyl(1,1-dimethylethyl)silyl]oxy]-25-(2-hexyl-1,3-dioxolan-2-yl)-26,27-cyclo-9,10-secocholesta-5,7,10(19)-trien-22-ol **85** als farblose Schäume anfallen.
$^1$H-NMR (300 MHz, $CDCl_3$): **84:** δ= 0,03 ppm (s, 12H); 0,20 (m, 2H); 0,46 (m, 2H); 0,52 (s, 3H); 0,85 (s, 18H); 0,88 (t, 3H); 0,99 (d, 3H); 3,85 (m, 4H); 4,17 (m, 1H); 4,37 (m, 1H); 4,82 (s, 1H); 5,15 (s, 1H); 5,20 (m, 2H); 6,00 (d, 1H); 6,22 (d, 1H) **85**: δ= 0,03 ppm (s, 12H); 0,20 (m, 2H); 0,53 (s, 3H); 0,55 (m, 2H); 0,87 (t, 3H); 0,88 (d, 3H); 0,89 (s, 18H); 3,55 (m, 1H); 3,84 (m, 4H); 4,16 (m, 1H); 4,36 (m, 1H); 4,82 (s, 1H); 5,16 (s, 1H); 6,01 (d, 1H); 6,23 (d, 1H)

79. Man behandelt 93 mg **84** analog 63. und erhält 31 mg der Titelverbindung **86** als farblosen Schaum.

$^1$H-NMR (300 MHz, $CD_2Cl_2$): δ= 0,53 ppm (s, 3H); 0,69 (m, 2H); 0,87 (t, 3H); 1,00 (d, 3H); 1,08 (m, 2H); 2,36 (t, 2H); 4,16 (m, 1H); 4,36 (m, 1H); 4,94 (s, 1H); 5,27 (s, 1H); 5,27 (m, 2H); 5,99 (d, 1H); 6,34 (d, 1H)

**Beispiel 10**

80. (5*Z*,7*E*)-(1*S*,3*R*,22*S*)-25-(1-Oxoheptyl)-26,27-cyclo-9,10-secocholesta-5,7,10(19)-trien-1,3,22-triol **87**

Man setzt 48 mg **85** in Analogie zu 63. um und erhält 16 mg der Titelverbindung als farblosen Schaum. $^1$H-NMR (300 MHz, $CD_2Cl_2$): δ= 0,53 ppm (s, 3H); 0,86 (d, 3H); 0,88 (t, 3H); 0,90 (m, 4H); 2,35 (t, 2H); 3,55 (m, 1H); 4,16 (m, 1H); 4,36 (m, 1H); 4,95 (s, 1H); 5,28 (s, 1H); 6,00 (d, 1H); 6,35 (d, 1H)

**Beispiel 11**

(5*Z*,7*E*,22*E*)-(1*S*,3*R*)-25-(1-Oxooctyl)-26,27-cyclo-9,10-secocholesta-5,7,10(19),22-tetraen-1,3-diol **91**

81. Man setzt 460 mg des Aldehydes **2** mit 600 mg des Sulfons **62** in Analogie zu 61. um und erhält 532 mg (5*Z*,7*E*)-(1*S*,3*R*)-1,3-Bis[[dimethyl(1,1-dimethylethyl)silyl]oxy]-25-(2-heptyl-1,3-dioxolan-2-yl)-23-phenylsulfonyl-26,27-cyclo-9,10-secocholesta-5,7,10(19)-trien-22-ol **88** als farblosen Schaum.

82. 500 mg **88** werden analog 62. umgesetzt, wobei nacheinander 145 mg (5*Z*,7*E*,22*E*)-(1*S*,3*R*)-1,3-Bis[[dimethyl(1,1-dimethylethyl)silyl]oxy]-25-(2-heptyl-1,3-dioxolan-2-yl)-26,27-cyclo-9,10-secocholesta-5,7,10(19),22-tetraen **89** und 158 mg (5*Z*,7*E*)-(1*S*,3*R*,22*S*)-1,3-Bis[[dimethyl(1,1-dimethylethyl)silyl]oxy]-25-(2-heptyl-1,3-dioxolan-2-yl)-26,27-cyclo-9,10-secocholesta-5,7,10(19)-trien-22-ol **90** als farblose Schäume anfallen.
$^1$H-NMR (300 MHz, $CDCl_3$): **89**: δ= 0,04 ppm (s, 12H); 0,21 (m, 2H); 0,45 (m, 2H); 0,52 (s, 3H); 0,87 (s, 18H); 0,87 (t, 3H); 0,99 (d, 3H); 3,85 (m, 4H); 4,17 (m, 1H); 4,36 (m, 1H); 4,82 (s, 1H); 5,15 (s, 1H); 5,20 (m, 2H); 6,00 (d, 1H); 6,22 (d, 1H) **90**: δ= 0,03 ppm (s, 12H); 0,21 (m, 2H); 0,53 (s, 3H); 0,54 (m, 2H); 0,88 (t, 3H); 0,88 (d, 3H); 0,89 (s, 18H); 3,54 (m, 1H); 3,84 (m, 4H); 4,17 (m, 1H); 4,36 (m, 1H); 4,83 (s, 1H); 5,18 (s, 1H); 6,00 (d, 1H); 6,23 (d, 1H)

83. Man behandelt 121 mg **89** analog 63. und erhält 52 mg der Titelverbindung **91** als farblosen Schaum.

$^1$H-NMR (300 MHz, $CD_2Cl_2$): δ= 0.52 ppm (s, 3H); 0,70 (m, 2H); 0,88 (t, 3H); 0,99 (d, 3H); 1,10 (m, 2H); 2,36 (t, 2H); 4,17 (m, 1H); 4,37 (m, 1H); 4,94 (s, 1H); 5,28 (s, 1H); 5,30 (m, 2H); 5,99 (d, 1H); 6,35 (d, 1H)

**Beispiel 12**

84. (5*Z*,7*E*)-(1*S*,3*R*,22*S*)-25-(1-Oxooctyl)-26,27-cyclo-9,10-secocholesta-5,7,10(19)-trien-1,3,22-triol **92**

Man setzt 130 mg **90** in Analogie zu 63. um und erhält 41 mg der Titelverbindung **92** als farblosen Schaum. $^1$H-NMR (300 MHz, $CD_2Cl_2$): δ= 0,55 ppm (s, 3H); 0,86 (d, 3H); 0,87 (t, 3H); 0,90 (m, 4H); 2,36 (t, 2H); 3,55 (m, 1H); 4,17 (m, 1H); 4,37 (m, 1H); 4,95 (s, 1H); 5,27 (s, 1H); 6,00 (d, 1H); 6,36 (d, 1H)

**Synthese der Ausgangsverbindungen in der 24-Methylen-24-homo-Reihe**

85. (*E*)-3-[1-(2-Methyl-1,3-dioxolan-2-yl)cyclopropyl]propensäuremethylester **93**

Man legt 3,2 g Natriumhydrid-Suspension (60% in Paraffinöl) in 750 ml THF vor, kühlt unter Stickstoff auf 0°C und gibt 10,9 g Dimethylphosphonoessigsäuremethylester hinzu. Anschließend werden 7,5 g des Aldehydes **7** in 15 ml THF zugetropft und es wird bei Raumtemperatur für 2 h gerührt. Nun quencht man vorsichtig mit Natriumchlorid-Lösung, extrahiert mit Essigester, wäscht mit Natriumchlorid-Lösung, trocknet über Natriumsulfat und entfernt das Solvens. Der Rückstand wird an Silicagel mit Essigester/Hexan chromatographiert, wobei 9,5 g der Titelverbindung **93** als farbloses Öl anfallen.
$^1$H-NMR (300 MHz, $CDCl_3$): δ= 0,73 ppm (m, 2H); 1,09 (m, 2H); 1,45 (s, 3H); 3,72 (s, 3H); 3,95 (m, 4H); 5,79 (d, 1H); 7,18 (d, 1H)

86. 3-[1-(2-Methyl-1,3-dioxolan-2-yl)cyclopropan]-1-propanol **94**

Man legt 100 ml flüssigen Ammoniak vor und gibt portionsweise 2 g Lithium hinzu. Anschließend tropft man 3,1 g **93** in 20 ml THF zu und rührt über Nacht bei Raumtemperatur nach, wobei der Ammoniak abdampft. Der Rückstand wird in Essigester aufgenommen, mit Natriumchlorid-Lösung gewaschen, über Natriumsulfat getrocknet, eingeengt und an Silicagel mit Essigester/Hexan chromatographiert, wobei man 1,5 g der Titelverbindung **94** als farbloses Öl erhält.
$^1$H-NMR (300 MHz, $CDCl_3$): δ= 0,29 ppm (m, 2H); 0,62 (m, 2H); 1,40 (s, 3H); 1,51-1.70 (m, 4H); 3,62 (t, 2H); 3,90 (m, 4H)

87. 4-Methylbenzolsulfonsäure 3-[1-(2-methyl-1,3-dioxolan-2-yl)cyclopropyl]propylester **95**

Man setzt 350 mg **94** analog 8. um, wobei 405 mg der Titelverbindung **95** als farbloses Öl anfallen. $^1$H-NMR (300 MHz, $CDCl_3$): δ= 0,19 ppm (m, 2H); 0,60 (m, 2H); 1,32 (s, 3H); 1,44 (m, 2H); 1,79 (m, 2H); 2,46 (s, 3H); 3,85 (m, 4H); 4,02 (t, 2H); 7,35 (d, 2H); 7,79 (d, 2H)

88. 2-Methyl-2-[1 -[3-(phenylsulfanyl)ethyl]cyclopropyl]-1,3-dioxolan **96**

Man setzt 400 mg **95** analog 9. um, wobei 380 mg der Titelverbindung **96** als farbloses Öl anfallen. $^1$H-NMR (300 MHz, $CDCl_3$): δ= 0,27 ppm (m, 2H); 0,60 (m, 2H); 1,38 (s, 3H); 1,61 (m, 2H); 1,76 (m, 2H); 2,92 (t, 2H); 3,89 (m, 4H); 7,28 (m, 5H)

89. 2-Methyl-2-[1-[3-(phenylsulfonyl)ethyl]cyclopropyl]-1,3-dioxolan **97**

Man setzt 375 mg **96** analog 10. um, wobei 268 mg der Titelverbindung **97** als farbloses Öl erhalten werden. $^1$H-NMR (300 MHz, $CDCl_3$): δ= 0,20 ppm (m, 2H); 0,62 (m, 2H); 1,31 (s, 3H); 1,52 (m, 2H); 1,87 (m, 2H); 3,12 (t, 2H); 3,83 (m, 4H); 7,58 (t, 2H); 7,66 (t, 1H); 7,91 (d, 2H)

90. (*E*)-3-[1-(2-Propyl-1,3-dioxolan-2-yl)cyclopropyl]propensäuremethylester **98**

Man setzt 6,0 g des Aldehydes **17** analog 85. um und erhält 6,7 g der Titelverbindung **98** als farbloses Öl. $^1$H-NMR (300 MHz, $CDCl_3$): δ= 0,70 ppm (m, 2H); 0,90 (t, 3H); 0,90 (m, 2H); 1,03 (m, 2H); 1,73 (m, 2H); 3,72 (s, 3H); 3,95 (m, 4H); 5,70 (d, 1H); 7,25 (d, 1H) 91. 3-[1-(2-Propyl-1,3-dioxolan-2-yl)cyclopropan]-1-propanol **99**

Man setzt 6,7 g **98** analog 86. um, wobei man 5,3 g der Titelverbindung **99** als farbloses Öl erhält. $^1$H-NMR (300 MHz, $CDCl_3$): δ= 0,23 ppm (m, 2H); 0,60 (m, 2H); 0,92 (t, 3H); 3,62 (t, 2H); 3,90 (m, 4H)

92. 4-Methylbenzolsulfonsäure 3-[1-(2-propyl-1,3-dioxolan-2-yl)cyclopropyl]propylester **100**

Man setzt 1,3 g **99** analog 8. um, wobei 1,52 g der Titelverbindung **100** als farbloses Öl anfallen. $^1$H-NMR (300 MHz, $CDCl_3$): δ= 0,12 ppm (m, 2H); 0,57 (m, 2H); 0,90 (t, 3H); 2,47 (s, 3H); 3,83 (m, 4H); 4,02 (t, 2H); 7,35 (d, 2H); 7,79 (d, 2H)

93. 2-[1-[3-(Phenylsulfanyl)ethyl]cyclopropyl]-2-propyl-1,3-dioxolan **101**

Man setzt 1,5 g **100** analog 9. um, wobei 1,11 g der Titelverbindung **96** als farbloses Öl anfallen. $^1$H-NMR (300 MHz, $CDCl_3$): δ= 0,20 ppm (m, 2H); 0,58 (m, 2H); 0,91 (t, 3H); 2,91 (t, 2H); 3,88 (m, 4H); 7,28 (m, 5H)

94. 2-[1-[3-(Phenylsulfonyl)ethyl]cyclopropyl]-2-propyl-1,3-dioxolan **102**

Man setzt 1,1 g **101** analog 10. um, wobei 785 mg der Titelverbindung **102** als farbloses Öl erhalten werden. $^1$H-NMR (300 MHz, $CDCl_3$): δ= 0,17 ppm (m, 2H); 0,59 (m, 2H); 0,90 (t, 3H); 3,10 (t, 2H); 3,85 (m, 4H); 7,58 (t, 2H); 7,67 (t, 1H); 7,90 (d, 2H)

95. (*E*)-3-[1-(2-Butyl-1,3-dioxolan-2-yl)cyclopropyl]propensäuremethylester **103**

Man setzt 5,0 g des Aldehydes **27** analog 85. um und erhält 4,6 g der Titelverbindung **103** als farbloses Öl. $^1$H-NMR (300 MHz, $CDCl_3$): δ= 0,70 ppm (m, 2H); 0,89 (t, 3H); 1,04 (m, 2H); 1,30 (m, 4H); 1,79 (m, 2H); 3,72 (s, 3H); 3,96 (m, 4H); 5,71 (d, 1H); 7,25 (d, 1H)

96. 3-[1-(2-Butyl-1,3-dioxolan-2-yl)cyclopropan]-1-propanol **104**

Man setzt 1,5 g **103** analog 86. um, wobei man 1,09 g der Titelverbindung **104** als farbloses Öl erhält. $^1$H-NMR (300 MHz, $CDCl_3$): δ= 0,23 ppm (m, 2H); 0,60 (m, 2H); 0,92 (t, 3H); 3,61 (t, 2H); 3,92 (m, 4H)

97. 4-Methylbenzolsulfonsäure 3-[1-(2-butyl-1,3-dioxolan-2-yl)cyclopropyl]propylester **105**

Man setzt 1,09 g **104** analog 8. um, wobei 1,36 g der Titelverbindung **105** als farbloses Öl anfallen. $^1$H-NMR (300 MHz, $CDCl_3$): δ= 0,12 ppm (m, 2H); 0,58 (m, 2H); 0,89 (t, 3H); 2,47 (s, 3H); 3,85 (m, 4H); 4,02 (t, 2H);

7,35 (d, 2H); 7,80 (d, 2H)

98. 2-Butyl-2-[1-[3-(phenylsulfanyl)ethyl]cyclopropyl]-1,3-dioxolan **106**

Man setzt 800 mg **105** analog 9. um, wobei 967 mg der Titelverbindung **106** als farbloses Öl anfallen. $^1$H-NMR (300 MHz, $CDCl_3$): δ= 0,20 ppm (m, 2H); 0,59 (m, 2H); 0,90 (t, 3H); 2,89 (t, 2H); 3,85 (m, 4H); 7,27 (m, 5H)

99. 2-Butyl-2-[1-[3-(phenylsulfonyl)ethyl]cyclopropyl]-1,3-dioxolan **107**

Man setzt 950 g **106** analog 10. um, wobei 634 mg der Titelverbindung **107** als farbloses Öl erhalten werden. $^1$H-NMR (300 MHz, $CDCl_3$): δ= 0,15 ppm (m, 2H); 0,60 (m, 2H); 0,90 (t, 3H); 3,09 (t, 2H); 3,83 (m, 4H); 7,58 (t, 2H); 7,68 (t, 1H); 7,91 (d, 2H)

100. (*E*)-3-[1-(2-Pentyl-1,3-dioxolan-2-yl)cyclopropyl]propensäuremethylester **108**

Man setzt 1,8 g des Aldehydes **37** analog 85. um und erhält 1,1 g der Titelverbindung **108** als farbloses Öl. $^1$H-NMR (300 MHz, $CDCl_3$): δ= 0,70 ppm (m, 2H); 0,88 (t, 3H); 1,03 (m, 2H); 1,30 (m, 6H); 3,72 (s, 3H); 3,95 (m, 4H); 5,71 (d, 1H); 7,24 (d, 1H)

101.3-[1-(2-Pentyl-1,3-dioxolan-2-yl)cyclopropan]-1-propanol **109**

Man setzt 2,68 g **108** analog 86. um, wobei man 1,57 g der Titelverbindung **109** als farbloses Öl erhält. $^1$H-NMR (300 MHz, $CDCl_3$): δ= 0,23 ppm (m, 2H); 0,60 (m, 2H); 0,90 (t, 3H); 3,61 (t, 2H); 3,91 (m, 4H)

102. 4-Methylbenzolsulfonsäure 3-[1-(2-pentyl-1,3-dioxolan-2-yl)cyclopropyl]propylester **110**

Man setzt 590 mg **109** analog 8. um, wobei 456 g der Titelverbindung **110** als farbloses Öl anfallen. $^1$H-NMR (300 MHz, $CDCl_3$): δ= 0,18 ppm (m, 2H); 0,60 (m, 2H); 0,90 (t, 3H); 2,48 (s, 3H); 3,85 (m, 4H); 4,02 (t, 2H); 7,35 (d, 2H); 7,81 (d, 2H)

103. 2-Pentyl-2-[1-[3-(phenylsulfanyl)ethyl]cyclopropyl]-1,3-dioxolan **111**

Man setzt 350 mg **110** analog 9. um, wobei 272 mg der Titelverbindung **111** als farbloses Öl anfallen. $^1$H-NMR (300 MHz, $CDCl_3$): δ= 0,19 ppm (m, 2H); 0,59 (m, 2H); 0,89 (t, 3H); 2,87 (t, 2H); 3,85 (m, 4H); 7,28 (m, 5H)

104. 2-Pentyl-2-[1-[3-(phenylsulfonyl)ethyl]cyclopropyl]-1,3-dioxolan **112**

Man setzt 245 mg **111** analog 10. um, wobei 191 mg der Titelverbindung **112** als farbloses Öl erhalten werden. $^1$H-NMR (300 MHz, $CDCl_3$): δ= 0,14 ppm (m, 2H); 0,57 (m, 2H); 0,88 (t, 3H); 3,09 (t, 2H); 3,82 (m, 4H); 7,58 (t, 2H); 7,67 (t, 1H); 7,89 (d, 2H)

105. (*E*)-3-[1-(2-Hexyl-1,3-dioxolan-2-yl)cyclopropyl]propensäuremethylester **113**

Man setzt 5,65 g des Aldehydes **47** analog 85. um und erhält 4,81 g der Titelverbindung **113** als farbloses Öl. $^1$H-NMR (300 MHz, $CDCl_3$): δ= 0,70 ppm (m, 2H); 0,89 (t, 3H); 1,04 (m, 2H); 1,29 (m, 8H); 3,72 (s, 3H); 3,96 (m, 4H); 5,71 (d, 1H); 7,25 (d, 1H)

106. 3-[1-(2-Hexyl-1,3-dioxolan-2-yl)cyclopropan]-1-propanol **114**

Man setzt 2,0 g **113** analog 86. um, wobei man 1,7 g der Titelverbindung **114** als farbloses Öl erhält. $^1$H-NMR (300 MHz, $CDCl_3$): δ= 0,22 ppm (m, 2H); 0,60 (m, 2H); 0,89 (t, 3H); 3,62 (t, 2H); 3,90 (m, 4H)

107. 4-Methylbenzolsulfonsäure 3-[1-(2-hexyl-1,3-dioxolan-2-yl)cyclopropyl]propylester **115**

Man setzt 1,7 mg **114** analog 8. um, wobei 1,6 g der Titelverbindung **115** als farbloses Öl anfallen. $^1$H-NMR (300 MHz, $CDCl_3$): δ= 0,20 ppm (m, 2H); 0,60 (m, 2H); 0,91 (t, 3H); 2,49 (s, 3H); 3,85 (m, 4H); 4,03 (t, 2H); 7,36 (d, 2H); 7,81 (d, 2H)

108. 2-Hexyl-2-[1-[3-(phenylsulfanyl)ethyl]cyclopropyl]-1,3-dioxolan **116**

Man setzt 1,5 mg **115** analog 9. um, wobei 1,37 g der Titelverbindung **116** als farbloses Öl anfallen. $^{1}$H-NMR (300 MHz, $CDCl_3$): δ= 0,19 ppm (m, 2H); 0,60 (m, 2H); 0,90 (t, 3H); 2,87 (t, 2H); 3,85 (m, 4H); 7,30 (m, 5H)

109. 2-Hexyl-2-[1-[3-(phenylsulfonyl)ethyl]cyclopropyl]-1,3-dioxolan **117**

Man setzt 654 mg **116** analog 10. um, wobei 630 mg der Titelverbindung **117** als farbloses Öl erhalten werden. $^{1}$H-NMR (300 MHz, $CDCl_3$): δ= 0,14 ppm (m, 2H); 0,58 (m, 2H); 0,86 (t, 3H); 3,09 (t, 2H); 3,82 (m, 4H); 7,57 (t, 2H); 7,65 (t, 1H); 7,89 (d, 2H)

110. (*E*)-3-[1-(2-Heptyl-1,3-dioxolan-2-yl)cyclopropyl]propensäuremethylester **118**

Man setzt 2,2 g des Aldehydes **57** analog 85. um und erhält 1,7 g der Titelverbindung **118** als farbloses Öl. $^{1}$H-NMR (300 MHz, $CDCl_3$): δ= 0,70 ppm (m, 2H); 0,89 (t, 3H); 1,04 (m, 2H); 1,29 (m, 10H); 3,72 (s, 3H); 3,95 (m, 4H); 5,71 (d, 1H); 7,25 (d, 1H)

111. 3-[1-(2-Heptyl-1,3-dioxolan-2-yl)cyclopropan]-1-propanol **119**

Man setzt 1,6 g **118** analog 86. um, wobei man 987 mg der Titelverbindung **119** als farbloses Öl erhält. $^{1}$H-NMR (300 MHz, $CDCl_3$): δ= 0,23 ppm (m, 2H); 0,60 (m, 2H); 0,89 (t, 3H); 3,62 (t, 2H); 3,90 (m, 4H)

112. 4-Methylbenzolsulfonsäure 3-[1-(2-heptyl-1,3-dioxolan-2-yl)cyclopropyl]propylester **120**

Man setzt 900 mg **119** analog 8. um, wobei 1,3 g der Titelverbindung **120** als farbloses Öl anfallen. $^{1}$H-NMR (300 MHz, $CDCl_3$): δ= 0,22 ppm (m, 2H); 0,60 (m, 2H); 0,90 (t, 3H); 2,48 (s, 3H); 3,85 (m, 4H); 4,03 (t, 2H); 7,37 (d, 2H); 7,81 (d, 2H)

113. 2-Heptyl-2-[1-[3-(phenylsulfanyl)ethyl]cyclopropyl]-1,3-dioxolan **121**

Man setzt 1,3 mg **120** analog 9. um, wobei 921 mg der Titelverbindung **121** als farbloses Öl anfallen. $^{1}$H-NMR (300 MHz, $CDCl_3$): δ= 0,22 ppm (m, 2H); 0,62 (m, 2H); 0,90 (t, 3H); 2,90 (t, 2H); 3,85 (m, 4H); 7,30 (m, 5H)

114. 2-Heptyl-2-[1-[3-(phenylsulfonyl)ethyl]cyclopropyl]-1,3-dioxolan **122**

Man setzt 456 mg **121** analog 10. um, wobei 376 mg der Titelverbindung **122** als farbloses Öl erhalten werden. $^{1}$H-NMR (300 MHz, $CDCl_3$): δ= 0,17 ppm (m, 2H); 0,59 (m, 2H); 0,89 (t, 3H); 3,10 (t, 2H); 3,82 (m, 4H); 7,58 (t, 2H); 7,67 (t, 1H); 7,90 (d, 2H)

**Beispiel 13**

(5*Z*,7*E*,22*E*)-(1*S*,3*R*)-25-Acetyl-26,27-cyclo-24a-homo-9,10-secocholesta-5,7,10(19),22-tetraen-1,3-diol **126**

115. Man setzt 343 mg des Aldehydes **2** mit 260 mg des Sulfons **97** analog 61. um und erhält 402 mg (5*Z*,7*E*)-(1*S*,3*R*)-1,3-Bis[[dimethyl(1,1-dimethylethyl)silyl]oxy]-25-(2-methyl-1,3-dioxolan-2-yl)-23-phenylsulfonyl-26,27-cyclo-24a-homo-9,10-secocholesta-5,7,10(19)-trien-22-ol **123** als farblosen Schaum.

116. Man setzt 400 mg **123** in Analogie zu 62. um, wobei nacheinander 123 mg (5*Z*,7*E*,22*E*)-(1*S*,3*R*)-1,3-Bis[[dimethyl(1,1-dimethylethyl)silyl]oxy]-25-(2-methyl-1,3-dioxolan-2-yl)-26,27-cyclo-24a-homo-9,10-secocholesta-5,7,10(19),22-tetraen **124** und 70 mg (5*Z*,7*E*)-(1*S*,3*R*,22*S*)-1,3-Bis[[dimethyl(1,1-dimethylethyl)silyl]oxy]-25-(2-methyl-1,3-dioxolan-2-yl)-26,27-cyclo-24a-homo-9,10-secocholesta-5,7,10(19)-trien-22-ol **125** als farblose Schäume anfallen.
$^{1}$H-NMR (300 MHz, $CDCl_3$): **124:** δ= 0,03 ppm (s, 12H); 0,23 (m, 2H); 0,52 (s, 3H); 0,55 (d, 2H); 0,86 (s, 18H); 0,86 (d, 3H); 1,32 (s, 3H); 3,83 (m, 4H); 4,16 (m, 1H); 4,36 (m, 1H); 4,82 (s, 1H); 5,15 (s, 1H); 5,22 (m, 2H); 6,00 (d, 1H); 6,23 (d, 1H) **125:** δ= 0,04 ppm (s, 12H); 0,23 (m, 2H); 0,52 (s, 3H); 0,55 (m, 2H); 0,85 (d, 3H); 0,86 (s, 18H); 1,32 (s, 3H); 3,61 (m, 1H); 3,82 (m, 4H); 4,16 (m, 1H); 4,36 (m, 1H); 4,82 (s, 1H); 5,15 (s, 1H); 6,01 (d, 1H); 6,23 (d, 1H)

117. Man setzt 98 mg **124** analog 63. um, wobei man 43 mg der Titelverbindung **126** als farblosen Schaum erhält.
$^{1}$H-NMR (300 MHz, $CD_2Cl_2$): δ= 0,53 ppm (s, 3H); 0,78 (m, 2H); 1,00 (d, 3H); 1,20 (m, 2H); 2,00 (s, 3H); 4,19 (m, 1H);

4,38 (m, 1H); 4,99 (s, 1H); 5,28 (m, 2H); 5,30 (s, 1H); 6,01 (d, 1H); 6,38 (d, 1H)

**Beispiel 14**

118. (5*Z*,7*E*)-(1*S*,3*R*,22*S*)-25-Acetyl-26,27-cyclo-24a-homo-9,10-secocholesta-5,7,10(19)-trien-1,3,22-triol **127**

Man setzt 61 mg **125** in Analogie zu 63. um und erhält 24 mg der Titelverbindung als farblosen Schaum. $^1$H-NMR (300 MHz, $CD_2Cl_2$): δ= 0,56 ppm (s, 3H); 0,80 (m, 2H); 0,86 (d, 3H); 0,89 (t, 3H); 1,18 (m, 2H); 2,03 (s, 3H); 3,67 (m, 1H); 4,17 (m, 1H); 4,38 (m, 1H); 4,99 (s, 1H); 5,32 (s, 1H); 6,01 (d, 1H); 6,38 (d, 1H)

**Beispiel 15**

(5*Z*,7*E*,22*E*)-(1*S*,3*R*)-25-(1-Oxobutyl)-26,27-cyclo-24a-homo-9,10-secocholesta-5,7,10(19),22-tetraen-1,3-diol **131**

119. Man setzt 570 mg des Aldehydes **2** mit 780 mg des Sulfons **102** in Analogie zu 61. um und erhält 606 mg (5*Z*,7*E*)-(1*S*,3*R*)-1,3-Bis[[dimethyl(1,1-dimethylethyl)silyl]oxy]-23-phenylsulfonyl-25-(2-propyl-1,3-dioxolan-2-yl)-26,27-cyclo-24a-homo-9,10-secocholesta-5,7,10(19)-trien-22-ol **128** als farblosen Schaum.

120. 600 mg **128** werden analog 62. umgesetzt, wobei nacheinander 230 mg (5*Z*,7*E*,22*E*)-(1*S*,3*R*)-1,3-Bis[[dimethyl(1,1-dimethylethyl)silyl]oxy]-25-(2-propyl-1,3-dioxolan-2-yl)-26,27-cyclo-24a-homo-9,10-secocholesta-5,7,10(19),22-tetraen **129** und 186 mg (5*Z*,7*E*)-(1*S*,3*R*,22*S*)-1,3-Bis[[dimethyl(1,1-dimethylethyl)silyl]oxy]-25-(2-propyl-1,3-dioxolan-2-yl)-26,27-cyclo-24a-homo-9,10-secocholesta-5,7,10(19)-trien-22-ol **130** als farblose Schäume anfallen.
$^1$H-NMR (300 MHz, $CDCl_3$): **129:** δ= 0,03 ppm (s, 12H); 0,18 (m, 2H); 0,50 (m, 2H); 0,51 (s, 3H); 0,84 (s, 18H); 0,85 (t, 3H); 0,96 (d, 3H); 3,85 (m, 4H); 4,17 (m, 1H); 4,36 (m, 1H); 4,82 (s, 1H); 5,15 (s, 1H); 5,25 (m, 2H); 6,00 (d, 1H); 6,23 (d, 1H)
**130:** δ= 0,03 ppm (s, 12H); 0,19 (m, 2H); 0,50 (s, 3H); 0,56 (m, 2H); 0,87 (t, 3H); 0,88 (d, 3H); 0,89 (s, 18H); 3,57 (m, 1H); 3,86 (m, 4H); 4,17 (m, 1H); 4,37 (m, 1H); 4,81 (s, 1H); 5,17 (s, 1H); 6,00 (d, 1H); 6,23 (d, 1H)

121. Man behandelt 220 mg **129** analog 63. und erhält 73 mg der Titelverbindung **131** als farblosen Schaum. $^1$H-NMR (300 MHz, $CD_2Cl_2$): δ= 0,56 ppm (s, 3H); 0,73 (m, 2H); 0,87 (t, 3H); 1,00 (d, 3H); 1,11 (m, 2H); 2,26 (t, 2H); 4,17 (m, 1H); 4,37 (m, 1H); 4,96 (s, 1H); 5,29 (s, 1H); 5,35 (m, 2H); 6,00 (d, 1H); 6,36 (d, 1H)

**Beispiel 16**

122. (5*Z*,7*E*)-(1*S*,3*R*,22*S*)-25-(1-Oxobutyl)-26,27-cyclo-24a-homo-9,10-secocholesta-5,7,10(19)-trien-1,3,22-triol **132**

Man setzt 180 mg **130** in Analogie zu 63. um und erhält 38 mg der Titelverbindung als farblosen Schaum. $^1$H-NMR (300 MHz, $CD_2Cl_2$): δ= 0,53 ppm (s, 3H); 0,70 (m, 2H); 0,88 (d, 3H); 0,89 (t, 3H); 1,12 (m, 2H); 2,19 (t, 2H); 3,62 (m, 1H); 4,17 (m, 1H); 4,37 (m, 1H); 4,96 (s, 1H); 5,29 (s, 1H); 6,00 (d, 1H); 6,36 (d, 1H)

**Beispiel 17**

(5*Z*,7*E*,22*E*)-(1*S*,3*R*)-25-( 1 -Oxopentyl)-26,27-cyclo-24a-homo-9,10-secocholesta-5,7,10(19),22-tetraen-1,3-diol **136**

123. Man setzt 286 mg des Aldehydes **2** mit 390 mg des Sulfons **107** in Analogie zu 61. um und erhält 325 mg (5*Z*,7*E*)-(1*S*,3*R*)-1,3-Bis[[dimethyl(1,1-dimethylethyl)silyl]oxy]-23-phenylsulfonyl-25-(2-butyl-1,3-dioxolan-2-yl)-26,27-cyclo-24a-homo-9,10-secocholesta-5,7,10(19)-trien-22-ol **133** als farblosen Schaum.

124. 200 mg **133** werden analog 62. umgesetzt, wobei nacheinander 85 mg (5*Z*,7*E*,22*E*)-(1*S*,3*R*)-1,3-Bis[[dimethyl(1,1-dimethylethyl)silyl]oxy]-25-(2-butyl-1,3-dioxolan-2-yl)-26,27-cyclo-24a-homo-9,10-secocholesta-5,7,10(19),22-tetraen **134** und 70 mg (5*Z*,7*E*)-(1*S*,3*R*,22*S*)-1,3-Bis[[dimethyl(1,1-dimethylethyl)silyl]oxy]-25-(2-butyl-1,3-dioxolan-2-yl)-26,27-cyclo-24a-homo-9,10-secocholesta-5,7,10(19)-trien-22-ol **135** als farblose Schäume anfallen.
$^1$H-NMR (300 MHz, $CDCl_3$): **134**: δ= 0,03 ppm (s, 12H); 0,18 (m, 2H); 0,51 (m, 2H); 0,52 (s, 3H); 0,86 (s, 18H); 0,87 (t, 3H); 0,99 (d, 3H); 3,85 (m, 4H); 4,17 (m, 1H); 4,36 (m, 1H); 4,82 (s, 1H); 5,15 (s, 1H); 5,18 (m, 2H); 6,00 (d, 1H); 6,22 (d, 1H)
**135:** δ= 0,04 ppm (s, 12H); 0,22 (m, 2H); 0,55 (s, 3H); 0,58 (m, 2H); 0,89 (t, 3H); 0,89 (d, 3H); 0,89 (s, 18H); 3,68 (m, 1H); 3,90 (m, 4H); 4,18 (m, 1H); 4,38 (m, 1H); 4,85 (s, 1H); 5,18 (s, 1H); 6,01 (d, 1H); 6,23 (d, 1H)

125. Man behandelt 43 mg **134** analog 63. und erhält 23 mg der Titelverbindung **136** als farblosen Schaum. $^1$H-NMR (300 MHz, $CD_2Cl_2$): δ= 0,54 ppm (s, 3H); 0,70 (m, 2H); 0,88 (t, 3H); 0,98 (d, 3H); 1,10 (m, 2H); 2,27 (t, 2H);

4,17 (m, 1H); 4,37 (m, 1H); 4,95 (s, 1H); 5,28 (s, 1H); 5,28 (m, 2H); 5,99 (d, 1H); 6,36 (d, 1H)

**Beispiel 18**

126. (5*Z*,7*E*)-(1*S*,3*R*,22*S*)-25-(1-Oxopentyl)-26,27-cyclo-24a-homo-9,10-secocholesta-5,7,10(19)-trien-1,3,22-triol **137**

Man setzt 48 mg **135** in Analogie zu 63. um und erhält 23 mg der Titelverbindung als farblosen Schaum. $^1$H-NMR (300 MHz, $CD_2Cl_2$): δ= 0,55 ppm (s, 3H); 0,71 (m, 2H); 0,87 (d, 3H); 0,89 (t, 3H); 1,12 (m, 4H); 2,23 (t, 2H); 3,64 (m, 1H); 4,16 (m, 1H); 4,36 (m, 1H); 4,94 (s, 1H); 5,28 (s, 1H); 6,00 (d, 1H); 6,35 (d, 1H)

**Beispiel 19**

(5*Z*,7*E*,22*E*)-(1*S*,3*R*)-25-(1-Oxohexyl)-26,27-cyclo-24a-homo-9,10-secocholesta-5,7,10(19),22-tetraen-1,3-diol **141**

127. Man setzt 286 mg des Aldehydes **2** mit 185 mg des Sulfons **112** in Analogie zu 61. um und erhält 187 mg (5*Z*,7*E*)-(1*S*,3*R*)-1,3-Bis[[dimethyl(1,1-dimethylethyl)silyl]oxy]-25-(2-pentyl-1,3-dioxolan-2-yl)-23-phenylsulfonyl-26,27-cyclo-24a-homo-9,10-secocholesta-5,7,10(19)-trien-22-ol **138** als farblosen Schaum.

128. 125 mg **138** werden analog 62. umgesetzt, wobei nacheinander 35 mg (5*Z*,7*E*,22*E*)-(1*S*,3*R*)-1,3-Bis[[dimethyl(1,1-dimethylethyl)silyl]oxy]-25-(2-pentyl-1,3-dioxolan-2-yl)-26,27-cyclo-24a-homo-9,10-secocholesta-5,7,10(19),22-tetraen **139** und 27 mg (5*Z*,7*E*)-(1*S*,3*R*,22*S*)-1,3-Bis[[dimethyl(1,1-dimethylethyl)silyl]oxy]-25-(2-pentyl-1,3-dioxolan-2-yl)-26,27-cyclo-24a-homo-9,10-secocholesta-5,7,10(19)-trien-22-ol **140** als farblose Schäume anfallen.
$^1$H-NMR (300 MHz, $CDCl_3$): **139:** δ= 0,04 ppm (s, 12H); 0,17 (m, 2H); 0,52 (m, 2H); 0,53 (s, 3H); 0,86 (s, 18H); 0,87 (t, 3H); 0,98 (d, 3H); 3,84 (m, 4H); 4,17 (m, 1H); 4,36 (m, 1H); 4,82 (s, 1H); 5,16 (s, 1H); 5,24 (m, 2H); 6,00 (d, 1H); 6,23 (d, 1H)
**140**: δ= 0,04 ppm (s, 12H); 0,18 (m, 2H); 0,54 (s, 3H); 0,55 (m, 2H); 0,87 (t, 3H); 0,89 (d, 3H); 0,89 (s, 18H); 3,62 (m, 1H); 3,84 (m, 4H); 4,17 (m, 1H); 4,37 (m, 1H); 4,83 (s, 1H); 5,16 (s, 1H); 6,00 (d, 1H); 6,23 (d, 1H)

129. Man behandelt 35 mg **139** analog 63. und erhält 16 mg der Titelverbindung **141** als farblosen Schaum. $^1$H-NMR (300 MHz, $CD_2Cl_2$): δ= 0,56 ppm (s, 3H); 0,70 (m, 2H); 0,88 (t, 3H); 0,98 (d, 3H); 1,09 (m, 2H); 2,23 (t, 2H); 4,17 (m, 1H); 4,36 (m, 1H); 4,97 (s, 1H); 5,28 (s, 1H); 5,29 (m, 2H); 5,99 (d, 1H); 6,34 (d, 1H)

**Beispiel 20**

130. (5*Z*,7*E*)-(1*S*,3*R*,22*S*)-25-(1 -Oxohexyl)-26,27-cyclo-24a-homo-9,10-secocholesta-5,7,10(19)-trien-1,3,22-triol **142**

Man setzt 25 mg **140** in Analogie zu 63. um und erhält 11 mg der Titelverbindung als farblosen Schaum. $^1$H-NMR (300 MHz, $CD_2Cl_2$): δ= 0,55 ppm (s, 3H); 0,71 (m, 2H); 0,87 (d, 3H); 0,87 (t, 3H); 1,14 (m, 2H); 2,23 (t, 2H); 3,65 (m, 1H); 4,16 (m, 1H); 4,36 (m, 1H); 4,95 (s, 1H); 5,28 (s, 1H); 6,00 (d, 1H); 6,36 (d, 1H)

**Beispiel 21**

(5*Z*,7*E*,22*E*)-(1*S*,3*R*)-25-(1-Oxoheptyl)-26,27-cyclo-24a-homo-9,10-secocholesta-5,7,10(19),22-tetraen-1,3-diol **146**

131. Man setzt 573 mg des Aldehydes **2** mit 630 mg des Sulfons **117** in Analogie zu 61. um und erhält 599 mg (5*Z*,7*E*)-(1*S*,3*R*)-1,3-Bis[[dimethyl(1,1-dimethylethyl)silyl]oxy]-25-(2-hexyl-1,3-dioxolan-2-yl)-23-phenylsulfonyl-26,27-cyclo-24a-homo-9,10-secocholesta-5,7,10(19)-trien-22-ol **143** als farblosen Schaum.

132. 500 mg **143** werden analog 62. umgesetzt, wobei nacheinander 156 mg (5*Z*,7*E*,22*E*)-(1*S*,3*R*)-1,3-Bis[[dimethyl(1,1-dimethylethyl)silyl]oxy]-25-(2-hexyl-1,3-dioxolan-2-yl)-26,27-cyclo-24a-homo-9,10-secocholesta-5,7,10(19),22-tetraen **144** und 98 mg (5*Z*,7*E*)-(1*S*,3*R*,22*S*)-1,3-Bis[[dimethyl(1,1-dimethylethyl)silyl]oxy]-25-(2-hexyl-1,3-dioxolan-2-yl)-26,27-cyclo-24a-homo-9,10-secocholesta-5,7,10(19)-trien-22-ol **145** als farblose Schäume anfallen.
$^1$H-NMR (300 MHz, $CDCl_3$): **144:** δ= 0,03 ppm (s, 12H); 0,19 (m, 2H); 0,48 (m, 2H); 0,53 (s, 3H); 0,85 (s, 18H); 0,90 (t, 3H); 0,98 (d, 3H); 3,86 (m, 4H); 4,16 (m, 1H); 4,36 (m, 1H); 4,83 (s, 1H); 5,16 (s, 1H); 5,25 (m, 2H); 6,00 (d, 1H); 6,24 (d, 1H)
**145:** δ= 0,04 ppm (s, 12H); 0,19 (m, 2H); 0,55 (s, 3H); 0,56 (m, 2H); 0,87 (t, 3H); 0,89 (d, 3H); 0,90 (s, 18H); 3,63 (m, 1H); 3,84 (m, 4H); 4,17 (m, 1H); 4,37 (m, 1H); 4,83 (s, 1H); 5,16 (s, 1H); 6,01 (d, 1H); 6,23 (d, 1H)

133. Man behandelt 95 mg **144** analog 63. und erhält 27 mg der Titelverbindung **146** als farblosen Schaum.

$^1$H-NMR (300 MHz, $CD_2Cl_2$): δ= 0,54 ppm (s, 3H); 0,71 (m, 2H); 0,86 (t, 3H); 1,00 (d, 3H); 1,12 (m, 2H); 2,22 (t, 2H); 4,16 (m, 1H); 4,36 (m, 1H); 4,94 (s, 1H); 5,25 (s, 1H); 5,25 (m, 2H); 5,99 (d, 1H); 6,34 (d, 1H)

**Beispiel 22**

134. (5*Z*,7*E*)-(1*S*,3*R*,22*S*)-25-(1-Oxoheptyl)-26,27-cyclo-24a-homo-9,10-secocholesta-5,7,10(19)-trien-1,3,22-triol **147**

Man setzt 43 mg **145** in Analogie zu 63. um und erhält 14 mg der Titelverbindung als farblosen Schaum. $^1$H-NMR (300 MHz, $CD_2Cl_2$): δ= 0,55 ppm (s, 3H); 0,73 (m, 2H); 0,85 (d, 3H); 0,86 (t, 3H); 1,13 (m, 2H); 2,21 (t, 2H); 3,64 (m, 1H); 4,16 (m, 1H); 4,36 (m, 1H); 4,94 (s, 1H); 5,28 (s, 1H); 6,00 (d, 1H); 6,35 (d, 1H)

**Beispiel 23**

(5*Z*,7*E*,22*E*)-(1*S*,3*R*)-25-(1-Oxooctyl)-26,27-cyclo-24a-homo-9,10-secocholesta-5,7,10(19),22-tetraen-1,3-diol **151**

135. Man setzt 200 mg des Aldehydes **2** mit 200 mg des Sulfons **122** in Analogie zu 61. um und erhält 272 mg (5*Z*,7*E*)-(1*S*,3*R*)-1,3-Bis[[dimethyl(1,1-dimethylethyl)silyl]oxy]-25-(2-heptyl-1,3-dioxolan-2-yl)-23-phenylsulfonyl-26,27-cyclo-24a-homo-9,10-secocholesta-5,7,10(19)-trien-22-ol **148** als farblosen Schaum.

136. 272 mg **148** werden analog 62. umgesetzt, wobei nacheinander 78 mg (5*Z*,7*E*,22*E*)-(1*S*,3*R*)-1,3-Bis[[dimethyl(1,1-dimethylethyl)silyl]oxy]-25-(2-heptyl-1,3-dioxolan-2-yl)-26,27-cyclo-24a-homo-9,10-secocholesta-5,7,10(19),22-tetraen **149** und 110 mg (5*Z*,7*E*)-(1*S*,3*R*,22*S*)-1,3-Bis[[dimethyl(1,1-dimethylethyl)silyl]oxy]-25-(2-heptyl-1,3-dioxolan-2-yl)-26,27-cyclo-24a-homo-9.10-secocholesta-5,7,10(19)-trien-22-ol **150** als farblose Schäume anfallen.
$^1$H-NMR (300 MHz, $CDCl_3$): **149:** δ= 0,05 ppm (s, 12H); 0,20 (m, 2H); 0,50 (m, 2H); 0,53 (s, 3H); 0,89 (s, 18H); 0,89 (t, 3H); 0,99 (d, 3H); 3,86 (m, 4H); 4,17 (m, 1H); 4,37 (m, 1H); 4,84 (s, 1H); 5,17 (s, 1H); 5,25 (m, 2H); 6,00 (d, 1H); 6,24 (d, 1H)
**150**: δ= 0,03 ppm (s, 12H); 0,18 (m, 2H); 0,52 (s, 3H); 0,52 (m, 2H); 0,88 (t, 3H); 0,88 (d, 3H); 0,89 (s, 18H); 3,60 (m, 1H); 3,83 (m, 4H); 4,17 (m, 1H); 4,37 (m, 1H); 4,82 (s, 1H); 5,17 (s, 1H); 6,00 (d, 1H); 6,24 (d, 1H)

137. Man behandelt 70 mg **149** analog 63. und erhält 27 mg der Titelverbindung **151** als farblosen Schaum. $^1$H-NMR (300 MHz, $CD_2Cl_2$): δ= 0,54 ppm (s, 3H); 0,70 (m, 2H); 0,88 (t, 3H); 0,99 (d, 3H); 1,10 (m, 2H); 2,22 (t, 2H); 4,17 (m, 1H); 4,37 (m, 1H); 4,95 (s, 1H); 5,29 (s, 1H); 5,30 (m, 2H); 5,99 (d, 1H); 6,35 (d, 1H)

**Beispiel 24**

138. (5*Z*,7*E*)-(1*S*,3*R*,22*S*)-25-(1-Oxooctyl)-26,27-cyclo-24a-homo-9,10-secocholesta-5,7,10(19)-trien-1,3,22-triol **152**

Man setzt 110 mg **150** in Analogie zu 63. um und erhält 38 mg der Titelverbindung als farblosen Schaum. $^1$H-NMR (300 MHz, $CD_2Cl_2$): δ= 0,54 ppm (s, 3H); 0,72 (m, 2H); 0,89 (d, 3H); 0,89 (t, 3H); 1,12 (m, 2H); 2,22 (t, 2H); 3,63 (m, 1H); 4,17 (m, 1H); 4,38 (m, 1H); 4,96 (s, 1H); 5,29 (s, 1H); 6,01 (d, 1H); 6,37 (d, 1H)

**Ausgangsmaterialien in der 24-Hydroxymethylen-24a,24b-dihomo-Reihe**

139. (*E*)-4-[1-(2-Methyl-1,3-dioxolan-2-yl)cyclopropyl]-3-buten-2-on **153**

Man legt 13,8 g Lithiumchlorid (wasserfrei) in 120 ml Acetonitril unter Stickstoff vor und gibt nacheinander 49,8 ml Oxopropylphoshonsäuredimethylester, 51,3 ml Diisopropyl-ethylamin und 5,4 g des Aldehydes **7** zu. Man rührt 18 h bei Raumtempertur und gibt dann Natriumchlorid-Lösung hinzu. Es wird mit Essigester extrahiert, mit Natriumchlorid-Lösung gewaschen und über Natriumsulfat getrocknet. Der Rückstand wird an Silicagel mit Essigester/Hexan chromatographiert, wobei 5,3 g der Titelverbindung als farbloses Öl anfallen.
$^1$H-NMR (300 MHz, $CDCl_3$): δ= 0,75 ppm (m, 2H); 1,10 (m, 2H); 1,42 (s, 3H); 2,25 (s, 3H); 3,96 (m, 4H); 6,00 (d, 1H); 7,10 (d, 1H)

140. 4-[1-(2-Methyl-1,3-dioxolan-2-yl)cyclopropyl]butan-2-on **154**

Man löst 1,23 g **153** in THF und fügt im Argon-Strom 200 mg Platin(IV)oxid zu. Mittels einer Hydrierapparatur hydriert man nun bis kein Wasserstoff mehr verbraucht wird, spült mit Stickstoff, filtriert und engt ein. Der Rückstand wird an Silicagel mit Essigester/Hexan chromatographiert, wobei 1,19 g der Titelverbindung **154** als farbloses Öl an-

fallen.
$^1$H-NMR (300 MHz, $CDCl_3$): δ= 0,23 ppm (m, 2H); 0,64 (m, 2H); 1,38 (s, 3H); 1,70 (dd, 2H); 2,15 (s, 3H); 2,64 (dd, 2H); 3,89 (m, 4H)

141. (*E*)-4-[1-(2-Propyl-1,3-dioxolan-2-yl)cyclopropyl]-3-buten-2-on **155**

Man setzt 2,0 g des Aldehydes **17** analog 139. um und erhält 2,1 g der Titelverbindung **155** als farbloses Öl.
$^1$H-NMR (300 MHz, $CDCl_3$): δ= 0,70 ppm (m, 2H); 0,90 (t, 3H); 1,09 (m, 2H); 1,40 (m, 2H); 2,27 (s, 3H); 3,97 (m, 4H); 5,92 (d, 1H); 7,18 (d, 1H)

142. 4-[1-(2-Propyl-1,3-dioxolan-2-yl)cyclopropyl]butan-2-on **156**

Man setzt 740 mg **155** analog 140. um und erhält 709 mg der Titelverbindung **156** als farbloses Öl.
$^1$H-NMR (300 MHz, $CDCl_3$): δ= 0,19 ppm (m, 2H); 0,60 (m, 2H); 0,91 (t, 3H); 2,13 (s, 3H); 2,65 (t, 2H); 3,90 (m, 4H)

143. (*E*)-4-[1-(2-Butyl-1,3-dioxolan-2-yl)cyclopropyl]-3-buten-2-on **157**

Man setzt 3,0 g des Aldehydes **27** analog 139. um und erhält 2,7 g der Titelverbindung **157** als farbloses Öl.
$^1$H-NMR (300 MHz, $CDCl_3$): δ= 0,72 ppm (m, 2H); 0,90 (t, 3H); 1,08 (m, 2H); 1,31 (m, 4H); 1,78 (m, 2H); 2,26 (s, 3H); 3,97 (m, 4H); 5,91 (d, 1H); 7,17 (d, 1H)

144. 4-[1-(2-Butyl-1,3-dioxolan-2-yl)cyclopropyl]butan-2-on **158**

Man setzt 1,6 g **157** analog 140. um und erhält 1,42 g der Titelverbindung **158** als farbloses Öl.
$^1$H-NMR (300 MHz, $CDCl_3$): δ= 0,19 ppm (m, 2H); 0,61 (m, 2H); 0,90 (t, 3H); 1,32 (m, 4H); 1,67 (m, 2H); 1,76 (m, 2H); 2,15 (s, 3H); 2,68 (t, 2H); 3,90 (m, 4H)

145. (*E*)-4-[1-(2-Pentyl-1,3-dioxolan-2-yl)cyclopropyl]-3-buten-2-on **159**

Man setzt 2,5 g des Aldehydes **37** analog 139. um und erhält 2,9 g der Titelverbindung **159** als farbloses Öl.
$^1$H-NMR (300 MHz, $CDCl_3$): δ= 0,71 ppm (m, 2H); 0,90 (t, 3H); 1,08 (m, 2H); 1,30 (m, 6H); 1,77 (m, 2H); 2,27 (s, 3H); 3,97 (m, 4H); 5,92 (d, 1H); 7,18 (d, 1H)

146. 4-[1-(2-Pentyl-1,3-dioxolan-2-yl)cyclopropyl]butan-2-on **160**

Man setzt 1,7 g **159** analog 140. um und erhält 1,51 g der Titelverbindung **160** als farbloses Öl.
$^1$H-NMR (300 MHz, $CDCl_3$): δ= 0,19 ppm (m, 2H); 0,61 (m, 2H); 0,90 (t, 3H); 1,32 (m, 6H); 1,75 (m, 2H); 2,17 (s, 3H); 2,64 (m, 2H); 3,89 (m, 4H)

147. (*E*)-4-[1-(2-Hexyl-1,3-dioxolan-2-yl)cyclopropyl]-3-buten-2-on **161**

Man setzt 3,44 g des Aldehydes **47** analog 139. um und erhält 2,3 g der Titelverbindung **161** als farbloses Öl.
$^1$H-NMR (300 MHz, $CDCl_3$): δ= 0,72 ppm (m, 2H); 0,89 (t, 3H); 1,08 (m, 2H); 1,29 (m, 8H); 1,77 (m, 2H); 2,27 (s, 3H); 3,96 (m, 4H); 5,92 (d, 1H); 7,18 (d, 1H)

148. 4-[1-(2-Hexyl-1,3-dioxolan-2-yl)cyclopropyl]butan-2-on **162**

Man setzt 800 mg **161** analog 140. um und erhält 710 g der Titelverbindung **162** als farbloses Öl.
$^1$H-NMR (300 MHz, $CDCl_3$): δ= 0,18 ppm (m, 2H); 0,59 (m, 2H); 0,89 (t, 3H); 1,29 (m, 8H); 1,63 (m, 2H); 1,74 (m, 2H); 2,13 (s, 3H); 2,65 (m, 2H); 3,90 (m, 4H)

**Beispiel 25**

(5*Z*,7*E*,22*E*)-(1*S*,3*R*,24*R*)-25-Acetyl-26,27-cyclo-24a,24b-dihomo-9,10-secocholesta-5,7,10(19),22-tetraen-1,3,24-triol **168a** und (5*Z*,7*E*,22*E*)-(1*S*,3*R*,24*S*)-25-Acetyl-26,27-cyclo-24a,24b-dihomo-9,10-secocholesta-5,7,10(19),22-tetraen-1,3,24-triol **168b**

149. Man stellt eine Lösung von 1 mmol Lithiumdiisopropylamid in 5 ml THF her, kühlt auf -78°C und tropft

202 mg des Ketons **154** in 1 ml THF zu. Es wird 30 min bei -78°C gerührt und anschließend werden 229 mg des Aldehydes **2** in **1** ml THF zugetropft. Man rührt weitere 30 min bei dieser Temperatur, quencht dann mit Natriumchlorid-Lösung, extrahiert mit Essigester, wäscht die organische Phase mit Natriumchlorid-Lösung, trocknet über Natriumsulfat und engt ein. Chromatographie des Rückstandes an Silicagel mit Essigester/Hexan ergibt 189 mg (5*Z*,7*E*)-(1*S*,3*R*)-1,3-Bis[[dimethyl(1,1-dimethylethyl)silyl]oxy]-22-hydroxy-25-(2-methyl-1,3-dioxolan-2-yl)-26,27-cyclo-24a,24b-dihomo-9,10-secocholesta-5,7,10(19)-trien-24-on **163** als farblosen Schaum, der direkt weiter umgesetzt werden.

150. Eine Lösung von 60 mg **163,** 0,2 ml Essigsäureanhydrid, 0,11 ml Triethylamin und einer Spatelspitze 4-Dimethylaminopyridin (DMAP) wird unter Stickstoff bei Raumtemperatur für 2 h gerührt. Man gibt nun Natriumhydrogencarbonat-Lösung zu, extrahiert mit Essigester, wäscht mit Natriumchlorid-Lösung, trocknet über Natriumsulfat und engt ein, wobei man 172 mg (5*Z*,7*E*)-(1*S*,3*R*)-22-Acetoxy-1,3-bis[[dimethyl(1,1-dimethylethyl)silyl]oxy]-25-(2-methyl-1,3-dioxolan-2-yl)-26,27-cyclo-24a,24b-dihomo-9,10-secocholesta-5,7,10(19)-trien-24-on **164** als farblosen Schaum erhält, der direkt weiter umgesetzt wird.

151. Man löst 172 mg **164** in 10 ml Toluol, gibt unter Stickstoff 1 ml Diazabicycloundecan (DBU) zu und erhitzt für 30 min auf 40°C. Anschließend wird eingeengt und der Rückstand an Silicagel mit Essigester/Hexan chromatographiert, wobei 129 mg (5*Z*,7*E*,22*E*)-(1*S*,3*R*)-1,3-Bis[[dimethyl(1,1-dimethylethyl)silyl]oxyl-25-(2-methyl-1,3-dioxolan-2-yl)-26,27-cydo-24a,24bdihomo-9,10-secocholesta-5,7,10(19),22-tetraen-24-on **165** als farbloser Schaum anfallen.
$^1$H-NMR (300 MHz, $CD_2Cl_2$): δ= 0,04 ppm (s, 12H); 0,21 (m, 2H); 0,54 (s, 3H); 0,60 (m, 2H); 0,86 (s, 18H); 1,09 (d, 3H); 1,33 (s, 3H); 3,83 (m, 4H); 4,17 (m, 1H); 4,35 (m, 1H); 4,82 (s, 1H); 5,16 (s, 1H); 5,94 (d, 1H); 6,01 (d, 1H); 6,23 (d, 1H); 6,63 (dd, 1H)

152. Man löst 130 mg **165** in 1 ml THF und gibt bei 0°C unter Stickstoff 5 ml Methanol, 75 mg Certrichlorid (Heptahydrat) und nach 10 min 8 mg Natriumborhydrid zu. Es wird 30 min bei 0°C gerührt und anschließend mit gesättigter Ammoniumchlorid-Lösung gequencht. Man extrahiert mit Essigester, trocknet über Natriumsulfat und engt ein. Der Rückstand wird an Silicagel mit Essigester/Hexan chromatographiert, wobei man nacheinander 60 mg (5*Z*, 7*E*,22*E*)-(1*S*,3*R*,24*R*)-1,3-Bis[[dimethyl(1,1-dimethylethyl)silyl]oxy]-25-(2-methyl-1,3-dioxolan-2-yl)-26,27-cyclo-24a, 24b-dihomo-9,10-secocholesta-5,7,10(19),22-tetraen-24-ol **166a** und 32 mg (5*Z*,7*E*,22*E*)-(1*S*,3*R*,24*S*)-1,3-B is[[dimethyl(1,1-dimethylethyl)silyl]oxy]-25-(2-methyl-1,3-dioxolan-2-yl)-26,27-cyclo-24a,24b-dihomo-9,10-secocholesta-5,7,10(19),22-tetraen-24-ol **166b** als farblose Schäume erhält.
$^1$H-NMR (300 MHz, $CD_2Cl_2$): **166a:** δ= 0,04 ppm (s, 12H); 0,22 (m, 2H); 0,53 (s, 3H); 0,56 (m, 2H); 0,86 (s, 18H); 1,01 (d, 3H); 1,32 (s, 3H); 3,83 (m, 4H); 3,91 (m, 1H); 4,17 (m, 1H); 4,36 (m, 1H); 4,82 (s, 1H); 5,16 (s, 1H); 5,33 (dd, 1H); 5,45 (dd, 1H); 6,00 (d, 1H); 6,23 (d, 1H)
**166b:** $^1$H-NMR (300 MHz, $CD_2Cl_2$): δ= 0,03 ppm (s, 12H); 0,22 (m, 2H); 0,53 (s, 3H); 0,56 (m, 2H); 0,86 (s, 18H); 1,01 (d, 3H); 1,32 (s, 3H); 3,82 (m, 4H); 3,85 (m, 1H); 4,17 (m, 1H); 4,36 (m, 1H); 4,82 (s, 1H); 5,16 (s, 1H); 5,30 (dd, 1H); 5,41 (dd, 1H); 6,00 (d, 1H); 6,23 (d, 1H)

153. Man legt 300 mg Silicagel in 10 ml Dichlormethan vor und gibt unter Stickstoff 0,3 ml wässrige Oxalsäure-Lösung (10%) zu. Man rührt 5 min bei Raumtemperatur und tropft dann 40 mg **166a** in 2 ml Dichlormethan hinzu. Es wird eine Stunde bei Raumtemperatur gerührt, Natriumhydrogencarbonat zugegeben, abfiltriert und eingeengt, wobei 39 mg
(5*Z*,7*E*,22*E*)-(1*S*,3*R*,24*R*)-25-Acetyl-1,3-bis[[dimethyl(1,1-dimethylethyl)silyl]oxy]-26,27-cyclo-24a,24b-dihomo-9,10-secocholesta-5,7,10(19),22-tetraen-24-ol **167a** als farbloser Schaum anfallen, die direkt weiter umgesetzt werden.

154. Man löst 38 mg **167a** in 5 ml THF, gibt 150 mg Tetrabutylammoniumfluorid (Hydrat) hinzu und rührt unter Stickstoff für 12 h bei Raumtemperatur nach. Anschließend wird Natriumchlorid-Lösung zugegeben, mit Essigester extrahiert, mit Natriumchlorid-Lösung gewaschen, über Natriumsulfat getrocknet und eingeengt. Der Rückstand wird an Silicagel mit Essigester/Hexan chromatographiert, wobei 9 mg der Titelverbindung **168a** als farbloser Schaum anfallen.
$^1$H-NMR (300 MHz, $CD_2Cl_2$): δ= 0,55 ppm (s, 3H); 0,78 (m, 2H); 1,02 (d, 3H); 1,18 (m, 2H); 1,93 (s, 3H); 3,92 (m, 1H); 4,16 (m, 1H); 4,37 (m, 1H); 4,95 (s, 1H); 5,28 (s, 1H); 5,36 (dd, 1H); 5,58 (dd, 1H); 5,99 (d, 1H); 6,31 (d, 1H)

155. Man setzt 32 mg **166b** analog 153. um, wobei man 24 mg (5*Z*,7*E*,22*E*)-(1*S*,3*R*,24*S*)-25-Acetyl-1,3-bis[[dimethyl(1,1-dimethylethyl)silyl]oxy]-26,27-cyclo-24a,24b-dihomo-9,10-secocholesta-5,7,10(19),22-tetraen-24-ol **167b** als farblosen Schaum erhält, der direkt weiter umgesetzt wird.

156. Man setzt 24 mg **167b** analog 154. um, wobei 6 mg der Titelverbindung **168b** als farbloser Schaum anfallen.
$^1$H-NMR (300 MHz, $CD_2Cl_2$): δ= 0,55 ppm (s, 3H); 0,77 (m, 2H); 1,03 (d, 3H); 1,20 (m, 2H); 1,93 (s, 3H); 3,91 (m, 1H); 4,16 (m, 1H); 4,37 (m, 1H); 4,95 (s, 1H); 5,28 (s, 1H); 5,32 (dd, 1H); 5,45 (dd, 1H); 5,99 (d, 1H); 6,31 (d, 1H)

**Beispiel 26**

157. (5*Z*,7*E*,22*E*)-(1*S*,3*R*,24*R*)-25-Acetyl-24-methoxy-26,27-cyclo-24a,24bdihomo-9,10-secocholesta-5,7,10(19), 22-tetraen-1,3-diol **169a** und (5*Z*,7*E*,22*E*)-(1 *S*,3*R*,24*S*)-25-Acetyl-24-methoxy-26,27-cyclo-24a,24b-dihomo-9,10-secocholesta-5,7,10(19),22-tetraen-1,3-diol **169b**

Man löst 58 mg **166a** in 5 ml Dichlormethan/Methanol (1:1), fügt 380 mg Dowex-lonentauscher hinzu und rührt 12 h unter Stickstoff bei Raumtemperatur. Es wird anschließend abfiltriert, das Filtrat mit Natriumhydrogencarbonat-Lösung gewaschen, über Natriumsulfat getrocknet und eingeengt. Der Rückstand wird an Silicagel mit Essigester/Hexan chromatographiert, wobei 32 mg eines Diastereomerengermisches anfallen. Durch HPLC-Trennung erhält man dann nacheinander 11 mg der Titelverbindung **169b** und 13 mg der Titelverbindung **169a** als farblose Schäume.
$^1$H-NMR (300 MHz, $CD_2Cl_2$): **169a**: δ= 0,56 ppm (s, 3H); 0,72 (m, 2H); 1,07 (d, 3H); 1,15 (m, 2H); 1,97 (s, 3H); 3,19 (s, 3H); 3,39 (m, 1H); 4,18 (m, 1H); 4,38 (m, 1H); 4,96 (s, 1H); 5,12 (dd, 1H); 5,29 (s, 1H); 5,55 (dd, 1H); 6,00 (d, 1H); 6,35 (d, 1H)
**169b**: δ= 0,56 ppm (s, 3H); 0,72 (m, 2H); 1,07 (d, 3H); 1,15 (m, 2H); 1,96 (s, 3H); 3,18 (s, 3H); 3,39 (m, 1H); 4,17 (m, 1H); 4,37 (m, 1H); 4,96 (s, 1H); 5,13 (dd, 1H); 5,29 (s, 1H); 5,55 (dd, 1H); 6,00 (d, 1H); 6,36 (d, 1H)

**Beispiel 27**

(5*Z*,7*E*,22*E*)-(1*S*,3*R*,24*R*)-25-(1-Oxobutyl)-26,27-cyclo-24a,24b-dihomo-9,10-secocholesta-5,7,10(19),22-tetraen-1,3,24-triol **175a** und (5*Z*,7*E*,22*E*)-(1*S*,3*R*,24*S*)-25-(1-Oxobutyl)-26,27-cyclo-24a,24b-dihomo-9,10-secocholesta-5,7,10(19),22-tetraen-1,3,24-triol **175b**

158. Man setzt 700 mg des Ketons **156** mit 350 mg des Aldehydes **2** analog 149. um und erhält 490 mg (5*Z*, 7*E*)-(1*S*,3*R*)-1,3-Bis[[dimethyl(1,1-dimethylethyl)silyl]oxy]-22-hydroxy-25-(2-propyl-1,3-dioxolan-2-yl)-26,27-cyclo-24a,24b-dihomo-9,10-secocholesta-5,7,10(19)-trien-24-on **170** als farblosen Schaum, der direkt weiter umgesetzt wird.

159. Eine Lösung von 490 mg **170** wird analog 150. umgesetzt, wobei man 505 mg (5*Z*,7*E*)-(1*S*,3*R*)-22-Acetoxy-1,3-Bis[[dimethyl(1,1-dimethylethyl)silyl]oxy]-25-(2-propyl-1,3-dioxolan-2-yl)-26,27-cyclo-24a,24b-dihomo-9,10-secocholesta-5,7,10(19)-trien-24-on **171** als farblosen Schaum erhält, der direkt weiter umgesetzt wird.

160. Man setzt 505 mg **171** analog 151. um, wobei 290 mg (5*Z*,7*E*,22*E*)-(1*S*,3*R*)-1,3-Bis[[dimethyl(1,1-dimethylethyl)silyl]oxy]-25-(2-propyl-1,3-dioxolan-2-yl)-26,27-cydo-24a,24b-dihomo-9,10-secocholesta-5,7,10(19),22-tetraen-24-on **172** als farbloser Schaum anfallen.
$^1$H-NMR (300 MHz, $CD_2Cl_2$): δ= 0,06 ppm (s, 12H); 0,19 (m, 2H); 0,54 (s, 3H); 0,58 (m, 2H); 0,86 (s, 18H); 0,92 (t, 3H); 1,08 (d, 3H); 3,84 (m, 4H); 4,17 (m, 1H); 4,36 (m, 1H); 4,83 (s, 1H); 5,16 (s, 1H); 5,96 (d, 1H); 6,02 (d, 1H); 6,24 (d, 1H); 6,64 (dd, 1H)

161. Man setzt 350 mg **172** analog 152. um und erhält nach Chromatographie an Silicagel mit Essigester/Hexan nacheinander 160 mg (5*Z*,7*E*,22*E*)-(1*S*,3*R*,24*R*)-1,3-Bis[[dimethyl(1,1-dimethylethyl)silyl]oxy]-25-(2-propyl-1,3-dioxolan-2-yl)-26,27-cyclo-24a,24b-dihomo-9,10-secocholesta-5,7,10(19),22-tetraen-24-ol **173a** und 109 mg (5*Z*, 7*E*,22*E*)-(1*S*,3*R*,24*S*)-1,3-Bis[[dimethyl(1,1-dimethylethyl)silyl]oxy]-25-(2-propyl-1,3-dioxolan-2-yl)-26,27-cyclo-24a, 24bdihomo-9,10-secocholesta-5,7,10(19),22-tetraen-24-ol **173b** als farblose Schäume.
$^1$H-NMR (300 MHz, $CD_2Cl_2$): **173a:** δ= 0,03 ppm (s, 12H); 0,18 (m, 2H); 0,53 (s, 3H); 0,55 (m, 2H); 0,87 (s, 18H); 0,90 (t, 3H); 1,02 (d, 3H); 3,83 (m, 4H); 3,90 (m, 1H); 4,16 (m, 1H); 4,35 (m, 1H); 4,83 (s, 1H); 5,15 (s, 1H); 5,31 (dd, 1H); 5,43 (dd, 1H); 6,00 (d, 1H); 6,23 (d, 1H)
**173b**: $^1$H-NMR (300 MHz, $CD_2Cl_2$): δ= 0,03 ppm (s, 12H); 0,17 (m, 2H); 0,54 (s, 3H); 0,55 (m, 2H); 0,87 (s, 18H); 0,90 (t, 3H); 1,02 (d, 3H); 3,84 (m, 4H); 3,85 (m, 1H); 4,16 (m, 1H); 4,36 (m, 1H); 4,83 (s, 1H); 5,15 (s, 1H); 5,30 (dd, 1H); 5,41 (dd, 1H); 6,00 (d, 1H); 6,23 (d, 1H)

162. Man setzt 85 mg **173a** analog 153. um, wobei 59 mg (5*Z*,7*E*,22*E*)-(1 *S*,3*R*,24*R*)-1,3-Bis[[dimethyl(1,1-dimethylethyl)silyl]oxy]-25-(1-oxobutyl)-26,27-cyclo-24a,24b-dihomo-9,10-secocholesta-5,7,10(19),22-tetraen-24-ol **174a** als farbloser Schaum anfallen, die direkt weiter umgesetzt werden.

163. Man setzt 59 mg **174a** analog 154. um, wobei 19 mg der Titelverbindung **175a** als farbloser Schaum anfallen.
$^1$H-NMR (300 MHz, $CD_2Cl_2$): δ= 0,55 ppm (s, 3H); 0,72 (m, 2H); 0,89 (t, 3H); 1,02 (d, 3H); 1,16 (m, 2H); 2,18 (t, 2H); 3,92 (m, 1H); 4,17 (m, 1H); 4,37 (m, 1H); 4,95 (s, 1H); 5,29 (s, 1H); 5,35 (dd, 1H); 5,51 (dd, 1H); 5,99 (d, 1H); 6,34 (d, 1H)

164. Man setzt 61 mg **173b** analog 153. um, wobei man 45 mg (5*Z*,7*E*,22*E*)-(1*S*,3*R*,24*S*)-1,3-Bis[[dimethyl (1,1-dimethylethyl)silyl]oxy]-25-(1-oxobutyl)-26,27-cyclo-24a,24b-dihomo-9,10-secocholesta-5,7,10(19),22-tetraen-24-ol **174b** als farblosen Schaum erhält, der direkt weiter umgesetzt wird.

165. Man setzt 45 mg **174b** analog 154. um, wobei 21 mg der Titelverbindung **175b** als farbloser Schaum

anfallen.
$^1H$-NMR (300 MHz, $CD_2Cl_2$): δ= 0,56 ppm (s, 3H); 0,72 (m, 2H); 0,88 (t, 3H); 1,02 (d, 3H); 1,15 (m, 2H); 2,20 (t, 2H); 3,92 (m, 1H); 4,18 (m, 1H); 4,38 (m, 1H); 4,95 (s, 1H); 5,28 (s, 1H); 5,33 (dd, 1H); 5,46 (dd, 1H); 6,00 (d, 1H); 6,34 (d, 1H)

## Beispiel 28

166. (5Z,7E,22E)-(1*S*,3*R*,24*R*)-24-Methoxy-25-(1-oxobutyl)-26,27-cyclo-24a,24bdihomo-9,10-secocholesta-5,7,10(19),22-tetraen-1,3-diol **176a** und (5*Z*,7*E*,22*E*)-(1*S*,3*R*,24*S*)-24-Methoxy-25-(1-oxobutyl)-26,27-cyclo-24a,24b-dihomo-9,10-secocholesta-5,7,10(19),22-tetraen-1,3-diol **176b**

Man setzt **71** mg **173a** analog 157. um und erhält 21 mg der Titelverbindung **176b** und 18 mg der Titelverbindung **176a** als farblose Schäume.
$^1H$-NMR (300 MHz, $CD_2Cl_2$): **173a:** δ= 0,54 ppm (s, 3H); 0,70 (m, 2H); 0,86 (t, 3H); 1,06 (d, 3H); 1,13 (m, 2H); 3,15 (s, 3H); 3,38 (m, 1H); 4,16 (m, 1H); 4,36 (m, 1H); 4,96 (s, 1H); 5,14 (dd, 1H); 5,28 (s, 1H); 5,53 (dd, 1H); 5,99 (d, 1H); 6,35 (d, 1H)
**173b**: δ= 0,55 ppm (s, 3H); 0,70 (m, 2H); 0,87 (t, 3H); 1,06 (d, 3H); 1,14 (m, 2H); 3,16 (s, 3H); 3,39 (m, 1H); 4,17 (m, 1H); 4,37 (m, 1H); 4,96 (s, 1H); 5,14 (dd, 1H); 5,29 (s, 1H); 5,52 (dd, 1H); 6,00 (d, 1H); 6,35 (d, 1H)

## Beispiel 29

(5*Z*,7*E*,22*E*)-(1*S*,3*R*,24*R*)-25-(1-Oxopentyl)-26,27-cyclo-24a,24b-dihomo-9,10-secocholesta-5,7,10(19),22-tetraen-1,3,24-triol **182a** und (5*Z*,7*E*,22*E*)-(1 *S*,3*R*,24*S*)-25-(1-Oxopentyl)-26,27-cyclo-24a,24b-dihomo-9,10-secocholesta-5,7,10(19),22-tetraen-1,3,24-triol **182b**

167. Man setzt 100 mg des Ketons **158** mit 200 mg des Aldehydes **2** analog 149. um und erhält 120 mg (5*Z*,7*E*)-(1*S*,3*R*)-1,3-Bis[[dimethyl(1,1-dimethylethyl)silyl]oxy]-25-(2-butyl-1,3-dioxolan-2-yl)-22-hydroxy-26,27-cyclo-24a,24b-dihomo-9,10-secocholesta-5,7,10(19)-trien-24-on **177** als farblosen Schaum, der direkt weiter umgesetzt wird.
168. Eine Lösung von 120 mg **177** wird analog 150. umgesetzt, wobei man 134 mg (5*Z*,7*E*)-(1*S*,3*R*)-22-Acetoxy-1,3-Bis[[dimethyl(1,1-dimethylethyl)silyl]oxy]-25-(2-butyl-1,3-dioxolan-2-yl)-26,27-cyclo-24a,24b-dihomo-9,10-secocholesta-5,7,10(19)-trien-24-on **178** als farblosen Schaum erhält, der direkt weiter umgesetzt wird.
169. Man setzt 134 mg **178** analog 151. um, wobei 72 mg (5*Z*,7*E*,22*E*)-(1*S*,3*R*)-1,3-Bis[[dimethyl(1,1-dimethylethyl)silyl]oxy]-25-(2-butyl-1,3-dioxolan-2-yl)-26,27-cyclo-24a,24b-dihomo-9,10-secocholesta-5,7,10(19),22-tetraen-24-on **179** als farbloser Schaum anfallen.
$^1H$-NMR (300 MHz, $CD_2Cl_2$): δ= 0,03 ppm (s, 12H); 0,18 (m, 2H); 0,56 (s, 3H); 0,57 (m, 2H); 0,89 (s, 18H); 0,90 (t, 3H); 1,09 (d, 3H); 3,85 (m, 4H); 4,17 (m, 1H); 4,36 (m, 1H); 4,85 (s, 1H); 5,16 (s, 1H); 5,96 (d, 1H); 5,98 (d, 1H); 6,24 (d, 1H); 6,65 (dd, 1H)
170. Man setzt 70 mg **179** analog 152. um und erhält nach Chromatographie an Silicagel mit Essigester/Hexan nacheinander 28 mg (5*Z*,7*E*,22*E*)-(1*S*,3*R*,24*R*)-1,3-Bis[[dimethyl(1,1-dimethylethyl)silyl]oxy]-25-(2-butyl-1,3-dioxolan-2-yl)-26,27-cyclo-24a,24b-dihomo-9,10-secocholesta-5,7,10(19),22-tetraen-24-ol **180a** und 29 mg (5*Z*,7*E*,22*E*)-(1*S*,3*R*,24*S*)-1,3-Bis[[dimethyl(1,1-dimethylethyl)silyl]oxy]-25-(2-butyl-1,3-dioxolan-2-yl)-26,27-cyclo-24a,24b-dihomo-9,10-secocholesta-5,7,10(19),22-tetraen-24-ol **180b** als farblose Schäume.
$^1H$-NMR (300 MHz, $CD_2Cl_2$): **180a**: δ= 0,05 ppm (s, 12H); 0,17 (m, 2H); 0,54 (s, 3H); 0,55 (m, 2H); 0,89 (s, 18H); 0,90 (t, 3H); 1,02 (d, 3H); 3,85 (m, 4H); 3,92 (m, 1H); 4,17 (m, 1H); 4,37 (m, 1H); 4,84 (s, 1H); 5,17 (s, 1H); 5,32 (dd, 1H); 5,46 (dd, 1H); 6,01 (d, 1H); 6,25 (d, 1H)
**180b**: $^1H$-NMR (300 MHz, $CD_2Cl_2$): δ= 0,05 ppm (s, 12H); 0,18 (m, 2H); 0,54 (s, 3H); 0,55 (m, 2H); 0,89 (s, 18H); 0,90 (t, 3H); 1,02 (d, 3H); 3,84 (m, 4H); 3,90 (m, 1H); 4,17 (m, 1H); 4,37 (m, 1H); 4,84 (s, 1H); 5,16 (s, 1H); 5,32 (dd, 1H); 5,42 (dd, 1H); 6,01 (d, 1H); 6,25 (d, 1H)
171. Man setzt 25 mg **180a** analog 153. um, wobei 19 mg (5*Z*,7*E*,22*E*)-(1*S*,3*R*,24*R*)-1,3-Bis[[dimethyl(1,1-dimethylethyl)silyl]oxy]-25-(1-oxopentyl)-26,27-cyclo-24a,24b-dihomo-9,10-secocholesta-5,7,10(19),22-tetraen-24-ol **181a** als farbloser Schaum anfallen, die direkt weiter umgesetzt werden.
172. Man setzt 19 mg **181a** analog 154. um, wobei 5 mg der Titelverbindung **182a** als farbloser Schaum anfallen.
$^1H$-NMR (300 MHz, $CD_2Cl_2$): δ= 0,54 ppm (s, 3H); 0,73 (m, 2H); 0,86 (t, 3H); 1,01 (d, 3H); 1,14 (m, 2H); 2,19 (t, 2H); 3,93 (m, 1H); 4,16 (m, 1H); 4,36 (m, 1H); 4,94 (s, 1H); 5,28 (s, 1H); 5,34 (dd, 1H); 5,48 (dd, 1H); 6,00 (d, 1H); 6,34 (d, 1H)
173. Man setzt 23 mg **180b** analog 1534. um, wobei man 20 mg (5*Z*,7*E*,22*E*)-(1 *S*,3*R*,24*S*)-1,3-Bis[[dimethyl(1,1-dimethylethyl)silyl]oxy]-25-(1-oxopentyl)-26,27-cyclo-24a,24b-dihomo-9,10-secocholesta-5,7,10(19),22-tetraen-24-ol **181b** als farblosen Schaum erhält, der direkt weiter umgesetzt wird.
174. Man setzt 20 mg **181b** analog 1545. um, wobei 4,5 mg der Titelverbindung **182b** als farbloser Schaum

anfallen.
$^1$H-NMR (300 MHz, $CD_2Cl_2$): δ= 0,54 ppm (s, 3H); 0,72 (m, 2H); 0,88 (t, 3H); 1,01 (d, 3H); 1,13 (m, 2H); 2,19 (t, 2H); 3,90 (m, 1H); 4,17 (m, 1H); 4,37 (m, 1H); 4,95 (s, 1H); 5,28 (s, 1H); 5,31 (dd, 1H); 5,45 (dd, 1H); 6,00 (d, 1H); 6,34 (d, 1H)

**Beispiel 30**

(5*Z*,7*E*,22*E*)-(1*S*,3*R*,24*R*)-25-(1-Oxohexyl)-26,27-cyclo-24a,24b-dihomo-9,10-secocholesta-5,7,10(19),22-tetraen-1,3,24-triol **188a** und (5*Z*,7*E*,22*E*)-(1 *S*,3*R*,24*S*)-25-(1-Oxohexyl)-26,27-cyclo-24a,24b-dihomo-9,10-secocholesta-5,7,10(19),22-tetraen-1,3,24-triol **188b**

175. Man setzt 180 mg des Ketons **160** mit 406 mg des Aldehydes **2** analog 149. um und erhält 195 mg (5*Z*,7*E*)-(1*S*,3*R*)-1,3-Bis[[dimethyl(1,1-dimethylethyl)silyl]oxy]-22-hydroxy-25-(2-pentyl-1,3-dioxolan-2-yl)-26,27-cyclo-24a,24b-dihomo-9,10-secocholesta-5,7,10(19)-trien-24-on **183** als farblosen Schaum, der direkt weiter umgesetzt wird.

176. Eine Lösung von 195 mg **183** wird analog 150. umgesetzt, wobei man 210 mg (5*Z*,7*E*)-(1*S*,3*R*)-22-Acetoxy-1,3-Bis[[dimethyl(1,1-dimethylethyl)silyl]oxy]-25-(2-pentyl-1,3-dioxolan-2-yl)-26,27-cyclo-24a,24b-dihomo-9,10-secocholesta-5,7,10(19)-trien-24-on **184** als farblosen Schaum erhält, der direkt weiter umgesetzt wird.

177. Man setzt 210 mg **184** analog 151. um, wobei 165 mg (5*Z*,7*E*,22*E*)-(1*S*,3*R*)-1,3-Bis[[dimethyl(1,1-dimethylethyl)silyl]oxy]-25-(2-pentyl-1,3-dioxolan-2-yl)-26,27-cyclo-24a,24b-dihomo-9,10-secocholesta-5,7,10(19),22-tetraen-24-on **185** als farbloser Schaum anfallen.
$^1$H-NMR (300 MHz, $CD_2Cl_2$): δ= 0,05 ppm (s, 12H); 0,17 (m, 2H); 0,56 (s, 3H); 0,57 (m, 2H); 0,88 (s, 18H); 0,89 (t, 3H); 1,08 (d, 3H); 3,83 (m, 4H); 4,16 (m, 1H); 4,36 (m, 1H); 4,83 (s, 1H); 5,15 (s, 1H); 5,94 (d, 1H); 6,00 (d, 1H); 6,24 (d, 1H); 6,64 (dd, 1H)

178. Man setzt 165 mg **185** analog 152. um und erhält nach Chromatographie an Silicagel mit Essigester/Hexan nacheinander 46 mg (5*Z*,7*E*,22*E*)-(1*S*,3*R*,24*R*)-1,3-Bis[[dimethyl(1,1-dimethylethyl)silyl]oxy]-25-(2-pentyl-1,3-dioxolan-2-yl)-26,27-cyclo-24a,24b-dihomo-9,10-secocholesta-5,7,10(19),22-tetraen-24-ol **186a** und 35 mg (5*Z*,7*E*,22*E*)-(1*S*,3*R*,24*S*)-1,3-Bis[[dimethyl(1,1-dimethylethyl)silyl]oxy]-25-(2-pentyl-1,3-dioxolan-2-yl)-26,27-cyclo-24a,24b-dihomo-9,10-secocholesta-5,7,10(19),22-tetraen-24-ol **186b** als farblose Schäume.
$^1$H-NMR (300 MHz, $CD_2Cl_2$): **186a**: δ= 0,05 ppm (s, 12H); 0,17 (m, 2H); 0,54 (s, 3H); 0,55 (m, 2H); 0,88 (s, 18H); 0,89 (t, 3H); 1,03 (d, 3H); 3,85 (m, 4H); 3,91 (m, 1H); 4,17 (m, 1H); 4,36 (m, 1H); 4,84 (s, 1H); 5,17 (s, 1H); 5,33 (dd, 1H); 5,47 (dd, 1H); 6,01 (d, 1H); 6,25 (d, 1H)
**186b**: $^1$H-NMR (300 MHz, $CD_2Cl_2$): δ= 0,05 ppm (s, 12H); 0,17 (m, 2H); 0,54 (s, 3H); 0,55 (m, 2H); 0,88 (s, 18H); 0,89 (t, 3H); 1,02 (d, 3H); 3,85 (m, 4H); 3,88 (m, 1H); 4,16 (m, 1H); 4,36 (m, 1H); 4,84 (s, 1H); 5,16 (s, 1H); 5,30 (dd, 1H); 5,42 (dd, 1H); 6,01 (d, 1H); 6,24 (d, 1H)

179. Man setzt 46 mg **186a** analog 153. um, wobei 39 mg (5*Z*,7*E*,22*E*)-(1 *S*,3*R*,24*R*)-1,3-Bis[[dimethyl(1,1-dimethylethyl)silyl]oxy]-25-(1-oxohexyl)-26,27-cyclo-24a,24b-dihomo-9,10-secocholesta-5,7,10(19),22-tetraen-24-ol **187a** als farbloser Schaum anfallen, die direkt weiter umgesetzt werden.

180. Man setzt 39 mg **187a** analog 154. um, wobei 11 mg der Titelverbindung **188a** als farbloser Schaum anfallen.
$^1$H-NMR (300 MHz, $CD_2Cl_2$): δ= 0,54 ppm (s, 3H); 0,71 (m, 2H); 0,86 (t, 3H); 1,00 (d, 3H); 1,12 (m, 2H); 2,18 (t, 2H); 3,93 (m, 1H); 4,16 (m, 1H); 4,36 (m, 1H); 4,94 (s, 1H); 5,28 (s, 1H); 5,34 (dd, 1H); 5,48 (dd, 1H); 5,99 (d, 1H); 6,33 (d, 1H)

181. Man setzt 35 mg **186b** analog 153. um, wobei man 27 mg (5*Z*,7*E*,22*E*)-(1*S*,3*R*,24*S*)-1,3-Bis[[dimethyl(1,1-dimethylethyl)silyl]oxy]-25-(1-oxohexyl)-26,27-cyclo-24a,24b-dihomo-9,10-secocholesta-5,7,10(19),22-tetraen-24-ol **187b** als farblosen Schaum erhält, der direkt weiter umgesetzt wird.

182. Man setzt 27 mg **187b** analog 154. um, wobei 8,5 mg der Titelverbindung **188b** als farbloser Schaum anfallen.
$^1$H-NMR (300 MHz, $CD_2Cl_2$): δ= 0,55 ppm (s, 3H); 0,72 (m, 2H); 0,88 (t, 3H); 1,01 (d, 3H); 1,15 (m, 2H); 2,19 (t, 2H); 3,92 (m, 1H); 4,17 (m, 1H); 4,37 (m, 1H); 4,95 (s, 1H); 5,27 (s, 1H); 5,33 (dd, 1H); 5,46 (dd, 1H); 6,00 (d, 1H); 6,34 (d, 1H)

**Beispiel 31**

(5*Z*,7*E*,22*E*)-(1*S*,3*R*,24*R*)-25-(1 -Oxoheptyl)-26,27-cyclo-24a,24b-dihomo-9,10-secocholesta-5,7,10(19),22-tetraen-1 ,3,24-triol **194a** und (5*Z*,7*E*,22*E*)-(1 *S*, 3*R*,24*S*)-25-(1-Oxoheptyl)-26,27-cyclo-24a,24b-dihomo-9,10-secocholesta-5,7,10(19),22-tetraen-1,3,24-triol **194b**

183. Man setzt 710 mg des Ketons **162** mit 573 mg des Aldehydes **2** analog 149. um und erhält 580 mg (5*Z*,7*E*)-(1*S*,3*R*)-1,3-Bis[[dimethyl(1,1-dimethylethyl)silyl]oxy]-25-(2-hexyl-1,3-dioxolan-2-yl)-22-hydroxy-26,27-cyclo-24a,24b-dihomo-9,10-secocholesta-5,7,10(19)-trien-24-on **189** als farblosen Schaum, der direkt weiter umgesetzt

wird.

184. Eine Lösung von 580 mg **189** wird analog 150. umgesetzt, wobei man 603 mg (5*Z*,7*E*)-(1*S*,3*R*)-22-Acetoxy-1,3-Bis[[dimethyl(1,1-dimethylethyl)silyl]oxy]-25-(2-hexyl-1,3-dioxolan-2-yl)-26,27-cyclo-24a,24b-dihomo-9,10-secocholesta-5,7,10(19)-trien-24-on **190** als farblosen Schaum erhält, der direkt weiter umgesetzt wird.

185. Man setzt 603 mg **190** analog 151. um, wobei 304 mg (5*Z*,7*E*,22*E*)-(1*S*,3*R*)-1,3-Bis[[dimethyl(1,1-dimethylethyl)silyl]oxy]-25-(2-hexyl-1,3-dioxolan-2-yl)-26,27-cyclo-24a,24b-dihomo-9,10-secocholesta-5,7,10(19),22-tetraen-24-on **191** als farbloser Schaum anfallen.

$^1$H-NMR (300 MHz, $CD_2Cl_2$): δ= 0,05 ppm (s, 12H); 0,19 (m, 2H); 0,57 (s, 3H); 0,59 (m, 2H); 0,89 (s, 18H); 0,90 (t, 3H); 1,09 (d, 3H); 3,85 (m, 4H); 4,17 (m, 1H); 4,37 (m, 1H); 4,85 (s, 1H); 5,16 (s, 1H); 5,96 (d, 1H); 6,01 (d, 1H); 6,24 (d, 1H); 6,66 (dd, 1H)

186. Man setzt 295 mg **191** analog 152. um und erhält nach Chromatographie an Silicagel mit Essigester/Hexan nacheinander 130 mg (5*Z*,7*E*,22*E*)-(1*S*,3*R*,24*R*)-1,3-Bis[[dimethyl(1,1-dimethylethyl)silyl]oxy]-25-(2-hexyl-1,3-dioxolan-2-yl)-26,27-cyclo-24a,24b-dihomo-9,10-secocholesta-5,7,10(19),22-tetraen-24-ol **192a** und 80 mg (5*Z*,7*E*,22*E*)-(1*S*,3*R*,24*S*)-1,3-Bis[[dimethyl(1,1-dimethylethyl)silyl]oxy]-25-(2-hexyl-1,3-dioxolan-2-yl)-26,27-cyclo-24a,24b-dihomo-9,10-secocholesta-5,7,10(19),22-tetraen-24-ol **192b** als farblose Schäume.

$^1$H-NMR (300 MHz, $CD_2Cl_2$): **192a:** δ= 0,03 ppm (s, 12H); 0,18 (m, 2H); 0,56 (s, 3H); 0,56 (m, 2H); 0,89 (s, 18H); 0,90 (t, 3H); 1,04 (d, 3H); 3,86 (m, 4H); 3,93 (m, 1H); 4,18 (m, 1H); 4,38 (m, 1H); 4,86 (s, 1H); 5,19 (s, 1H); 5,35 (dd, 1H); 5,48 (dd, 1H); 6,01 (d, 1H); 6,26 (d, 1H)

**192b**: $^1$H-NMR (300 MHz, $CD_2Cl_2$): δ= 0,03 ppm (s, 12H); 0,18 (m, 2H); 0,55 (s, 3H); 0,55 (m, 2H); 0,88 (s, 18H); 0,89 (t, 3H); 1,03 (d, 3H); 3,84 (m, 4H); 3,89 (m, 1H); 4,16 (m, 1H); 4,36 (m, 1H); 4,85 (s, 1H); 5,17 (s, 1H); 5,31 (dd, 1H); 5,43 (dd, 1H); 6,01 (d, 1H); 6,24 (d, 1H)

187. Man setzt 80 mg **192a** analog 153. um, wobei 64 mg (5*Z*,7*E*,22*E*)-(1 *S*,3*R*,24*R*)-1,3-Bis[[dimethyl(1,1-dimethylethyl)silyl]oxy]-25-(1-oxoheptyl)-26,27-cyclo-24a,24b-dihomo-9,10-secocholesta-5,7,10(19),22-tetraen-24-ol **193a** als farbloser Schaum anfallen, die direkt weiter umgesetzt werden.

188. Man setzt 64 mg **193a** analog 154. um, wobei 21 mg der Titelverbindung **194a** als farbloser Schaum anfallen.

$^1$H-NMR (300 MHz, $CD_2Cl_2$): δ= 0,56 ppm (s, 3H); 0,71 (m, 2H); 0,87 (t, 3H); 1,01 (d, 3H); 1,14 (m, 2H); 2,20 (t, 2H); 3,93 (m, 1H); 4,16 (m, 1H); 4,36 (m, 1H); 4,95 (s, 1H); 5,27 (s, 1H); 5,35 (dd, 1 H); 5,48 (dd, 1H); 5,99 (d, 1H); 6,34 (d, 1H)

189. Man setzt 76 mg **192b** analog 153. um, wobei man 57 mg (5*Z*,7*E*,22*E*)-(1*S*,3*R*,24*S*)-1,3-Bis[[dimethyl(1,1-dimethylethyl)silyl]oxy]-25-(1-oxoheptyl)-26,27-cyclo-24a,24b-dihomo-9,10-secocholesta-5,7,10(19),22-tetraen-24-ol **193b** als farblosen Schaum erhält, der direkt weiter umgesetzt wird.

1901. Man setzt 57 mg **193b** analog 154. um, wobei 17 mg der Titelverbindung **194b** als farbloser Schaum anfallen.

$^1$H-NMR (300 MHz, $CD_2Cl_2$): δ= 0,56 ppm (s, 3H); 0,73 (m, 2H); 0,88 (t, 3H); 1,01 (d, 3H); 1,15 (m, 2H); 2,19 (t, 2H); 3,91 (m, 1H); 4,17 (m, 1H); 4,37 (m, 1H); 4,95 (s, 1H); 5,28 (s, 1H); 5,32 (dd, 1H); 5,47 (dd, 1H); 6,00 (d, 1H); 6,35 (d, 1H)

**Synthese der Ausgangsmaterialien in der 25-Alkyl-Reihe**

191. 2-(1-Ethylcyclopropyl)-2-methyl-1,3-dioxolan **195**

Man löst 500 mg des Olefins **8** in 15 ml Essigester, fügt 150 mg Platin(IV)oxid hinzu und hydriert in einer Hydrierapparatur bis kein Wasserstoff mehr aufgenommen wird. Nach Filtration engt man ein, wobei man 398 mg der Titelverbindung **195** als farbloses Öl erhält, das direkt weiter umgesetzt wird.

192. 1-(1-Ethylcyclopropyl)-1-ethanon **196**

Man löst 370 mg **195** in 15 ml Aceton und gibt bei Raumtemperatur unter Stickstoff 1 ml 4 N Salzsäure hinzu. Es wird 1 h gerührt und anschließend mit Natriumhydrogencarbonat-Lösung verdünnt. Nach Extraktion mit Ether, Trocknung über Natriumsulfat, Entfernen des Lösungsmittels und Chromatographie an Silicagel mit Essigester/Hexan fallen 178 mg der Titelverbindung **196** als farbloses Öl an.

$^1$H-NMR (300 MHz, $CDCl_3$): δ= 0,77 ppm (m, 2H); 0,90 (t, 3H); 1,18 (m, 2H); 1,64 (q, 2H); 2,03 (s, 3H)

193. 2-[1-(1-Butenyl)cyclopropyl]-2-methyl-1,3-dioxolan **197**

Man löst 7,2 g Propyltriphenylphosphoniumbromid in 100 ml Diethylether und tropft unter Stickstoff bei Raumtemperatur 7,5 ml n-Butyllithium-Lösung (2.5 M in Hexan) zu. Man rührt 1 h und gibt dann 2,34 g des Aldehydes **7** in 10 ml Diethylether zu. Es wird 1 h nachgerührt, mit Natriumchlorid-Lösung gequencht, mit Essigester extrahiert, mit Natriumchlorid-Lösung gewaschen, über Natriumsulfat getrocknet und eingeengt. Der Rückstand wird an Silicagel mit

Essigester/Hexan chromatographiert, wobei 1,8 g der Titelsubstanz **197** als farbloses Öl erhalten werden (nicht trennbares *E,Z*-Gemisch 1:3).
$^1$H-NMR (300 MHz, $CDCl_3$): *E*-Isomer: δ= 0,50 ppm (m, 2H); 0,78 (m, 2H); 0,97 (t, 3H); 1,38 (s, 3H); 2,02 (q, 2H); 3,89 (m, 4H); 5,48 (dt, 1H); 5,80 (d, 1H) Z-Isomer: δ= 0,47 ppm (m, 2H); 0,81 (m, 2H); 0,97 (t, 3H); 1,38 (s, 3H); 2,22 (q, 2H); 3,90 (m, 4H); 5,46 (dt, 1H); 5,60 (d, 1H)

194. 1-[1-(1-Butenyl)cyclopropyl)-1-ethanon **198**

Man setzt 1,1 g **197** analog 192. um und erhält 740 mg der Titelverbindung **198** als farbloses Öl (nicht trennbares *E*,*Z*-Gemisch 1:3).
$^1$H-NMR (300 MHz, $CDCl_3$): *E*-Isomer: δ= 0,98 ppm (t, 3H); 1,01 (m, 2H); 1,33 (m, 2H); 2,11 (q, 2H); 2,20 (s, 3H); 5,54 (dt, 1H); 6,01 (d, 1 H)
*Z*-Isomer: δ= 0,88 ppm (m, 2H); 0,99 (t, 3H); 1,42 (m, 2H); 2,15 (q, 2H); 2,21 (s, 3H); 5,62 (dt, 1H); 5,71 (d, 1H)

195. 1-(1-Butylcyclopropyl)-1-ethanon **199**

Man setzt 250 mg **198** analog 191 um und erhält 195 mg der Titelverbindung **199** als farbloses Öl.
$^1$H-NMR (300 MHz, $CDCl_3$): δ= 0,75 ppm (m, 2H); 0,90 (t, 3H); 1,18 (m, 2H); 1,32 (m, 2H); 1,59 (m, 2H); 2,02 (s, 3H)

196. 2-[1-(1-Hexenyl)cyclopropyl]-2-methyl-1,3-dioxolan **200**

Man setzt 2,07 g Pentyltriphenylphosphoniumbromid und 600 mg des Aldehydes **7** analog 193. um und erhält 567 mg der Titelsubstanz **200** als farbloses Öl (nicht trennbares *E*,*Z*-Gemisch 8:1).
$^1$H-NMR (300 MHz, $CDCl_3$): *E*-Isomer (Hauptdiastereomer): δ= 0,52 ppm (m, 2H); 0,78 (m, 2H); 0,90 (t, 3H); 1,40 (s, 3H); 2,00 (q, 2H); 3,90 (m, 4H); 5,42 (dt, 1H); 5,82 (d, 1H)

197. 1-(1-Hexylcyclopropyl)-2-methyl-1,3-dioxolan **201**

Man setzt 250 mg **200** analog 191. um und erhält 243 mg der Titelverbindung **201** als farbloses Öl.
$^1$H-NMR (300 MHz, $CDCl_3$): δ= 0,25 ppm (m, 2H); 0,58 (m, 2H); 0,87 (t, 3H); 1,28 (m, 8H); 1,39 (s, 3H); 1,48 (m, 2H); 3,88 (m, 4H)

198. 1-(1-Hexylcyclopropyl)-1-ethanon **202**

Man setzt 330 mg **201** analog 192. um und erhält 181 mg der Titelverbindung **202** als farbloses Öl.
$^1$H-NMR (300 MHz, $CDCl_3$): δ= 0,75 ppm (m, 2H); 0,89 (t, 3H); 1,18 (m, 2H); 1,28 (m, 8H); 1,58 (m, 2H); 2,02 (s, 3H)

199. 2-[1-(1-Heptenyl)cyclopropyl]-2-methyl-1,3-dioxolan **203**

Man setzt 1,93 g Hexyltriphenylphosphoniumbromid und 900 mg des Aldehydes **7** analog 193. um und erhält 879 mg der Titelsubstanz **203** als farbloses Öl (nicht trennbares *E*,*Z*-Gemisch 1:3).
$^1$H-NMR (300 MHz, $CDCl_3$): *E*-Isomer: δ= 0,52 ppm (m, 2H); 0,78 (m, 2H); 0,90 (t, 3H); 1,40 (6H); 1,41 (s, 3H); 2,02 (q, 2H); 3,90 (m, 4H); 5,43 (dt, 1H); 5,80 (d, 1H)
*Z*-Isomer: δ= 0,47 ppm (m, 2H); 0,82 (m, 2H); 0,90 (t, 3H); 1,40 (m, 6H); 2,20 (q, 2H); 3,91 (m, 4H); 5,47 (dt, 1H); 5,61 (d, 1H)

200. 1-(1-Heptylcyclopropyl)-2-methyl-1,3-dioxolan **204**

Man setzt 550 mg **203** analog 191. um und erhält 450 mg der Titelverbindung **204** als farbloses Öl.
$^1$H-NMR (300 MHz, $CDCl_3$): δ= 0,28 ppm (m, 2H); 0,59 (m, 2H); 0,89 (t, 3H); 1,28 (m, 10H); 1,40 (s, 3H); 1,48 (m, 2H); 3,90 (m, 4H)

201. 1-(1-Heptylcyclopropyl)-1-ethanon **205**

Man setzt 445 mg **204** analog 192. um und erhält 307 mg der Titelverbindung **205** als farbloses Öl.
$^1$H-NMR (300 MHz, $CDCl_3$): δ= 0,75 ppm (m, 2H); 0,89 (t, 3H); 1,18 (m, 2H); 1,28 (m, 10H); 1,58 (m, 2H); 2,02 (s, 3H)

202. (Z)-2-Methyl-2-[1-(1-octenyl)cyclopropyl]-1,3-dioxolan **206**

Man setzt 4,87 g Heptyltriphenylphosphoniumbromid und 1,25 g des Aldehydes **7** analog 193. um und erhält 978 mg der Titelsubstanz **206** als farbloses Öl (nur *Z*-Isomer).
$^1$H-NMR (300 MHz, $CDCl_3$): δ= 0,48 ppm (m, 2H); 0,81 (m, 2H); 0,89 (t, 3H); 1,29 (8H); 1,40 (s, 3H); 2,02 (m, 2H); 3,92 (m, 4H); 5,48 (dt, 1H); 5,62 (d, 1H)

203. 2-Methyl-1-(1-octylcyclopropyl)-1,3-dioxolan **207**

Man setzt 400 mg **206** analog 191. um und erhält 390 mg der Titelverbindung **207** als farbloses Öl.
$^1$H-NMR (300 MHz, $CDCl_3$): δ= 0,28 ppm (m, 2H); 0,59 (m, 2H); 0,90 (t, 3H); 1,28 (m, 12H); 1,39 (s, 3H); 1,48 (m, 2H); 3,90 (m, 4H)

204. 1-(1-Octylcyclopropyl)-1-ethanon **208**

Man setzt 390 mg **207** analog 192. um und erhält 250 mg der Titelverbindung **208** als farbloses Öl.
$^1$H-NMR (300 MHz, $CDCl_3$): δ= 0,77 ppm (m, 2H); 0,90 (t, 3H); 1,18 (m, 2H); 1,28 (m, 12H); 1,58 (m, 2H); 2,02 (s, 3H)

205. 1-[1-(1-Octenyl)cyclopropyl]-1-ethanon **209**

Man setzt 330 mg **206** analog 192. um und erhält 270 mg der Titelverbindung **209** als farbloses Öl.
$^1$H-NMR (300 MHz, $CDCl_3$): δ= 0,90 ppm (m, 2H); 0,90 (t, 3H); 1,18 (m, 2H); 1,30 (m, 8H); 1,44 (m, 2H); 2,10 (q, 2H); 2,22 (s, 3H); 5,64 (dt, 1H); 5,74 (d, 1H)

**Beispiel 32**

(5*Z*,7*E*,22*E*)-(1*S*,3*R*,24*S*)-25-Ethyl-26,27-cyclo-9,10-secocholesta-5,7,10(19),22-tetraen-1,3,24-triol **214a** und (5*Z*,7*E*,22*E*)-(1*S*,3*R*,24*R*)-25-Ethyl-26,27-cyclo-9,10-secocholesta-5,7,10(19),22-tetraen-1,3,24-triol **214b**

206. Man setzt 129 mg **196** mit 286 mg des Aldehydes **2** analog 149. um und erhält 187 mg (5*Z*,7*E*)-(1*S*,3*R*)-1,3-Bis[[dimethyl(1,1-dimethylethyl)silyl]oxy]-25-ethyl-22-hydroxy-26,27-cyclo-9,10-secocholesta-5,7,10(19)-trien-24-on **210** als farblosen Schaum, der direkt weiter umgesetzt wird.

207. Man setzt 170 mg **210** analog 150. um und erhält 151 mg (5*Z*,7*E*)-(1*S*,3*R*)-22-Acetoxy-1,3-bis[[dimethyl(1,1-dimethylethyl)silyl]oxy]-25-ethyl-26,27-cyclo-9,10-secocholesta-5,7,10(19)-trien-24-on **211** als farblosen Schaum, der direkt weiter umgesetzt wird.

208. Man setzt 151 mg **211** analog 151. um und erhält 110 mg (5*Z*,7*E*,22*E*)-(1 *S*,3*R*)-1,3-Bis[[dimethyl(1,1-dimethylethyl)silyl]oxy]-25-ethyl-26,27-cyclo-9,10-secocholesta-5,7,10(19),22-tetraen-24-on **212** als farblosen Schaum.
$^1$H-NMR (300 MHz, $CD_2Cl_2$): δ= 0,04 ppm (s, 12H); 0,54 (s, 3H); 0,73 (m, 2H); 0,87 (s, 18H); 0,92 (m, 2H); 0,93 (t, 3H); 1,05 (d, 3H); 4,17 (m, 1H); 4,37 (m, 1H); 4,83 (s, 1H); 5,17 (s, 1H); 6,01 (d, 1H); 6,11 (d, 1H); 6,23 (d, 1H); 6,70 (dd, 1H)

209. Man setzt 110 mg **212** analog 152. um und erhält nach Chromatographie an Silicagel mit Essigester/Hexan nacheinander 42 mg (5*Z*,7*E*,22*E*)-(1*S*,3*R*,24*S*)-1,3-Bis[[dimethyl(1,1 -dimethylethyl)silyl]oxy]-25-ethyl-26,27-cyclo-9,10-secocholesta-5,7,10(19),22-tetraen-24-ol **213a** und 25 mg (5*Z*,7*E*,22*E*)-(1*S*,3*R*,24*R*)-1,3-Bis[[dimethyl(1,1-dimethylethyl)silyl]oxy]-25-ethyl-26,27-cyclo-9,10-secocholesta-5,7,10(19),22-tetraen-24-ol **213b** als farblose Schäume.
$^1$H-NMR (300 MHz, $CD_2Cl_2$): **213a**: δ= 0,03 ppm (s, 12H); 0,25 (m, 2H); 0,42 (m, 2H); 0,53 (s, 3H); 0,85 (t, 3H); 0,86 (s, 18H); 1,02 (d, 3H); 3,80 (m, 1H); 4,16 (m, 1H); 4,36 (m, 1H); 4,83 (s, 1H); 5,16 (s, 1H); 5,33 (dd, 1H); 5,50 (dd, 1H); 6,01 (d, 1H); 6,23 (d, 1H)
**213b:** δ= 0,03 ppm (s, 12H); 0,25 (m, 2H); 0,43 (m, 2H); 0,53 (s, 3H); 0,85 (t, 3H); 0,85 (s, 18H); 1,03 (d, 3H); 3,78 (m, 1H); 4,17 (m, 1H); 4,36 (m, 1H); 4,83 (s, 1H); 5,16 (s, 1H); 5,33 (dd, 1H); 5,45 (dd, 1H); 6,01 (d, 1H); 6,23 (d, 1H)

210. Man setzt 42 mg **213a** analog 154. um und erhält 18 mg der Titelverbindung **214a** als farblosen Schaum.
$^1$H-NMR (300 MHz, $CD_2Cl_2$) δ= 0,29 ppm (m, 2H); 0,42 (m, 2H); 0,57 (s, 3H); 0,88 (t, 3H); 1,03 (d, 3H); 3,81 (d, 1H); 4,17 (m, 1H); 4,38 (m, 1H); 4,95 (s, 1H); 5,29 (s, 1H); 5,35 (dd, 1H); 5,51 (dd, 1H); 6,00 (d, 1H); 6,35 (d, 1H)

211. Man setzt 25 mg **213b** analog 154. um und erhält 9,5 mg der Titelverbindung **214b** als farblosen Schaum.
$^1$H-NMR (300 MHz, $CD_2Cl_2$) δ= 0,27 ppm (m, 2H); 0,44 (m, 2H); 0,57 (s, 3H); 0,89 (t, 3H); 1,02 (d, 3H); 3,79 (d, 1H); 4,17 (m, 1H); 4,38 (m, 1H); 4,96 (s, 1H); 5,29 (s, 1H); 5,34 (dd, 1H); 5,48 (dd, 1H); 6,00 (d, 1H); 6,36 (d, 1H)

### Beispiel 33

[5*Z*,7*E*,22*E*,25(*Z*)]-(1*S*,3*R*,24*S*)-25-(1-Butenyl)-26,27-cyclo-9,10-secocholesta-5,7,10(19),22-tetraen-1,3,24-triol **220b**, [5*Z*,7*E*,22*E*,25(*E*)]-(1*S*,3*R*,24*S*)-25-(1-Butenyl)-26,27-cyclo-9,10-secocholesta-5,7,10(19), 22-tetraen-1,3,24-triol **221a** und [5*Z*,7*E*,22*E*,25(*E*)]-(1*S*,3*R*,24*R*)-25-(1-Butenyl)-26,27-cyclo-9,10-secocholesta-5,7,10(19), 22-tetraen-1,3,24-triol **221b**

212. Man setzt 460 mg **198** mit 573 mg des Aldehydes **2** analog 149. um und erhält 546 mg (5*Z*,7*E*)-(1*S*,3*R*)-1,3-Bis[[dimethyl(1,1-dimethylethyl)silyl]oxy]-25-(1-butenyl)-22-hydroxy-26,27-cyclo-9,10-secocholesta-5,7,10(19)-trien-24-on **215** als farblosen Schaum, der direkt weiter umgesetzt wird.

213. Man setzt 275 mg **215** analog 150. um und erhält 265 mg (5*Z*,7*E*)-(1*S*,3*R*)-22-Acetoxy-1,3-bis[[dimethyl (1,1-dimethylethyl)silyl]oxy]-25-(1-butenyl)-26,27-cyclo-9,10-secocholesta-5,7,10(19)-trien-24-on **216** als farblosen Schaum, der direkt weiter umgesetzt wird.

214. Man setzt 265 mg **216** analog 151. um und erhält 214 mg (5*Z*,7*E*,22*E*)-(1*S*,3*R*)-1,3-Bis[[dimethyl(1,1-dimethylethyl)silyl]oxy]-25-(1-butenyl)-26,27-cyclo-9,10-secocholesta-5,7,10(19),22-tetraen-24-on **217** als farblosen Schaum (*E*:*Z*-Gemisch 1:3).

215. Man setzt 214 mg **217** analog 152. um und erhält nach Chromatographie an Silicagel mit Essigester/ Hexan nacheinander 31 mg [5*Z*,7*E*,22*E*,25(*Z*)]-(1*S*,3*R*,24*R*)-1,3-Bis[[dimethyl(1,1-dimethylethyl)silyl]oxy]-25-(1-butenyl)-26,27-cyclo-9,10-secocholesta-5,7,10(19),22-tetraen-24-ol **218b,** 27 mg [5*Z*,7*E*,22*E*,25(*E*)]-(1*S*,3*R*,24*S*)-1,3-Bis[[dimethyl(1,1-dimethylethyl)silyl]oxy]-25-(1-butenyl)-26,27-cyclo-9,10-secocholesta-5,7,10(19),22-tetraen-24-ol **219a** und 22 mg [5*Z*,7*E*,22*E*,25(*E*)]-(1S,3R,24R)-1,3-Bis[[dimethyl(1,1-dimethylethyl)silyl]oxy]-25-(1 -butenyl)-26,27-cyclo-9,10-secocholesta-5,7,10(19),22-tetraen-24-ol **219b** als farblose Schäume.
$^1$H-NMR (300 MHz, $CD_2Cl_2$): **218b:** δ= 0,03 ppm (s, 12H); 0,53 (s, 3H); 0,86 (s, 18H); 0,93 (t, 3H); 1,01 (d, 3H); 3,49 (d, 1H); 4,17 (m, 1H); 4,37 (m, 1H); 4,84 (s, 1H); 5,17 (s, 1H); 5,42 (m, 2H); 5,49 (m, 2H); 6,01 (d, 1H); 6,23 (d, 1H)
**219a**: δ= 0,03 ppm (s, 12H); 0,53 (s, 3H); 0,87 (s, 18H); 0,94 (t, 3H); 1,03 (d, 3H); 3,60 (d, 1H); 4,17 (m, 1H); 4,37 (m, 1H); 4,83 (s, 1H); 5,17 (s, 1H); 5,40 (dt, 1H); 5,46 (m, 2H); 5,59 (d, 1H); 6,01 (d, 1H); 6,23 (d, 1H)
**219b**: δ= 0,04 ppm (s, 12H); 0,53 (s, 3H); 0,87 (s, 18H); 0,95 (t, 3H); 1,01 (d, 3H); 3,48 (d, 1H); 4,17 (m, 1H); 4,37 (m, 1H); 4,84 (s, 1H); 5,18 (s, 1H); 5,41 (m, 2H); 5,50 (m, 2H); 6,01 (d, 1H); 6,24 (d, 1H)

216. Man setzt 17 mg **218b** analog 154. um und erhält 7 mg der Titelverbindung **220b** als farblosen Schaum.
$^1$H-NMR (300 MHz, $CD_2Cl_2$) δ= 0,42 ppm (m, 2H); 0,53 (s, 3H); 0,56 (m, 2H); 0,94 (t, 3H); 1,02 (d, 3H); 3,49 (m, 1H); 4,16 (m, 1H); 4,36 (m, 1H); 4,93 (s, 1H); 5,29 (s, 1H); 5,41 (m, 2H); 5,45 (m, 1H); 6,00 (d, 1H); 6,33 (d, 1H)

217. Man setzt 27 mg **219a** analog 154. um und erhält 11 mg der Titelverbindung **221a** als farblosen Schaum.
$^1$H-NMR (300 MHz, $CD_2Cl_2$) δ= 0,44 ppm (m, 2H); 0,54 (s, 3H); 0,57 (m, 2H); 0,97 (t, 3H); 1,03 (d, 3H); 3,59 (d, 1H); 4,17 (m, 1H); 4,38 (m, 1H); 4,95 (s, 1H); 5,29 (s, 1H); 5,45 (m, 3H); 5,58 (d, 1H); 6,00 (d, 1H); 6,36 (d, 1H)

218. Man setzt 22 mg **219b** analog 154. um und erhält 8 mg der Titelverbindung **221b** als farblosen Schaum.
$^1$H-NMR (300 MHz, $CD_2Cl_2$) δ= 0,45 ppm (m, 2H); 0,55 (s, 3H); 0,58 (m, 2H); 0,97 (t, 3H); 1,02 (d, 3H); 3,49 (d, 1H); 4,18 (m, 1H); 4,38 (m, 1H); 4,96 (s, 1H); 5,29 (s, 1H); 5,42 (m, 2H); 5,50 (m, 2H); 6,01 (d, 1H); 6,36 (d, 1H)

### Beispiel 34

(5*Z*,7*E*,22*E*)-(1*S*,3*R*,24*S*)-25-Butyl-26,27-cyclo-9,10-secocholesta-5,7,10(19),22-tetraen-1,3,24-triol **226a** und (5*Z*, 7*E*,22*E*)-(1*S*,3*R*,24*R*)-25-Butyl-26,27-cyclo-9,10-secocholesta-5,7,10(19),22-tetraen-1,3,24-triol **226b**

219. Man setzt 195 mg **199** mit 400 mg des Aldehydes **2** analog 149. um und erhält 275 mg (5*Z*,7*E*)-(1*S*,3*R*)-1,3-Bis[[dimethyl(1,1-dimethylethyl)silyl]oxy]-25-butyl-22-hydroxy-26,27-cyclo-9,10-secocholesta-5,7,10(19)-trien-24-on **222** als farblosen Schaum, der direkt weiter umgesetzt wird.

220. Man setzt 275 mg **222** analog 150. um und erhält 245 mg (5*Z*,7*E*)-(1*S*,3*R*)-22-Acetoxy-1,3-bis[[dimethyl (1,1-dimethylethyl)silyl]oxy]-25-butyl-26,27-cyclo-9,10-secocholesta-5,7,10(19)-trien-24-on **223** als farblosen Schaum, der direkt weiter umgesetzt wird.

221. Man setzt 245 mg **223** analog 151. um und erhält 214 mg (5*Z*,7*E*,22*E*)-(1 *S*, 3*R*)-1,3-Bis[[dimethyl(1,1-dimethylethyl)silyl]oxy]-25-butyl-26,27-cyclo-9,10-secocholesta-5,7,10(19),22-tetraen-24-on **224** als farblosen Schaum.
$^1$H-NMR (300 MHz, $CD_2Cl_2$): δ= 0,04 ppm (s, 12H); 0,54 (s, 3H); 0,74 (m, 2H); 0,87 (s, 18H); 0,88 (t, 3H); 0,92 (m, 2H); 1,06 (d, 3H); 4,17 (m, 1H); 4,36 (m, 1H); 4,83 (s, 1H); 5,15 (s, 1H); 6,01 (d, 1H); 6,14 (d, 1H); 6,23 (d, 1H); 6,66 (dd, 1H)

222. Man setzt 214 mg **224** analog 152. um und erhält nach Chromatographie an Silicagel mit Essigester/ Hexan nacheinander 80 mg (5*Z*,7*E*,22*E*)-(1*S*,3*R*,24*S*)-1,3-Bis[[dimethyl(1,1-dimethylethyl)silyl]oxy]-25-butyl-26,27-cyclo-9,10-secocholesta-5,7,10(19),22-tetraen-24-ol **225a** und 41 mg (5*Z*,7*E*,22*E*)-(1*S*,3*R*,24*R*)-1,3-Bis[[dimethyl(1,1-dimethylethyl)silyl]oxy]-25-butyl-26,27-cyclo-9,10-secocholesta-5,7,10(19),22-tetraen-24-ol **225b** als farblose

Schäume.
$^1$H-NMR (300 MHz, $CD_2Cl_2$): **225a:** δ= 0,03 ppm (s, 12H); 0,25 (m, 2H); 0,43 (m, 2H); 0,53 (s, 3H); 0,86 (t, 3H); 0,86 (s, 18H); 1,03 (d, 3H); 3,79 (m, 1H); 4,17 (m, 1H); 4,36 (m, 1H); 4,83 (s, 1H); 5,16 (s, 1H); 5,35 (dd, 1H); 5,50 (dd, 1H); 6,01 (d, 1H); 6,23 (d, 1H)
**225b:** δ= 0,03 ppm (s, 12H); 0,24 (m, 2H); 0,43 (m, 2H); 0,53 (s, 3H); 0,86 (t, 3H); 0,87 (s, 18H); 1,03 (d, 3H); 3,77 (m, 1H); 4,17 (m, 1H); 4,36 (m, 1H); 4,83 (s, 1H); 5,16 (s, 1H); 5,33 (dd, 1H); 5,45 (dd, 1H); 6,01 (d, 1H); 6,23 (d, 1H)
223. Man setzt 80 mg **225a** analog 154. um und erhält 37 mg der Titelverbindung **226a** als farblosen Schaum.
$^1$H-NMR (300 MHz, $CD_2Cl_2$) δ= 0.28 ppm (m, 2H); 0,44 (m, 2H); 0,58 (s, 3H); 0,89 (t, 3H); 1,03 (d, 3H); 3,79 (d, 1H); 4,18 (m, 1H); 4,38 (m, 1H); 4,96 (s, 1H); 5,29 (s, 1H); 5,37 (dd, 1H); 5,51 (dd, 1H); 6,00 (d, 1H); 6,36 (d, 1H)
224. Man setzt 41 mg **225b** analog 154. um und erhält 17 mg der Titelverbindung **226b** als farblosen Schaum.
$^1$H-NMR (300 MHz, $CD_2Cl_2$) δ= 0,26 ppm (m, 2H); 0,44 (m, 2H); 0,58 (s, 3H); 0,88 (t, 3H); 1,02 (d, 3H); 3,78 (d, 1H); 4,18 (m, 1H); 4,38 (m, 1H); 4,97 (s, 1H); 5,29 (s, 1H); 5,34 (dd, 1H); 5,48 (dd, 1H); 6,00 (d, 1H); 6,36 (d, 1H)

**Beispiel 35**

(5*Z*,7*E*,22*E*)-(1*S*,3*R*,24*S*)-25-Hexyl-26,27-cyclo-9,10-secocholesta-5,7,10(19),22-tetraen-1,3,24-triol **231a** und (5*Z*,7*E*,22*E*)-(1*S*,3*R*,24*R*)-25-Hexyl-26,27-cyclo-9,10-secocholesta-5,7,10(19),22-tetraen-1,3,24-triol **231b**

225. Man setzt 120 mg **202** mit 287 mg des Aldehydes **2** analog 149. um und erhält 256 mg (5*Z*,7*E*)-(1S,3R)-1,3-Bis[[dimethyl(1,1-dimethylethyl)silyl]oxy]-25-hexyl-22-hydroxy-26,27-cyclo-9,10-secocholesta-5,7,10(19)-trien-24-on **227** als farblosen Schaum, der direkt weiter umgesetzt wird.
226. Man setzt 256 mg **227** analog 150. um und erhält 234 mg (5*Z*,7*E*)-(1*S*,3*R*)-22-Acetoxy-1,3-bis[[dimethyl (1,1-dimethylethyl)silyl]oxy]-25-hexyl-26,27-cyclo-9,10-secocholesta-5,7,10(19)-trien-24-on **228** als farblosen Schaum, der direkt weiter umgesetzt wird.
227. Man setzt 234 mg **228** analog 151. um und erhält 104 mg (5*Z*,7*E*,22*E*)-(1 *S*,3*R*)-1,3-Bis[[dimethyl(1,1-dimethylethyl)silyl]oxy]-25-hexyl-26,27-cyclo-9,10-secocholesta-5,7,10(19),22-tetraen-24-on **229** als farblosen Schaum.
$^1$H-NMR (300 MHz, $CD_2Cl_2$): δ= 0,05 ppm (s, 12H); 0,56 (s, 3H); 0,74 (m, 2H); 0,88 (s, 18H); 0,89 (t, 3H); 0,93 (m, 2H); 1,08 (d, 3H); 4,18 (m, 1H); 4,38 (m, 1H); 4,85 (s, 1H); 5,17 (s, 1H); 6,01 (d, 1H); 6,15 (d, 1H); 6,23 (d, 1H); 6,73 (dd, 1H)
228. Man setzt 104 mg **229** analog 1523. um und erhält nach Chromatographie an Silicagel mit Essigester/Hexan nacheinander 37 mg (5*Z*,7*E*,22*E*)-(1*S*,3*R*,24*S*)-1,3-Bis[[dimethyl(1,1-dimethylethyl)silyl]oxy]-25-hexyl-26,27-cyclo-9,10-secocholesta-5,7,10(19),22-tetraen-24-ol **230a** und 35 mg (5*Z*,7*E*,22*E*)-(1*S*,3*R*,24*R*)-1,3-Bis[[dimethyl(1,1-dimethylethyl)silyl]oxy]-25-hexyl-26,27-cyclo-9,10-secocholesta-5,7,10(19),22-tetraen-24-ol **230b** als farblose Schäume.
$^1$H-NMR (300 MHz, $CD_2Cl_2$): **230a:** δ= 0,04 ppm (s, 12H); 0,24 (m, 2H); 0,43 (m, 2H); 0,54 (s, 3H); 0,87 (t, 3H); 0,87 (s, 18H); 1,02 (d, 3H); 3,77 (m, 1H); 4,17 (m, 1H); 4,36 (m, 1H); 4,84 (s, 1H); 5,16 (s, 1H); 5,34 (dd, 1H); 5,50 (dd, 1H); 6,01 (d, 1H); 6,24 (d, 1H)
**230b**: δ= 0,04 ppm (s, 12H); 0,23 (m, 2H); 0,43 (m, 2H); 0,54 (s, 3H); 0,87 (t, 3H); 0,87 (s, 18H); 1,03 (d, 3H); 3,75 (d, 1H); 4,17 (m, 1H); 4,37 (m, 1H); 4,84 (s, 1H); 5,16 (s, 1H); 5,33 (dd, 1H); 5,46 (dd, 1H); 6,01 (d, 1H); 6,24 (d, 1H)
229. Man setzt 37 mg **230a** analog 154. um und erhält 17 mg der Titelverbindung **231a** als farblosen Schaum.
$^1$H-NMR (300 MHz, $CD_2Cl_2$) δ= 0,28 ppm (m, 2H); 0,43 (m, 2H); 0,58 (s, 3H); 0,90 (t, 3H); 1,04 (d, 3H); 3,80 (d, 1H); 4,18 (m, 1H); 4,38 (m, 1H); 4,96 (s, 1H); 5,28 (s, 1H); 5,35 (dd, 1H); 5,51 (dd, 1H); 6,01 (d, 1H); 6,36 (d, 1H)
230. Man setzt 31 mg **230b** analog 154. um und erhält 10 mg der Titelverbindung **231b** als farblosen Schaum.
$^1$H-NMR (300 MHz, $CD_2Cl_2$) δ= 0,28 ppm (m, 2H); 0,46 (m, 2H); 0,59 (s, 3H); 0,89 (t, 3H); 1,04 (d, 3H); 3,79 (d, 1H); 4,18 (m, 1H); 4,38 (m, 1H); 4,97 (s, 1H); 5,29 (s, 1H); 5,35 (dd, 1H); 5,48 (dd, 1H); 6,00 (d, 1H); 6,37 (d, 1H)

**Beispiel 36**

(5*Z*,7*E*,22*E*)-(1*S*,3*R*,24*S*)-25-Heptyl-26,27-cyclo-9,10-secocholesta-5,7,10(19),22-tetraen-1,3,24-triol **236a** und (5*Z*,7*E*,22*E*)-(1*S*,3*R*,24*R*)-25-Heptyl-26,27-cyclo-9,10-secocholesta-5,7,10(19),22-tetraen-1,3,24-triol **236b**

231. Man setzt 300 mg **205** mit 573 mg des Aldehydes **2** analog 149. um und erhält 556 mg (5*Z*,7*E*)-(1*S*,3*R*)-1,3-Bis[[dimethyl(1,1-dimethylethyl)silyl]oxy]-25-heptyl-22-hydroxy-26,27-cyclo-9,10-secocholesta-5,7,10(19)-trien-24-on **232** als farblosen Schaum, der direkt weiter umgesetzt wird.
232. Man setzt 305 mg **232** analog 150. um und erhält 278 mg (5*Z*,7*E*)-(1*S*,3*R*)-22-Acetoxy-1,3-bis[[dimethyl (1,1 -dimethylethyl)silyl]oxy]-25-heptyl-26,27-cyclo-9,10-secocholesta-5,7,10(19)-trien-24-on **233** als farblosen Schaum, der direkt weiter umgesetzt wird.

233. Man setzt 278 mg **233** analog 151. um und erhält 203 mg (5*Z*,7*E*,22*E*)-(1 *S*,3*R*)-1,3-Bis[[dimethyl(1,1-dimethylethyl)silyl]oxy]-25-heptyl-26,27-cyclo-9,10-secocholesta-5,7,10(19),22-tetraen-24-on **234** als farblosen Schaum.
$^1$H-NMR (300 MHz, $CD_2Cl_2$): δ= 0,05 ppm (s, 12H); 0,57 (s, 3H); 0,74 (m, 2H); 0,89 (s, 18H); 0,89 (t, 3H); 0,94 (m, 2H); 1,09 (d, 3H); 4,18 (m, 1H); 4,38 (m, 1H); 4,87 (s, 1H); 5,18 (s, 1H); 6,01 (d, 1H); 6,14 (d, 1H); 6,22 (d, 1H); 6,75 (dd, 1H)

234. Man setzt 200 mg **234** analog 152. um und erhält nach Chromatographie an Silicagel mit Essigester/Hexan nacheinander 80 mg (5*Z*,7*E*,22*E*)-(1*S*,3*R*,24*S*)-1,3-Bis[[dimethyl(1,1-dimethylethyl)silyl]oxy]-25-heptyl-26,27-cyclo-9,10-secocholesta-5,7,10(19),22-tetraen-24-ol **235a** und 38 mg (5*Z*,7*E*,22*E*)-(1*S*,3*R*,24*R*)-1,3-Bis[[dimethyl(1,1 -dimethylethyl)silyl]oxy]-25-heptyl-26,27-cyclo-9,10-secocholesta-5,7,10(19),22-tetraen-24-ol **235b** als farblose Schäume.
$^1$H-NMR (300 MHz, $CD_2Cl_2$): **235a**: δ= 0,04 ppm (s, 12H); 0,24 (m, 2H); 0,43 (m, 2H); 0,53 (s, 3H); 0,86 (t, 3H); 0,86 (s, 18H); 1,02 (d, 3H); 3,75 (m, 1H); 4,17 (m, 1H); 4,36 (m, 1H); 4,84 (s, 1H); 5,16 (s, 1H); 5,33 (dd, 1H); 5,50 (dd, 1H); 6,00 (d, 1H); 6,23 (d, 1H)
**235b**: δ= 0.04 ppm (s, 12H); 0,24 (m, 2H); 0,43 (m, 2H); 0,53 (s, 3H); 0,87 (t, 3H); 0,87 (s, 18H); 1,03 (d, 3H); 3,76 (d, 1H); 4,17 (m, 1H); 4,36 (m, 1H); 4,83 (s, 1H); 5,15 (s, 1H); 5,33 (dd, 1H); 5,45 (dd, 1H); 6,00 (d, 1H); 6,23 (d, 1H)
235. Man setzt 75 mg **235a** analog 154. um und erhält 51 mg der Titelverbindung **236a** als farblosen Schaum.
$^1$H-NMR (300 MHz, $CD_2Cl_2$) δ= 0,26 ppm (m, 2H); 0,43 (m, 2H); 0,56 (s, 3H); 0,88 (t, 3H); 1,02 (d, 3H); 3,78 (d, 1H); 4,17 (m, 1H); 4,37 (m, 1H); 4,95 (s, 1H); 5,28 (s, 1H); 5,34 (dd, 1H); 5,50 (dd, 1H); 6,00 (d, 1H); 6,35 (d, 1H)

236. Man setzt 33 mg **235b** analog 154. um und erhält 17 mg der Titelverbindung **236b** als farblosen Schaum.
$^1$H-NMR (300 MHz, $CD_2Cl_2$) δ= 0,26 ppm (m, 2H); 0,44 (m, 2H); 0,56 (s, 3H); 0,87 (t, 3H); 1,03 (d, 3H); 3,76 (d, 1H); 4,16 (m, 1H); 4,36 (m, 1H); 4,95 (s, 1H); 5,28 (s, 1H); 5,33 (dd, 1H); 5,45 (dd, 1H); 6,00 (d, 1H); 6,35 (d, 1H)

**Beispiel 37**

(5*Z*,7*E*,22*E*)-(1*S*,3*R*,24*S*)-25-Octyl-26,27-cyclo-9,10-secocholesta-5,7,10(19),22-tetraen-1,3,24-triol **241a** und (5*Z*,7*E*,22*E*)-(1*S*,3*R*,24*R*)-25-Octyl-26,27-cyclo-9,10-secocholesta-5,7,10(19),22-tetraen-1,3,24-triol **241 b**

237. Man setzt 250 mg **208** mit 500 mg des Aldehydes **2** analog 149. um und erhält 432 mg (5*Z*,7*E*)-(1*S*,3*R*)-1,3-Bis[[dimethyl(1,1-dimethylethyl)silyl]oxy]-22-hydroxy-25-octyl-26,27-cyclo-9,10-secocholesta-5,7,10(19)-trien-24-on **237** als farblosen Schaum, der direkt weiter umgesetzt wird.

238. Man setzt 380 mg **237** analog 150. um und erhält 356 mg (5*Z*,7*E*)-(1*S*,3*R*)-22-Acetoxy-1,3-bis[[dimethyl(1,1-dimethylethyl)silyl]oxy]-25-octyl-26,27-cyclo-9,10-secocholesta-5,7,10(19)-trien-24-on **238** als farblosen Schaum, der direkt weiter umgesetzt wird.

239. Man setzt 356 mg **238** analog 151. um und erhält 310 mg (5*Z*,7*E*,22*E*)-(1*S*,3*R*)-1,3-Bis[[dimethyl(1,1-dimethylethyl)silyl]oxy]-25-octyl-26,27-cyclo-9,10-secocholesta-5,7,10(19),22-tetraen-24-on **239** als farblosen Schaum.
$^1$H-NMR (300 MHz, $CD_2Cl_2$): δ= 0,04 ppm (s, 12H); 0,57 (s, 3H); 0,74 (m, 2H); 0,88 (s, 18H); 0,89 (t, 3H); 0,93 (m, 2H); 1,09 (d, 3H); 4,18 (m, 1H); 4,38 (m, 1H); 4,87 (s, 1H); 5,18 (s, 1H); 6,01 (d, 1H); 6,14 (d, 1H); 6,23 (d, 1H); 6,76 (dd, 1H)
240. Man setzt 310 mg **239** analog 152. um und erhält nach Chromatographie an Silicagel mit Essigester/Hexan nacheinander 130 mg (5*Z*,7*E*,22*E*)-(1*S*,3*R*,24*S*)-1,3-Bis[[dimethyl(1,1-dimethylethyl)silyl]oxy]-25-octyl-26,27-cyclo-9,10-secocholesta-5,7,10(19),22-tetraen-24-ol **240a** und 53 mg (5*Z*,7*E*,22*E*)-(1 *S*,3*R*,24*R*)-1,3-Bis[[dimethyl(1,1-dimethylethyl)silyl]oxy]-25-octyl-26,27-cyclo-9,10-secocholesta-5,7,10(19),22-tetraen-24-ol 240b als farblose Schäume.
$^1$H-NMR (300 MHz, $CD_2Cl_2$): **240a**: δ= 0,04 ppm (s, 12H); 0,24 (m, 2H); 0,43 (m, 2H); 0,55 (s, 3H); 0,88 (t, 3H); 0,88 (s, 18H); 1,04 (d, 3H); 3,79 (m, 1H); 4,18 (m, 1H); 4,38 (m, 1H); 4,86 (s, 1H); 5,18 (s, 1H); 5,36 (dd, 1H); 5,51 (dd, 1H); 6,01 (d, 1H); 6,25 (d, 1H)
**240b:** δ= 0,04 ppm (s, 12H); 0,24 (m, 2H); 0,43 (m, 2H); 0,56 (s, 3H); 0,88 (t, 3H); 0,89 (s, 18H); 1,05 (d, 3H); 3,78 (d, 1H); 4,18 (m, 1H); 4,38 (m, 1H); 4,84 (s, 1H); 5,18 (s, 1H); 5,35 (dd, 1H); 5,48 (dd, 1H); 6,01 (d, 1H); 6,25 (d, 1H)

241. Man setzt 130 mg **240a** analog 154. um und erhält 67 mg der Titelverbindung **241a** als farblosen Schaum.
$^1$H-NMR (300 MHz, $CD_2Cl_2$) δ= 0,28 ppm (m, 2H); 0,45 (m, 2H); 0,57 (s, 3H); 0,89 (t, 3H); 1,04 (d, 3H); 3,79 (d, 1H); 4,18 (m, 1H); 4,38 (m, 1H); 4,97 (s, 1H); 5,29 (s, 1H); 5,36 (dd, 1H); 5,51 (dd, 1H); 6,01 (d, 1H); 6,36 (d, 1H)

242. Man setzt 53 mg **240b** analog 154. um und erhält 16 mg der Titelverbindung **241b** als farblosen Schaum.
$^1$H-NMR (300 MHz, $CD_2Cl_2$) δ= 0,28 ppm (m, 2H); 0,47 (m, 2H); 0,57 (s, 3H); 0,89 (t, 3H); 1,05 (d, 3H); 3,79 (d, 1H); 4,17 (m, 1H); 4,38 (m, 1H); 4,96 (s, 1H); 5,29 (s, 1H); 5,35 (dd, 1H); 5,48 (dd, 1H); 6,01 (d, 1H); 6,37 (d, 1H)

**Beispiel 38**

[5*Z*,7*E*,22*E*,25(*Z*)]-(1*S*,3*R*,24*S*)-25-(1-Octenyl)-26,27-cyclo-9,10-secocholesta-5,7,10(19),22-tetraen-1,3,24-triol **246a** und [5*Z*,7*E*,22*E*,25(*Z*)]-(1*S*,3*R*,24*S*)-25-(1-Octenyl)-26,27-cyclo-9,10-secocholesta-5,7,10(19),22-tetraen-1,3,24-triol **246b**

243. Man setzt 250 mg **209** mit 573 mg des Aldehydes **2** analog 149. um und erhält 550 mg [5*Z*,7*E*,25(*Z*)]-(1*S*,3*R*)-1,3-Bis[[dimethyl(1,1-dimethylethyl)silyl]oxy]-22-hydroxy-25-(1-octenyl)-26,27-cyclo-9,10-secocholesta-5,7,10(19)-trien-24-on **242** als farblosen Schaum, der direkt weiter umgesetzt wird.

244. Man setzt 540 mg **242** analog 150. um und erhält 476 mg [5*Z*,7*E*,25(*Z*)]-(1 *S*,3*R*)-22-Acetoxy-1,3-bis[[dimethyl(1,1-dimethylethyl)silyl]oxy]-25-(1-octenyl)-26,27-cyclo-9,10-secocholesta-5,7,10(19)-trien-24-on **243** als farblosen Schaum, der direkt weiter umgesetzt wird.

245. Man setzt 476 mg **243** analog 151. um und erhält 323 mg [5*Z*,7*E*,22*E*,25(*Z*)]-(1 *S*,3*R*)-1,3-Bis[[dimethyl(1,1-dimethylethyl)silyl]oxy]-25-(1-octenyl)-26,27-cyclo-9,10-secocholesta-5,7,10(19),22-tetraen-24-on **244** als farblosen Schaum.
$^1$H-NMR (300 MHz, $CD_2Cl_2$): $\delta$= 0,06 ppm (s, 12H); 0,57 (s, 3H); 0,74 (m, 2H); 0,89 (s, 18H); 0,89 (t, 3H); 0,93 (m, 2H); 1,09 (d, 3H); 4,18 (m, 1H); 4,38 (m, 1H); 4,88 (s, 1H); 5,19 (s, 1H); 6,01 (d, 1H); 6,23 (d, 1H); 6,54 (d, 1H); 6,72 (dd, 1H)

246. Man setzt 320 mg **244** analog 152. um und erhält nach Chromatographie an Silicagel mit Essigester/Hexan nacheinander 128 mg **245a** und 68 mg [5*Z*,7*E*,22*E*,25(*E*)]-(1*S*,3*R*,24*S*)-1,3-Bis[[dimethyl(1,1-dimethylethyl)silyl]oxy]-25-(1-octenyl)-26,27-cyclo-9,10-secocholesta-5,7,10(19),22-tetraen-24-ol **245b** als farblose Schäume.
$^1$H-NMR (300 MHz, $CD_2Cl_2$): **245a:** $\delta$= 0,03 ppm (s, 12H); 0,24 (m, 2H); 0,43 (m, 2H); 0,54 (s, 3H); 0,86 (t, 3H); 0,87 (s, 18H); 1,02 (d, 3H); 3,50 (m, 1H); 4,18 (m, 1H); 4,37 (m, 1H); 4,85 (s, 1H); 5,16 (s, 1H); 5,42 (m, 2H); 5,50 (m, 2H); 6,00 (d, 1H); 6,23 (d, 1H)
**245b:** $\delta$= 0,03 ppm (s, 12H); 0,24 (m, 2H); 0,43 (m, 2H); 0,54 (s, 3H); 0,87 (t, 3H); 0,87 (s, 18H); 1,03 (d, 3H); 3,48 (d, 1H); 4,17 (m, 1H); 4,37 (m, 1H); 4,83 (s, 1H); 5,17 (s, 1H); 5,39 (m, 2H); 5,51 (m, 2H); 6,01 (d, 1H); 6,24 (d, 1H)

247. Man setzt 48 mg **245a** analog 154. um und erhält 26 mg der Titelverbindung **246a** als farblosen Schaum.
$^1$H-NMR (300 MHz, $CD_2Cl_2$) $\delta$= 0,55 ppm (m, 2H); 0,58 (s, 3H); 0,69 (m, 2H); 0,90 (t, 3H); 1,04 (d, 3H); 3,53 (d, 1H); 4,18 (m, 1H); 4,38 (m, 1H); 4,98 (s, 1H); 5,29 (s, 1H); 5,47 (m, 2H); 5,52 (m, 2H); 6,01 (d, 1H); 6,37 (d, 1H)

248. Man setzt 40 mg **245b** analog 154. um und erhält 16 mg der Titelverbindung **246b** als farblosen Schaum.
$^1$H-NMR (300 MHz, $CD_2Cl_2$) $\delta$= 0,54 ppm (m, 2H); 0,59 (s, 3H); 0,68 (s, 3H); 0,89 (t, 3H); 1,04 (d, 3H); 3,50 (d, 1H); 4,17 (m, 1H); 4,38 (m, 1H); 4,97 (s, 1H); 5,29 (s, 1H); 5,40 (m, 2H); 5,51 (m, 2H); 6,01 (d, 1H); 6,38 (d, 1H)

**Patentansprüche**

1. Vitamin D-Derivate der allgemeinen Formel **I**,

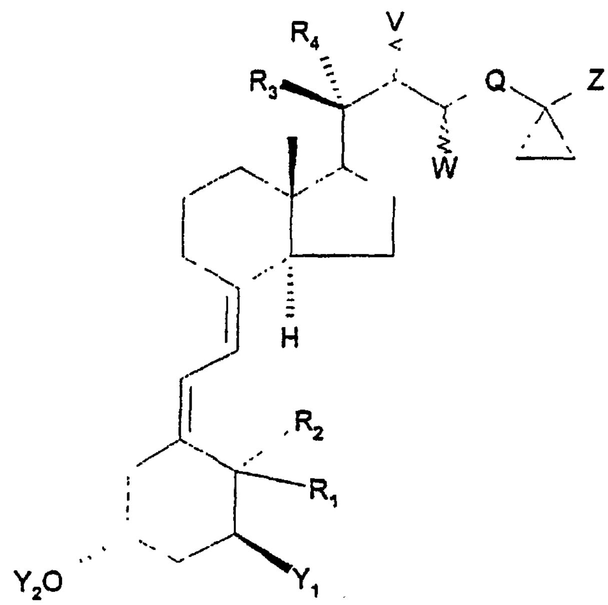

worin

$Y_1$ ein Wasserstoffatom, eine Hydroxylgruppe, ein Fluor-, Chlor- oder Bromatom oder eine Gruppe $-OCOR_8$, worin

$R_8$ ein aliphatischer oder aromatischer Rest mit 1 bis 12 C-Atomen ist,

$Y_2$ ein Wasserstoffatom oder eine Gruppe $-(CO)R_9$, worin

$R_9$ ein aliphatischer oder aromatischer Rest mit 1 bis 12 C-Atomen ist,

$R_1$ und $R_2$ je ein Wasserstoffatom oder gemeinsam eine exocyclische Methylengruppe,

$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,

V und W zusammen eine E-Doppelbindung oder V eine Hydroxylgruppe und W ein Wasserstoffatom,

Q eine Gruppe ausgewählt aus

- $CH_2$-, $-(CH_2)_2$-, $-(CH_2)_3$-, $-(CH_2)_4$-, $-(CH_2)_7$-, $-CH_2-C(CH_3)_2-CH_2$-,
- $CH_2-CH(CH_3)-CH_2-CH(CH_3)-CH_2$-,
- $CH_2$-CH(OH)-, $-CH_2-CH_2$-CH(OH)-, -CH(OH)-$CH_2$-,
- CH(OH)-$CH_2-CH_2$-, $-CH_2$-CH(OH)-$CH_2$-CH(OH)-$CH_2$-,
- $CH_2$-CH(OH)-$CH_2$-, $-CH_2-CH(OC_2H_5)$-,
- $CH_2-CH(OCOCH_3)-CH_2-CH(OCOCH_3)-CH_2$-,
- $CH_2-CH(OCOC_4H_9)-CH_2$-, -CO-, -CO-$CH_2$, -CO-$CH_2-CH_2$-,
- $CH_2COCH_2$-, -CH(Cl)-, -CH(Cl)-$CH_2$-, $-CH_2$-CH(Cl)-, $-CH(NH_2)$-,
- $CH(NH_2)-CH_2$-, $-CH(N(CH_3)_2)$-, $-CH(N(CH_3)_2)-CH_2$-,
- $CH_2-CH(N(CH_3)_2)-CH_2-CH(N(CH_3)_2)-CH_2$-, -CH(F)-, -CH(F)-$CH_2$-, $-CH_2$-CH(F)-$CH_2$, wobei die Hydroxysubstituenten in α- oder in β-Position stehen können,

Z einen gerad- oder verzweigtkettigen, gesättigten oder ungesättigten Kohlenwasserstoffrest mit bis zu 12 Kohtenstoffatomen, der an beliebigen Positionen Ketogruppen, α- oder β-Hydroxylgruppen,

welche ihrerseits verethert oder verestert sein können, der Aminogruppen, Fluor-, Chlor-, Bromatome aufweisen kann,

bedeuten.

**2.** Vitamin D-Derivate der allgemeinen Formel I nach Anspruch 1, worin Q eine unsubstituierte, unverzweigte Alkyleneinheit mit 1 oder 2 Kohlenstoffatomen und Z einen geradkettigen 1-Oxoalkylrest bedeutet.

**3.** Vitamin D-Derivate der allgemeinen Formel I nach Anspruch 1, worin Q eine $-CH(OH)-CH_2-CH_2-$ und Z einen geradkettigen 1-Oxoalkylrest bedeutet.

**4.** Vitamin D-Derivate der allgemeinen Formel I nach Anspruch 1, nämlich

(5*Z*,7*E*,22*E*)-(1*S*,3*R*)-25-Acetyl-26,27-cyclo-9,10-secocholesta-5,7,10(19),22-tetraen-1,3-diol,
(5*Z*,7*E*,22*E*)-(1*S*,3*R*)-25-(1-Oxopropyl)-26,27-cyclo-9,10-secocholesta-5,7,10(19),22-tetraen-1,3-diol,
(5*Z*,7*E*,22*E*)-(1*S*,3*R*)-25-(1-Oxobutyl)-26,27-cyclo-9,10-secocholesta-5,7,10(19),22-tetraen-1,3-diol,
(5*Z*,7*E*,22*E*)-(1*S*,3*R*)-25-(1-Oxopentyl)-26,27-cyclo-9,10-secocholesta-5,7,10(19),22-tetraen-1,3-diol,
(5*Z*,7*E*,22*E*)-(1*S*,3*R*)-25-(1 -Oxohexyl)-26,27-cyclo-9,10-secocholesta-5,7,10(19),22-tetraen-1,3-diol,
(5*Z*,7*E*,22*E*)-(1*S*,3*R*)-25-(1-Oxoheptyl)-26,27-cyclo-9,10-secocholesta-5,7,10(19),22-tetraen-1,3-diol,
(5*Z*,7*E*,22*E*)-(1*S*,3*R*)-25-(1 -Oxooctyl)-26,27-cyclo-9,10-secocholesta-5,7,10(19),22-tetraen-1,3-diol,
(5*Z*,7*E*,22*E*)-(1*S*,3*R*)-25-(1-Oxononyl)-26,27-cyclo-9,10-secocholesta-5,7,10(19),22-tetraen-1,3-diol,
(5*Z*,7*E*,22*E*)-(1*S*, 3*R*)-25-(1-Oxodecyl)-26,27-cyclo-9,10-secocholesta-5,7,10(19),22-tetraen-1,3-diol,
(5*Z*,7*E*)-(1*S*,3*R*,22*S*)-25-Acetyl-26,27-cyclo-9,10-secocholesta-5,7,10(19)-trien-1,3,22-triol,
(5*Z*,7*E*)-(1*S*,3*R*,22*R*)-25-Acetyl-26,27-cyclo-9,10-secocholesta-5,7,10(19)-trien-1,3,22-triol,
(5*Z*,7*E*)-(1*S*,3*R*,22*S*)-25-(1-Oxopropyl)-26,27-cyclo-9,10-secocholesta-5,7,10(19)-trien-1,3,22-triol,
(5*Z*,7*E*)-(1*S*,3*R*,22*R*)-25-(1-Oxopropyl)-26,27-cyclo-9,10-secocholesta-5,7,10(19)-trien-1,3,22-triol,
(5*Z*,7*E*)-(1*S*,3*R*,22*S*)-25-(1-Oxobutyl)-26,27-cyclo-9,10-secocholesta-5,7,10(19)-trien-1,3,22-triol,
(5*Z*,7*E*)-(1*S*,3*R*,22*R*)-25-(1-Oxobutyl)-26,27-cyclo-9,10-secocholesta-5,7,10(19)-trien-1,3,22-triol,
(5*Z*,7*E*)-(1*S*,3*R*,22*S*)-25-(1-Oxopentyl)-26,27-cyclo-9,10-secocholesta-5,7,10(19)-trien-1,3,22-triol,
(5*Z*,7*E*)-(1*S*,3*R*,22*R*)-25-(1-Oxopentyl)-26,27-cyclo-9,10-secocholesta-5,7,10(19)-trien-1,3,22-triol
(5*Z*,7*E*)-(1*S*,3*R*,22*S*)-25-(1-Oxohexyl)-26,27-cyclo-9,10-secocholesta-5,7,10(19)-trien-1,3,22-triol
(5*Z*,7*E*)-(1*S*,3*R*,22*R*)-25-(1-Oxohexyl)-26,27-cyclo-9,10-secocholesta-5,7,10(19)-trien-1,3,22-triol
(5*Z*,7*E*)-(1*S*,3*R*,22*S*)-25-(1-Oxoheptyl)-26,27-cyclo-9,10-secocholesta-5,7,10(19)-trien-1,3,22-triol
(5*Z*,7*E*)-(1*S*,3*R*,22*R*)-25-(1-Oxoheptyl)-26,27-cyclo-9,10-secocholesta-5,7,10(19)-trien-1,3,22-triol
(5*Z*,7*E*)-(1*S*,3*R*,22*S*)-25-(1-Oxooctyl)-26,27-cyclo-9,10-secocholesta-5,7,10(19)-trien-1,3,22-triol
(5*Z*,7*E*)-(1*S*,3*R*,22*R*)-25-(1-Oxooctyl)-26,27-cyclo-9,10-secocholesta-5,7,10(19)-trien-1,3,22-triol
(5*Z*,7*E*)-(1*S*,3*R*,22*S*)-25-(1-Oxononyl)-26,27-cyclo-9,10-secocholesta-5,7,10(19)-trien-1,3,22-triol
(5*Z*,7*E*)-(1*S*,3*R*,22*R*)-25-(1-Oxononyl)-26,27-cyclo-9,10-secocholesta-5,7,10(19)-trien-1,3,22-triol
(5*Z*,7*E*)-(1*S*,3*R*,22*S*)-25-(1 -Oxodecyl)-26,27-cyclo-9,10-secocholesta-5,7,10(19)-trien-1,3,22-triol
(5*Z*,7*E*)-(1*S*,3*R*,22*R*)-25-(1-Oxodecyl)-26,27-cyclo-9,10-secocholesta-5,7,10(19)-trien-1,3,22-triol
(5*Z*,7*E*,22*E*)-(1*S*,3*R*)-25-Acetyl-26,27-cyclo-24a-homo-9,10-secocholesta-5,7,10(19),22-tetraen-1,3-diol,
(5*Z*,7*E*,22*E*)-(1*S*,3*R*)-25-(1-Oxopropyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxobutyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxopentyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxohexyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxoheptyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxooctyl)-26,27-cyclo-24a-homo-9,10-secocholesta-5,7,10(19),22-tetraen-1,3-diol,
(5*Z*,7*E*,22*E*)-(1*S*,3*R*)-25-(1 -Oxononyl)-26,27-cyclo-24a-homo-9,10-secocholesta-5,7,10(19),22-tetraen-1,3-diol,
(5*Z*,7*E*,22*E*)-(1*S*,3*R*)-25-(1-Oxodecyl)-26,27-cyclo-24a-homo-9,10-secocholesta-5,7,10(19),22-tetraen-1,3-diol,
(5*Z*,7*E*)-(1*S*,3*R*,22*S*)-25-Acetyl-26,27-cyclo-24a-homo-9,10-secocholesta-5,7,10(19)-trien-1,3,22-triol,

(5*Z*,7*E*)-(1*S*,3*R*,22*R*)-25-Acetyl-26,27-cyclo-24a-homo-9,10-secocholesta-5,7,10(19)-trien-1,3,22-triol,
(5*Z*,7*E*)-(1*S*,3*R*,22*S*)-25-(1-Oxopropyl)-26,27-cyclo-24a-homo-9,10-secocholesta-5,7,10(19)-trien-1,3,22-triol,
(5*Z*,7*E*)-(1*S*,3*R*,22*R*)-25-(1-Oxopropyl)-26,27-cyclo-24a-homo-9,10-secocholesta-5,7,10(19)-trien-1,3,22-triol,
(5*Z*,7*E*)-(1*S*,3*R*,22*S*)-25-(1-Oxobutyl)-26,27-cyclo-24a-homo-9,10-secocholesta-5,7,10(19)-trien-1,3,22-triol,
(5*Z*,7*E*)-(1*S*,3*R*,22*R*)-25-(1-Oxobutyl)-26,27-cyclo-24a-homo-9,10-secocholesta-5,7,10(19)-trien-1,3,22-triol,
(5*Z*,7*E*)-(1*S*,3*R*,22*S*)-25-(1-Oxopentyl)-26,27-cyclo-24a-homo-9,10-secocholesta-5,7,10(19)-trien-1,3,22-triol,
(5*Z*,7*E*)-(1*S*,3*R*,22*R*)-25-(1-Oxopentyl)-26,27-cyclo-24a-homo-9,10-secocholesta-5,7,10(19)-trien-1,3,22-triol,
(5*Z*,7*E*)-(1*S*,3*R*,22*S*)-25-(1-Oxohexyl)-26,27-cyclo-24a-homo-9,10-secocholesta-5,7,10(19)-trien-1,3,22-triol,
(5*Z*,7*E*)-(1*S*,3*R*,22*R*)-25-(1-Oxohexyl)-26,27-cyclo-24a-homo-9,10-secocholesta-5,7,10(19)-trien-1,3,22-triol,
(5*Z*,7*E*)-(1*S*,3*R*,22*S*)-25-(1-Oxoheptyl)-26,27-cyclo-24a-homo-9,10-secocholesta-5,7,10(19)-trien-1,3,22-triol,
(5*Z*,7*E*)-(1*S*,3*R*,22*R*)-25-(1-Oxoheptyl)-26,27-cyclo-24a-homo-9,10-secocholesta-5,7,10(19)-trien-1,3,22-triol,
(5*Z*,7*E*)-(1*S*,3*R*,22*S*)-25-(1-Oxooctyl)-26,27-cyclo-24a-homo-9,10-secocholesta-5,7,10(19)-trien-1,3,22-triol,
(5*Z*,7*E*)-(1*S*,3*R*,22*R*)-25-(1-Oxooctyl)-26,27-cyclo-24a-homo-9,10-secocholesta-5,7,10(19)-trien-1,3,22-triol,
(5*Z*,7*E*)-(1*S*,3*R*,22*S*)-25-(1-Oxononyl)-26,27-cyclo-24a-homo-9,10-secocholesta-5,7,10(19)-trien-1,3,22-triol,
(5*Z*,7*E*)-(1*S*,3*R*,22*R*)-25-(1-Oxononyl)-26,27-cyclo-24a-homo-9,10-secocholesta-5,7,10(19)-trien-1,3,22-triol,
(5*Z*,7*E*)-(1*S*,3*R*,22*S*)-25-(1-Oxodecyl)-26,27-cyclo-24a-homo-9,10-secocholesta-5,7,10(19)-trien-1,3,22-triol,
(5*Z*,7*E*)-(1*S*,3*R*,22*R*)-25-(1 -Oxodecyl)-26,27-cyclo-24a-homo-9,10-secocholesta-5,7,10(19)-trien-1,3,22-triol,
(5*Z*,7*E*,22*E*)-(1*S*,3*R*,24*R*)-25-Acetyl-26,27-cyclo-24a,24b-dihomo-9,10-secocholesta-5,7,10(19),22-tetraen-1,3,24-triol,
(5*Z*,7*E*,22*E*)-(1*S*,3*R*,24*S*)-25-Acetyl-26,27-cyclo-24a,24b-dihomo-9,10-secocholesta-5,7,10(19),22-tetraen-1,3,24-triol,
(5*Z*,7*E*,22*E*)-(1*S*,3*R*,24*R*)-25-(1-Oxopropyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*S*)-25-(1-Oxopropyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*R*)-25-(1-Oxobutyl)-26,27-cyclo-24a,24b-dihomo-9,10-secocholesta-5,7,10(19), 22-tetraen-1,3,24-triol,
(5*Z*,7*E*,22*E*)-(1*S*,3*R*,24*S*)-25-(1-Oxobutyl)-26,27-cyclo-24a,24b-dihomo-9,10-secocholesta-5,7,10(19), 22-tetraen-1,3,24-triol,
(5*Z*,7*E*,22*E*)-(1*S*,3*R*,24*R*)-25-(1-Oxopentyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*S*)-25-(1-Oxopentyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*R*)-25-(1-Oxohexyl)-26,27-cyclo-24a,24b-dihomo-9,10-secocholesta-5,7,10(19), 22-tetraen-1,3,24-triol,
(5*Z*,7*E*,22*E*)-*(1S,3R,24S)-25*-(1-Oxohexyl)-26,27-cyclo-24a,24b-dihomo-9,10-secocholesta-5,7,10(19), 22-tetraen-1,3,24-triol,
(5*Z*,7*E*,22*E*)-(1*S*,3*R*,24*R*)-25-(1-Oxoheptyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*S*)-25-(1-Oxoheptyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*R*)-25-(1-Oxooctyl)-26,27-cyclo-24a,24b-dihomo-9,10-secocholesta-5,7,10(19),

22-tetraen-1,3,24-triol,
(5*Z*,7*E*,22*E*)-(1*S*,3*R*,24*S*)-25-(1-Oxooctyl)-26,27-cyclo-24a,24b-dihomo-9,10-secocholesta-5,7,10(19), 22-tetraen-1,3,24-triol,
(5*Z*,7*E*,22*E*)-(1*S*,3*R*,24*R*)-25-(1-Oxononyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*S*)-25-(1-Oxononyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*R*)-25-(1-Oxodecyl)-26,27-cyclo-24a,24bdihomo-9,10-secocholesta-5,7,10(19), 22-tetraen-1,3,24-triol,
(5*Z*,7*E*,22*E*)-(1*S*,3*R*,24*S*)-25-(1-Oxodecyl)-26,27-cyclo-24a,24b-dihomo-9,10-secocholesta-5,7,10(19), 22-tetraen-1,3,24-triol,
(5*Z*,7*E*,22*E*)-(1*S*,3*R*,24*R*)-25-Acetyl-24-methoxy-26,27-cyclo-24a,24bdihomo-9,10-secocholesta-5,7,10 (19),22-tetraen-1,3-diol,
(5*Z*,7*E*,22*E*)-(1*S*,3*R*,24*S*)-25-Acetyl-24-methoxy-26,27-cyclo-24a,24bdihomo-9,10-secocholesta-5,7,10(19), 22-tetraen-1,3-diol,
(5*Z*,7*E*,22*E*)-(1*S*,3*R*,24*R*)-24-Methoxy-25-(1-oxopropyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxopropyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxobutyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxobutyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxopentyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxopentyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxohexyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxohexyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxoheptyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxoheptyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxooctyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxooctyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxononyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxononyl)-26,27-cyclo-24a,24b-dihomo-9,10-secocholesta-5,7,10(19),22-tetraen-1,3-diol,
(5*Z*,7*E*,22*E*)-(1*S*,3*R*,24*R*)-24-Methoxy-25-(1-oxodecyl)-26,27-cyclo-24a,24b-dihomo-9,10-secocholesta-5,7,10(19),22-tetraen-1,3-diol,
(5*Z*,7*E*,22*E*)-(1*S*,3*R*,24*S*)-24-Methoxy-25-(1-oxodecyl)-26,27-cyclo-24a,24b-dihomo-9,10-secocholesta-5,7,10(19),22-tetraen-1,3-diol,

**5.** Verfahren zur Herstellung der Vitamin D-Derivate der allgemeinen Formel I, wobei eine Verbindung der allgemeinen Formel II

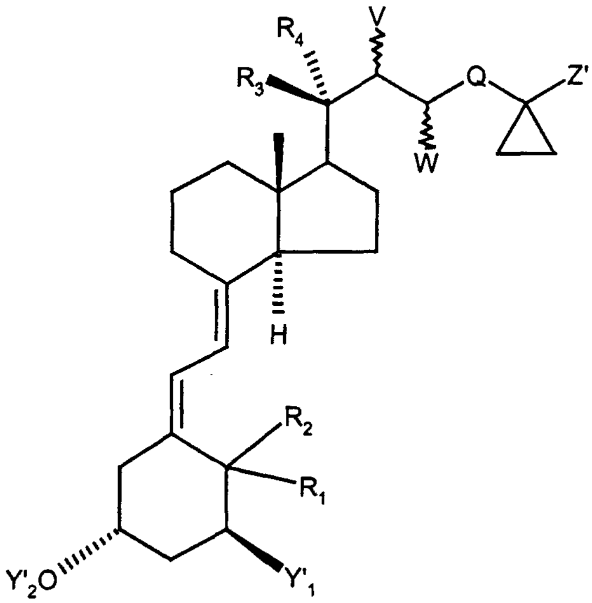

(II)

worin Y'$_1$ ein Wasserstoffatom, ein Halogenatom oder eine geschützte Hydroxylgruppe und Y'$_2$ eine Hydroxyschutzgruppe bedeuten,
durch gleichzeitige oder sukzessive Abspaltung der Hydroxy- sowie Ketoschutzgruppen und gegebenenfalls durch partielle oder vollständige Veresterung der freien Hydroxylgruppen in die Verbindung der allgemeinen Formel I überführt wird.

6. Verwendung der Vitamin D-Derivate der allgemeinen Formel I zur Herstellung von Arzneimitteln.

7. Verwendung der Vitamin D-Derivate der allgemeinen Formel I nach Anspruch 6 zur Herstellung von Arzneimitteln zur Therapie von hyperproliferativen Hauterkrankungen, Pruritus, Tumorerkrankungen, Präkanzerosen, Störungen des Immunsystems, entzündliche Erkrankungen, rheumatoide Arthritis, Asthma, Autoimmunerkrankungen, Multiple Sklerose, Diabetes Mellitus, AIDS sowie Abstoßungsreaktionen bei autologen, allogenen oder xenogenen Transplantaten.

8. Verwendung gemäß Anspruch 6, **dadurch gekennzeichnet, daß** das Arzneimittel weiterhin mit anderen immunsuppressiv wirksamen Stoffen wie Cyclosporin A, FK 506, Rapamycin und Anti-CD 4-Antikörper kombiniert werden.

9. Verwendung der Vitamin D-Derivate der allgemeinen Formel I nach Anspruch 6 zur Herstellung von Arzneimitteln zur Therapie von atrophischer Haut-oder Wundheilung, der Therapie von sekundären Hyperparathyroidimus, renaler Osteodystrophie sowie seniler und postmenopausaler Osteoporose, Diabetes mellitus Typ II und der Therapie von degenerativen Erkrankungen des peripheren und zentralen Nervensystems sowie auch der Regulation des Haarwachstums.

10. Verwendung von Vitamin D-Derivaten der allgemeinen Formel I nach Anspruch 6, die die Wirkung von Calcitriol in HL 60 - Zellen antagonisieren, zur Therapie von Hypercalcämien oder granulomatösen Erkrankungen, von paraneoplastischer Hypercalcämien, von Hypercalcämie bei Hyperparathyroidismus, zur männlichen und weiblichen Fertilitätskontrolle oder als Immunstimulantien sowie bei Hirsutismus, zur Therapie und Prophylaxe der Arteriosklerose und zur Therapie von entzündlichen Erkrankungen.

11. Zwischenprodukte der allgemeinen Formel **XII** innerhalb der Herstellung der Vitamin D-Derivate der allgemeinen Formel **I** nach Anspruch 1:

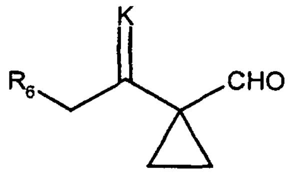

worin
$R_6$ eine geradkettige oder verzweigte Alkylgruppe mit bis zu 11 Kohlenstoffatomen und K eine Ketoschutzgruppe bedeutet.

**12.** Zwischenprodukt nach Anspruch 11, **dadurch gekennzeichnet, daß** K für 1,3-Dioxolan, 1,3-Dioxan, 5,5-Dimethyl-1,3-dioxan oder ein Dialkoxyketal steht .

## Claims

**1.** Vitamin D derivatives of the general formula I

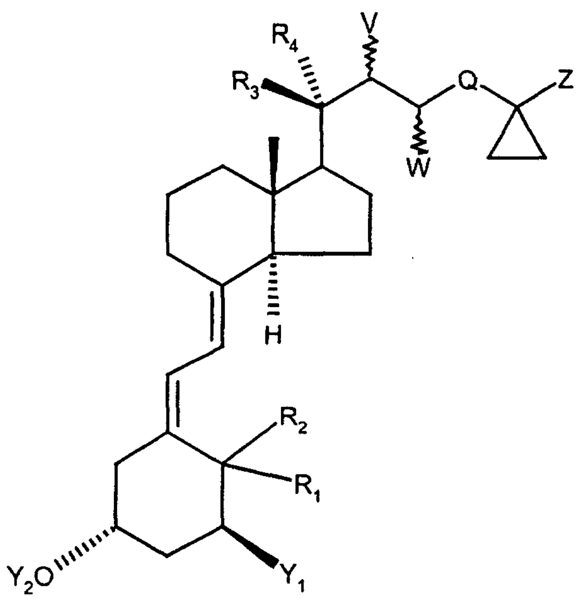

in which

| | |
|---|---|
| $Y_1$ | is a hydrogen atom, a hydroxyl group, a fluorine, chlorine or bromine atom or a group $-OCOR_8$ in which |
| | $R_8$ is an aliphatic or aromatic radical having 1 to 12 C atoms, |
| $Y_2$ | is a hydrogen atom or a group $-(CO)R_9$ in which |
| | $R_9$ is an aliphatic or aromatic radical having 1 to 12 C atoms, |
| $R_1$ and $R_2$ | are each a hydrogen atom or together are an exocyclic methylene group, |
| $R_3$ and $R_4$ | independently of one another are a hydrogen atom, a chlorine or fluorine atom, an alkyl group having 1 to 4 carbon atoms, together are a methylene group or together with the quaternary carbon atom |

20 are a 3-7-membered, saturated or unsaturated carbocyclic ring,

V and W together are an *E* double bond or V is a hydroxyl group and W is a hydrogen atom,

Q is a group selected from

$-CH_2-$, $-(CH_2)_2-$, $-(CH_2)_3-$, $-(CH_2)_4-$,
$-(CH_2)_7-$, $-CH_2-C(CH_3)_2-CH_2-$,
$-CH_2-CH(CH_3)-CH_2-CH(CH_3)-CH_2-$, $-CH_2-CH(OH)-$,
$-CH_2-CH_2-CH(OH)-$, $-CH(OH)-CH_2-$,
$-CH(OH)-CH_2-CH_2-$, $-CH_2-CH(OH)-CH_2-CH(OH)-CH_2-$,
$-CH_2-CH(OH)-CH_2-$, $-CH_2-CH(OC_2H_5)-$,
$-CH_2-CH(OCOCH_3)-CH_2-CH(OCOCH_3)-CH_2-$,
$-CH_2-CH(OCOC_4H_9)-CH_2-$, $-CO-$, $-CO-CH_2$,
$-CO-CH_2-CH_2-$, $-CH_2COCH_2-$, $-CH(Cl)-$,
$-CH(Cl)-CH_2-$, $-CH_2-CH(Cl)-$, $-CH(NH_2)-$,
$-CH(NH_2)-CH_2-$, $-CH(N(CH_3)_2)-$, $-CH(N(CH_3)_2)-CH_2-$,
$-CH_2-CH(N(CH_3)_2)-CH_2-CH(N(CH_3)_2)-CH_2-$, $-CH(F)-$,
$-CH(F)-CH_2-$, $-CH_2-CH(F)-CH_2$, where the hydroxyl substituents may be in the α or β position,

Z is a straight- or branched-chain, saturated or unsaturated hydrocarbon radical having up to 12 carbon atoms, which may have at any positions keto groups, α- or β-hydroxyl groups, which in turn may be etherified or esterified, or amino groups, fluorine, chlorine or bromine atoms.

**2.** Vitamin D derivatives of the general formula I according to Claim 1, in which Q is an unsubstituted, unbranched alkylene unit having 1 or 2 carbon atoms and Z is a straight-chain 1-oxoalkyl radical.

**3.** Vitamin D derivatives of the general formula I according to Claim 1, in which Q is $-CH(OH)-CH_2-CH_2-$ and Z is a straight-chain 1-oxoalkyl radical.

**4.** Vitamin D derivatives of the general formula I according to Claim 1, namely

(5*Z*,7*E*,22*E*)-(1*S*,3*R*)-25-acetyl-26,27-cyclo-9,10-secocholesta-5,7,10(19),22-tetraene-1,3-diol,
(5*Z*,7*E*,22*E*)-(1*S*,3*R*)-25- (1-oxopropyl) -26,27-cyclo-9,10-secocholesta-5,7,10(19),22-tetraene-1,3-diol,
(5*Z*,7*E*,22*E*)-(1*S*,3*R*)-25-(1-oxobutyl)-26,27-cyclo-9,10-secocholesta-5,7,10(19),22-tetraene-1,3-diol,
(5*Z*,7*E*,22*E*)-(1*S*,3*R*)-25-(1-oxopentyl)-26,27-cyclo-9,10-secocholesta-5,7,10(19),22-tetraene-1,3-diol,
(5*Z*,7*E*,22*E*)-(1*S*,3*R*)-25- (1-oxohexyl) -26,27-cyclo-9,10-secocholesta-5,7,10(19),22-tetraene-1,3-diol,
(5*Z*,7*E*,22*E*)-(1*S*,3*R*)-25-(1-oxoheptyl) -26,27-cyclo-9,10-secocholesta-5,7,10(19),22-tetraene-1,3-diol,
(5*Z*,7*E*,22*E*)-(1*S*,3*R*)-25- (1-oxooctyl) -26,27-cyclo-9,10-secocholesta-5,7,10(19),22-tetraene-1,3-diol,
(5*Z*,7*E*,22*E*)-(1*S*,3*R*)-25-(1-oxononyl) -26,27-cyclo-9,10-secocholesta-5,7,10(19),22-tetraene-1,3-diol,
(5*Z*,7*E*,22*E*)-(1*S*,3*R*)-25-(1-oxodecyl)-26,27-cyclo-9,10-secocholesta-5,7,10(19),22-tetraene-1,3-diol,
(5*Z*,7*E*)-(1*S*,3*R*,22*S*)-25-acetyl-26,27-cyclo-9,10-secocholesta-5,7,10(19)-triene-1,3,22-triol,
(5*Z*,7*E*)-(1*S*,3*R*,22*R*)-25-acetyl-26,27-cyclo-9,10-secocholesta-5,7,10(19)-triene-1,3,22-triol,
(5*Z*,7*E*)-(1*S*,3*R*,22*S*)-25- (1-oxopropyl)-26,27-cyclo-9,10-secocholesta-5,7,10(19)-triene-1,3,22-triol,
(5*Z*,7*E*)-(1*S*,3*R*,22*R*)-25-(1-oxopropyl)-26,27-cyclo-9,10-secocholesta-5,7,10(19)-triene-1,3,22-triol,
(5*Z*,7*E*)-(1*S*,3*R*,22*S*)-25- (1-oxobutyl)-26,27-cyclo-9,10-secocholesta-5,7,10(19)-triene-1,3,22-triol,
(5*Z*,7*E*)-(1*S*,3*R*,22*R*)-25-(1-oxobutyl)-26,27-cyclo-9,10-secocholesta-5,7,10(19)-triene-1,3,22-triol,
(5*Z*,7*E*)-(1*S*,3*R*,22*S*)-25-(1-oxopentyl)-26,27-cyclo-9,10-secocholesta-5,7,10(19)-triene-1,3,22-triol,
(5*Z*,7*E*)-(1*S*,3*R*,22*R*)-25- (1-oxopentyl)-26,27-cyclo-9,10-secocholesta-5,7,10(19)-triene-1,3,22-triol,
(5*Z*,7*E*)-(1*S*,3*R*,22*S*)-25-(1-oxohexyl)-26,27-cyclo-9,10-secocholesta-5,7,10(19)-triene-1,3,22-triol,
(5*Z*,7*E*)-(1*S*,3*R*,22*R*)-25-(1-oxohexyl)-26,27-cyclo-9,10-secocholesta-5,7,10(19)-triene-1,3,22-triol,
(5*Z*,7*E*)-(1*S*,3*R*,22*S*)-25- (1-oxoheptyl)-26,27-cyclo-9,10-secocholesta-5,7,10(19)-triene-1,3,22-triol,
(5*Z*,7*E*)-(1*S*,3*R*,22*R*)-25-(1-oxoheptyl)-26,27-cyclo-9,10-secocholesta-5,7,10(19)-triene-1,3,22-triol,
(5*Z*,7*E*)-(1*S*,3*R*,22*S*)-25-(1-oxooctyl)-26,27-cyclo-9,10-secocholesta-5,7,10(19)-triene-1,3,22-triol,
(5*Z*,7*E*)-(1*S*,3*R*,22*R*)-25- (1-oxooctyl)-26,27-cyclo-9,10-secocholesta-5,7,10(19)-triene-1,3,22-triol,
(5*Z*,7*E*)-(1*S*,3*R*,22*S*)-25-(1-oxononyl)-26,27-cyclo-9,10-secocholesta-5,7,10(19)-triene-1,3,22-triol,
(5*Z*,7*E*)-(1*S*,3*R*,22*R*)-25- (1-oxononyl) -26,27-cyclo-9,10-secocholesta-5,7,10(19)-triene-1,3,22-triol,
(5*Z*,7*E*)-(1*S*,3*R*,22*S*)-25-(1-oxodecyl)-26,27-cyclo-9,10-secocholesta-5,7,10(19)-triene-1,3,22-triol,

(5*Z*,7*E*)-(1*S*,3*R*,22*R*)-25-(1-oxodecyl)-26,27-cyclo-9,10-secocholesta-5,7,10(19)-triene-1,3,22-triol,
(5*Z*,7*E*,22*E*) - (1*S*,3*R*)-25-acetyl-26,27-cyclo-24a-homo-9,10-secocholesta-5,7,10(19),22-tetraene-1,3-diol,
(5*Z*,7*E*,22*E*)-(1*S*,3*R*)-25-(1-oxopropyl)-26,27-cyclo-24a-homo-9,10-secocholesta-5,7,10(19),22-tetraene-1,3-diol,
(5*Z*,7*E*,22*E*)-(1*S*,3*R*)-25- (1-oxobutyl) -26,27-cyclo-24a-homo-9,10-secocholesta-5,7,10(19),22-tetraene-1,3-diol,
(5*Z*,7*E*,22*E*)-(1*S*,3*R*)-25-(1-oxopentyl)-26,27-cyclo-24a-homo-9,10-secocholesta-5,7,10(19),22-tetraene-1,3-diol,
(5*Z*,7*E*,22*E*)-(1*S*,3*R*)-25-(1-oxohexyl) -26,27-cyclo-24a-homo-9,10-secocholesta-5,7,10(19),22-tetraene-1,3-diol,
(5*Z*,7*E*,22*E*)-(1*S*,3*R*)-25- (1-oxoheptyl) -26,27-cyclo-24a-homo-9,10-secocholesta-5,7,10(19),22-tetraene-1,3-diol,
(5*Z*,7*E*,22*E*)-(1*S*,3*R*)-25-(1-oxooctyl)-26,27-cyclo-24a-homo-9,10-secocholesta-5,7,10(19),22-tetraene-1,3-diol,
(5*Z*,7*E*,22*E*)-(1*S*,3*R*)-25- (1-oxononyl) -26,27-cyclo-24a-homo-9,10-secocholesta-5,7,10(19),22-tetraene-1,3-diol,
(5*Z*,7*E*,22*E*)-(1*S*,3*R*)-25- (1-oxodecyl) -26,27-cyclo-24a-homo-9,10-secocholesta-5,7,10(19),22-tetraene-1,3-diol,
(5*Z*,7*E*)-(1*S*,3*R*,22*S*)-25-acetyl-26,27-cyclo-24a-homo-9,10-secocholesta-5,7,10(19)-triene-1,3,22-triol,
(5*Z*,7*E*)-(1*S*,3*R*,22*R*)-25-acetyl-26,27-cyclo-24a-homo-9,10-secocholesta-5,7,10(19)-triene-1,3,22-triol,
(5*Z*,7*E*)-(1*S*,3*R*,22*S*)-25-(1-oxopropyl)-26,27-cyclo-24a-homo-9,10-secocholesta-5,7,10(19)-triene-1,3,22-triol,
(5*Z*,7*E*)-(1*S*,3*R*,22*R*)-25- (1-oxopropyl) -26,27-cyclo-24a-homo-9,10-secocholesta-5,7,10(19)-triene-1,3,22-triol,
(5*Z*,7*E*)-(1*S*,3*R*,22*S*)-25- (1-oxobutyl) -26,27-cyclo-24a-homo-9,10-secocholesta-5,7,10(19)-triene-1,3,22-triol,
(5*Z*,7*E*)-(1*S*,3*R*,22*R*)-25-(1-oxobutyl)-26,27-cyclo-24a-homo-9,10-secocholesta-5,7,10(19)-triene-1,3,22-triol,
(5*Z*,7*E*)-(1*S*,3*R*,22*S*)-25- (1-oxopentyl) -26,27-cyclo-24a-homo-9,10-secocholesta-5,7,10(19)-triene-1,3,22-triol,
(5*Z*,7*E*)-(1*S*,3*R*,22*R*)-25-(1-oxopentyl)-26,27-cyclo-24a-homo-9,10-secocholesta-5,7,10(19)-triene-1,3,22-triol,
(5*Z*,7*E*)-(1*S*,3*R*,22*S*)-25-(1-oxohexyl)-26,27-cyclo-24a-homo-9,10-secocholesta-5,7,10(19)-triene-1,3,22-triol,
(5*Z*,7*E*)-(1*S*,3*R*,22*R*)-25-(1-oxohexyl)-26,27-cyclo-24a-homo-9,10-secocholesta-5,7,10(19)-triene-1,3,22-triol,
(5*Z*,7*E*)-(1*S*,3*R*,22*S*)-25- (1-oxoheptyl) -26,27-cyclo-24a-homo-9,10-secocholesta-5,7,10(19)-triene-1,3,22-triol,
(5*Z*,7*E*)-(1*S*,3*R*,22*R*)-25-(1-oxoheptyl)-26,27-cyclo-24a-homo-9,10-secocholesta-5,7,10(19)-triene-1,3,22-triol,
(5*Z*,7*E*)-(1*S*,3*R*,22*S*)-25-(1-oxooctyl) -26,27-cyclo-24a-homo-9,10-secocholesta-5,7,10(19)-triene-1,3,22-triol,
(5*Z*,7*E*)-(1*S*,3*R*,22*R*)-25-(1-oxooctyl)-26,27-cyclo-24a-homo-9,10-secocholesta-5,7,10(19)-triene-1,3,22-triol,
(5*Z*,7*E*)-(1*S*,3*R*,22*S*)-25-(1-oxononyl)-26,27-cyclo-24a-homo-9,10-secocholesta-5,7,10(19)-triene-1,3,22-triol,
(5*Z*,7*E*)-(1*S*,3*R*,22*R*)-25-(1-oxononyl)-26,27-cyclo-24a-homo-9,10-secocholesta-5,7,10(19)-triene-1,3,22-triol,
(5*Z*,7*E*)-(1*S*,3*R*,22*S*)-25-(1-oxodecyl)-26,27-cyclo-24a-homo-9,10-secocholesta-5,7,10(19)-triene-1,3,22-triol,
(5*Z*,7*E*)-(1*S*,3*R*,22*R*)-25- (1-oxodecyl) -26,27-cyclo-24a-homo-9,10-secocholesta-5,7,10(19)-triene-1,3,22-triol,
(5*Z*,7*E*,22*E*)-(1*S*,3*R*,24*R*)-25-acetyl-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25-acetyl-26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25- (1-oxopropyl)-26,27-cyclo-24a,24b-dihomo-9,10-secocholesta-5,7,10(19),22-tetraene-1,3,24-triol,

(5*Z*,7*E*,22*E*)-(1*S*,3*R*,24*S*)-25- (1-oxopropyl) -26,27-cyclo-24a,24b-dihomo-9,10-secocholesta-5,7,10(19), 22-tetraene-1,3,24-triol,
(5*Z*,7*E*,22*E*)-(1*S*,3*R*,24*R*)-25-(1-oxobutyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25- (1-oxobutyl) -26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25-(1-oxopentyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25-(1-oxopentyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25- (1-oxohexyl) -26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25- (1-oxohexl)-26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25-(1-oxoheptyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25-(1-oxoheptyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25-(1-oxooctyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25- (1-oxooctyl) -26,27-cyclo-24a,24b-dihomo-9,10-secocholesta-5,7,10 (19),22-tetraene-1,3,24-triol,
(5*Z*,7*E*,22*E*)-(1*S*,3*R*,24*R*)-25-(1-oxononyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25-(1-oxononyl)-26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25- (1-oxodecyl) -26,27-cyclo-24a,24b-dihomo-9,10-secocholesta-5,7,10(19),22-tetraene-1,3,24-triol,
(5*Z*,7*E*,22*E*)-(1*S*,3*R*,24*S*)-25- (1-oxodecyl) -26,27-cyclo-24a,24b-dihomo-9,10-secocholesta-5,7,10(19),22-tetraene-1,3,24-triol,
(5*Z*,7*E*,22*E*)-(1*S*,3*R*,24*R*)-25-acetyl-24-methoxy-26,27-cyclo-24a,24b-dihomo-9,10-secocholesta-5,7,10 (19),22-tetraene-1,3-diol,
(5*Z*,7*E*,22*E*)-(1*S*,3*R*,24*S*)-25-acetyl-24-methoxy-26,27-cyclo-24a,24b-dihomo-9,10-secocholesta-5,7,10 (19),22-tetraene-1,3-diol,
(5*Z*,7*E*,22*E*)-(1*S*,3*R*,24*R*)-24-methoxy-25- (1-oxopropyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25- (1-oxopropyl)-26,27-cyclo-24a,24b-dihomo-9,10-secocholesta-5, 7,10(19),22-tetraene-1,3-diol,
(5*Z*,7*E*,22*E*)-(1*S*,3*R*,24*R*)-24-methoxy-25-(1-oxobutyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25-(1-oxobutyl)-26,27-cyclo-24a,24b-dihomo-9,10-secocholesta-5, 7,10(19),22-tetraene-1,3-diol,
(5*Z*,7*E*,22*E*)-(1*S*,3*R*,24*R*)-24-methoxy-25-(1-oxopentyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25- (1-oxopentyl)-26,27-cyclo-24a,24b-dihomo-9,10-secocholesta-5, 7,10(19),22-tetraene-1,3-diol,
(5*Z*,7*E*,22*E*)-(1*S*,3*R*,24*R*)-24-methoxy-25- (1-oxohexyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25-(1-oxohexyl)-26,27-cyclo-24a,24b-dihomo-9,10-secocholesta-5, 7,10(19),22-tetraene-1,3-diol,
(5*Z*,7*E*,22*E*)-(1*S*,3*R*,24*R*)-24-methoxy-25-(1-oxoheptyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25-(1-oxoheptyl)-26,27-cyclo-24a,24b-dihomo-9,10-secocholesta-5, 7,10(19),22-tetraene-1,3-diol,
(5*Z*,7*E*,22*E*)-(1*S*,3*R*,24*R*)-24-methoxy-25- (1-oxooctyl)-26,27-cyclo-24a,24b-dihomo-9,10-secocholesta-5, 7,10(19),22-tetraene-1,3-diol,
(5*Z*,7*E*,22*E*)-(1*S*,3*R*,24*S*)-24-methoxy-25-(1-oxooctyl)-26,27-cyclo-24a,24b-dihomo-9,10-secocholesta-5, 7,10(19),22-tetraene-1,3-diol,

(5*Z*,7*E*,22*E*)-(1*S*,3*R*,24*R*)-24-methoxy-25- (1-oxononyl)-26,27-cyclo-24a,24b-dihomo-9,10-secocholesta-5,7,10(19),22-tetraene-1,3-diol,
(5*Z*,7*E*,22*E*)-(1*S*,3*R*,24*S*)-24-methoxy-25- (1-oxononyl)-26,27-cyclo-24a,24b-dihomo-9,10-secocholesta-5,7,10(19),22-tetraene-1,3-diol,
(5*Z*,7*E*,22*E*)-(1*S*,3*R*,24*R*)-24-methoxy-25- (1-oxodecyl)-26,27-cyclo-24a,24b-dihomo-9,10-secocholesta-5,7,10(19),22-tetraene-1,3-diol,
(5*Z*,7*E*,22*E*)-(1*S*,3*R*,24*S*)-24-methoxy-25- (1-oxodecyl)-26,27-cyclo-24a,24b-dihomo-9,10-secocholesta-5,7,10(19),22-tetraene-1,3-diol.

**5.** Process for the preparation of the vitamin D derivatives of the general formula I, where a compound of the general formula II

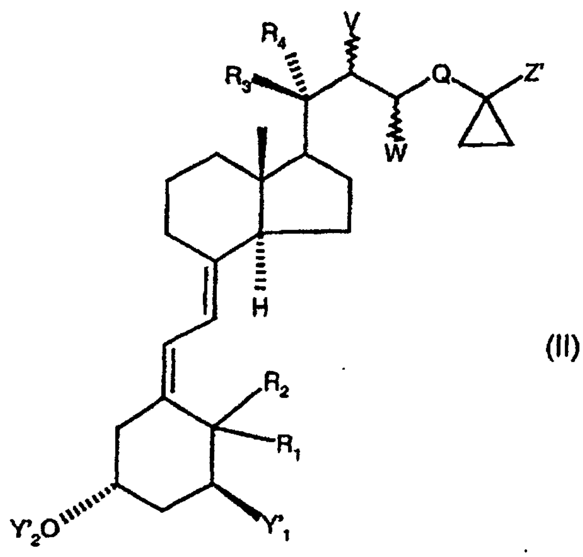

(II)

in which $Y'_1$ is a hydrogen atom, a halogen atom or a protected hydroxyl group and $Y'_2$ is a hydroxyl protective group is converted by a simultaneous or successive elimination of the hydroxyl and keto protective groups and, where appropriate, by partial or complete esterification of the free hydroxyl groups into the compound of the general formula I.

**6.** Use of the vitamin D derivatives of the general formula I for producing medicaments.

**7.** Use of the vitamin D derivatives of the general formula I according to Claim 6 for producing medicaments for the therapy of hyperproliferative skin disorders, pruritus, oncoses, precancerous diseases, disorders of the immune system, inflammatory disorders, rheumatoid arthritis, asthma, autoimmune diseases, multiple sclerosis, diabetes mellitus, AIDS and rejection reactions in cases of autologous, allogeneic or xenogeneic transplants.

**8.** Use according to Claim 6, **characterized in that** the medicament is additionally combined with other substances with immunosuppressant activity, such as cyclosporin A, FK 506, rapamycin and anti-CD 4 antibodies.

**9.** Use of the vitamin D derivatives of the general formula I according to Claim 6 for producing medicaments for the therapy of atrophic skin or wound healing, the therapy of secondary hyperparathyroidism, renal osteodystrophy, and senile and post-menopausal osteoporosis, type 2 diabetes mellitus and the therapy of degenerative disorders of the peripheral and central nervous system, and also of the regulation of hair growth.

**10.** Use of vitamin D derivatives of the general formula I according to Claim 6, which antagonise the action of calcitriol in HL 60 cells, for the therapy of hypercalcaemias or granulomatous disorders, of paraneoplastic hypercalcaemias, of hypercalcaemia associated with hyperparathyroidism, for control of male and female fertility, or as immunostimulants, and for hirsutism, for the therapy and prophylaxis of arteriosclerosis and for the therapy of inflammatory disorders.

**11.** Intermediates of the general formula **XII** within the preparation of the vitamin D derivatives of the general formula **I** according to Claim 1:

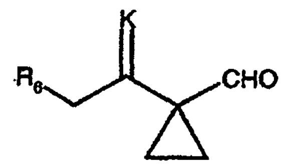

in which
$R_6$ is a straight-chain or branched alkyl group having up to 11 carbon atoms and K is a keto protective group.

**12.** Intermediate according to Claim 11, **characterized in that** K is 1,3-dioxolane, 1,3-dioxane, 5,5-dimethyl-1,3-dioxane or a dialkoxy ketal.

**Revendications**

**1.** Dérivés de la vitamine D de formule générale I

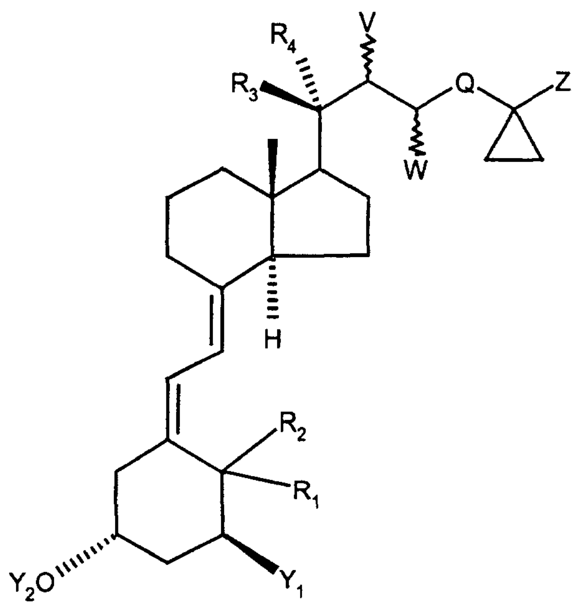

dans laquelle

$Y_1$ représente un atome d'hydrogène, un groupe hydroxy, un atome de fluor, de chlore ou de brome ou un groupe $-OCOR_8$, dans lequel

$R^8$ représente un radical aliphatique ou aromatique ayant de 1 à 12 atomes de carbone,

$Y_2$ représente un atome d'hydrogène ou un groupe $-(CO)R_9$, dans lequel

$R_9$ représente un radical aliphatique ou aromatique ayant de 1 à 12 atomes de carbone,

$R_1$ et $R_2$ représentent chacun un atome d'hydrogène ou conjointement un groupe méthylène exocyclique,

$R_3$ et $R_4$ représentent, indépendamment l'un de l'autre, un atome d'hydrogène, un atome de chlore ou de fluor, un groupe alkyle ayant de 1 à 4 atomes de carbone, conjointement avec un groupe méthylène ou conjointement avec l'atome de carbone quaternaire 20, un noyau carbocyclique saturé ou insaturé à 3-7 chaînons,

V et W représentent conjointement une double liaison E ou V représente un groupe hydroxy et W représente un atome d'hydrogène,

Q représente un groupe choisi parmi

$-CH_2-$, $-(CH_2)_2-$, $-(CH_2)_3-$, $-(CH_2)_4-$, $-(CH_2)_7-$,
$-CH_2-C(CH_3)_2-CH_2-$, $-CH_2-CH(CH_3)-CH_2-CH(CH_3)-CH_2-$,
$-CH_2-CH(OH)-$, $-CH_2-CH_2-CH(OH)-$, $-CH(OH)-CH_2-$,
$-CH(OH)-CH_2-CH_2-$, $-CH_2-CH(OH)-CH_2-CH(OH)-CH_2-$,
$-CH_2-CH(OH)-CH_2-$, $-CH_2-CH(OC_2H_5)-$,
$-CH_2-CH(OCOCH_3)-CH_2-CH(OCOCH_3)-CH_2-$,
$-CH_2-CH(OCOC_4H_9)-CH_2-$, $-CO-$, $-CO-CH_2$, $-CO-CH_2-CH_2-$,
$-CH_2COCH_2-$, $-CH(Cl)-$, $-CH(Cl)-CH_2-$, $-CH_2-CH(Cl)-$,
$-CH(NH_2)-$, $-CH(NH_2)-CH_2-$, $-CH(N(CH_3)_2)-$,
$-CH(N(CH_3)_2)-CH_2-$, $-CH_2-CH(N(CH_3)_2)-CH_2-CH$ $(N-$ $(CH_3)_2)-CH_2-$,
$-CH(F)-$, $-CH(F)-CH_2-$, $-CH_2-CH(F)-CH_2$, où les substituants hydroxy peuvent se trouver en position $\alpha$ ou $\beta$,

Z représente un radical hydrocarboné linéaire ou ramifié, saturé ou insaturé, ayant jusqu'à 12 atomes de carbone, qui peut renfermer en positions quelconques des groupes céto, des groupes $\alpha$ ou $\beta$-hydroxy qui peuvent être à leur tour éthérifiés ou estérifiés, des groupes amino, des atomes de fluor, de chlore, de brome.

**2.** Dérivés de la vitamine D de formule générale I selon la revendication 1, dans laquelle Q représente un motif alkylène non ramifié et non substitué ayant 1 ou 2 atomes de carbone et Z représente un radical 1-oxoalkyle linéaire.

**3.** Dérivés de la vitamine D de formule générale I selon la revendication 1, dans laquelle Q représente un radical $-CH(OH)-CH_2-CH_2-$ et Z représente un radical 1-oxoalkyle linéaire.

**4.** Dérivé de la vitamine D de formule générale I selon la revendication 1, à savoir

le (5*Z*,7*E*,22*E*)-(1*S*,3*R*)-25-acétyl-26,27-cyclo-9,10-sécocholesta-5,7,10(19),22-tétraène-1,3-diol,
le (5*Z*,7*E*,22*E*)-(1*S*,3*R*)-25-(1-oxopropyl)-26,27-cyclo-9,10-sécocholesta-5,7,10(19),22-tétraène-1,3-diol,
le (5*Z*,7*E*,22*E*)-(1*S*,3*R*)-25-(1-oxobutyl)-26,27-cyclo-9,10-sécocholesta-5,7,10(19),22-tétraène-1,3-diol,
le (5*Z*,7*E*,22*E*)-(1*S*,3*R*)-25-(1-oxopentyl)-26,27-cyclo-9,10-sécocholesta-5,7,10(19),22-tétraène-1,3-diol,
le (5*Z*,7*E*,22*E*)-(1*S*,3*R*)-25-(1-oxohexyl)-26,27-cyclo-9,10-sécocholesta-5,7,10(19),22-tétraène-1,3-diol,
le (5*Z*,7*E*,22*E*)-(1*S*,3*R*)-25-(1-oxoheptyl)-26,27-cyclo-9,10-sécocholesta-5,7,10(19),22-tétraène-1,3-diol,
le (5*Z*,7*E*,22*E*)-(1*S*,3*R*)-25-(1-oxooctyl)-26,27-cyclo-9,10-sécocholesta-5,7,10(19),22-tétraène-1,3-diol,
le (5*Z*,7*E*,22*E*)-(1*S*,3*R*)-25-(1-oxononyl)-26,27-cyclo-9,10-sécocholesta-5,7,10(19),22-tétraène-1,3-diol,
le (5*Z*,7E,22E)-(1*S*,3*R*)-25-(1-oxodécyl)-26,27-cyclo-9,10-sécocholesta-5,7,10(19),22-tétraène-1,3-diol,
le (5*Z*,7E)-(1*S*,3*R*,22*S*)-25-acétyl-26,27-cyclo-9,10-sécocholesta-5,7,10(19)-triène-1,3,22-triol,
le (5*Z*,7E)-(1*S*,3*R*,22*R*)-25-acétyl-26,27-cyclo-9,10-sécocholesta-5,7,10(19)-triène-1,3,22-triol,
le (5*Z*,7E)-(1*S*,3*R*,22*S*)-25-(1-oxopropyl)-26,27-cyclo-9,10-sécocholesta-5,7,10(19)-triène-1,3,22-triol,
le (5*Z*,7*E*)-(1*S*,3*R*,22*R*)-25-(1-oxopropyl)-26,27-cyclo-9,10-sécocholesta-5,7,10(19)-triène-1,3,22-triol,
le (5*Z*,7E)-(1*S*,3*R*,22*S*)-25-(1-oxobutyl)-26,27-cyclo-9,10-sécocholesta-5,7,10(19)-triène-1,3,22-triol,
le (5*Z*,7E)-(1*S*,3*R*,22*R*)-25-(1-oxobutyl)-26,27-cyclo-9,10-sécocholesta-5,7,10(19)-triène-1,3,22-triol,
le (5*Z*,7E)-(1*S*,3*R*,22*S*)-25-(1-oxopentyl)-26,27-cyclo-9,10-sécocholesta-5,7,10(19)-triène-1,3,22-triol,
le (5*Z*,7E)-(1*S*,3*R*,22*R*)-25-(1-oxopentyl)-26,27-cyclo-9,10-sécocholesta-5,7,10(19)-triène-1,3,22-triol,
le (5*Z*,7E)-(1*S*,3*R*,22*S*)-25-(1-oxohexyl)-26,27-cyclo-9,10-sécocholesta-5,7,10(19)-triène-1,3,22-triol,
le (5*Z*,7*E*)-(1*S*,3*R*,22*R*)-25-(1-oxohexyl)-26,27-cyclo-9,10-sécocholesta-5,7,10(19)-triène-1,3,22-triol,
le (5*Z*,7*E*)-(1*S*,3*R*,22*S*)-25-(1-oxoheptyl)-26,27-cyclo-9,10-sécocholesta-5,7,10(19)-triène-1,3,22-triol,
le (5*Z*,7E)-(1*S*,3*R*,22*R*)-25-(1-oxoheptyl)-26,27-cyclo-9,10-sécocholesta-5,7,10(19)-triène-1,3,22-triol,
le (5*Z*,7E)-(1*S*,3*R*,22*S*)-25-(1-oxooctyl)-26,27-cyclo-9,10-sécocholesta-5,7,10(19)-triène-1,3,22-triol,

le (5*Z*,7*E*)-(1*S*,3*R*,22*R*)-25-(1-oxooctyl)-26,27-cyclo-9,10-sécocholesta-5,7,10(19)-triène-1,3,22-triol,
le (5*Z*,7*E*)-(1*S*,3*R*,22*S*)-25-(1-oxononyl)-26,27-cyclo-9,10-sécocholesta-5,7,10(19)-triène-1,3,22-triol,
le (5*Z*,7*E*)-(1*S*,3*R*,22*R*)-25-(1-oxononyl)-26,27-cyclo-9,10-sécocholesta-5,7,10(19)-triène-1,3,22-triol,
le (5*Z*,7*E*)-(1*S*,3*R*,22*S*)-25-(1-oxodécyl)-26,27-cyclo-9,10-sécocholesta-5,7,10(19)-triène-1,3,22-triol,
le (5*Z*,7*E*)-(1*S*,3*R*,22*R*)-25-(1-oxodécyl)-26,27-cyclo-9,10-sécocholesta-5,7,10(19)-triène-1,3,22-triol,
le (5*Z*,7*E*,22*E*)-(1*S*,3*R*)-25-acétyl-26,27-cyclo-24a-homo-9,10-sécocholesta-5,7,10(19),22-tétraène-1,3-diol,
le (5*Z*,7*E*,22*E*)-(1*S*,3*R*)-25-(1-oxopropyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxobutyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxopentyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxohexyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxoheptyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxooctyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxononyl)-26,27-cyclo-24a-homo-9,10-sécocholesta-5,7,10(19),22-tétraène-1,3-diol,
le (5*Z*,7*E*,22*E*)-(1*S*,3*R*)-25-(1-oxodécyl)-26,27-cyclo-24a-homo-9,10-sécocholesta-5,7,10(19),22-tétraène-1,3-diol,
le (5*Z*,7*E*)-(1*S*,3*R*,22*S*)-25-acétyl-26,27-cyclo-24a-homo-9,10-sécocholesta-5,7,10(19)-triène-1,3,22-triol,
le (5*Z*,7*E*)-(1*S*,3*R*,22*R*)-25-acétyl-26,27-cyclo-24a-homo-9,10-sécocholesta-5,7,10(19)-triène-1,3,22-triol,
le (5*Z*,7*E*)-(1*S*,3*R*,22*S*)-25-(1-oxopropyl)-26,27-cyclo-24a-homo-9,10-sécocholesta-5,7,10(19)-triène-1,3,22-triol,
le (5*Z*,7*E*)-(1*S*,3*R*,22*R*)-25- (1-oxopropyl) -26,27-cyclo-24a-homo-9,10-sécocholesta-5,7,10(19)-triène-1,3,22-triol,
le (5*Z*,7*E*)-(1*S*,3*R*,22*S*)-25-(1-oxobutyl)-26,27-cyclo-24a-homo-9,10-sécocholesta-5,7,10(19)-triène-1,3,22-triol,
le (5*Z*,7*E*)-(1*S*,3*R*,22*R*)-25-(1-oxobutyl)-26,27-cyclo-24a-homo-9,10-sécocholesta-5,7,10(19)-triène-1,3,22-triol,
le (5*Z*,7*E*)-(1*S*,3*R*,22*S*)-25-(1-oxopentyl)-26,27-cyclo-24a-homo-9,10-sécocholesta-5,7,10(19)-triène-1,3,22-triol,
le (5*Z*,7*E*)-(1*S*,3*R*,22*R*)-25-(1-oxopentyl)-26,27-cyclo-24a-homo-9,10-sécocholesta-5,7,10(19)-triène-1,3,22-triol,
le (5*Z*,7*E*)-(1*S*,3*R*,22*S*)-25-(1-oxohexyl)-26,27-cyclo-24a-homo-9,10-sécocholesta-5,7,10(19)-triène-1,3,22-triol,
le (5*Z*,7*E*)-(1*S*,3*R*,22*R*)-25-(1-oxohexyl)-26,27-cyclo-24a-homo-9,10-sécocholesta-5,7,10(19)-triène-1,3,22-triol,
le (5*Z*,7*E*)-(1*S*,3*R*,22*S*)-25- (1-oxoheptyl) -26,27-cyclo-24a-homo-9,10-sécocholesta-5,7,10(19)-triène-1,3,22-triol,
le (5*Z*,7*E*)-(1*S*,3*R*,22*R*)-25-(1-oxoheptyl)-26,27-cyclo-24a-homo-9,10-sécocholesta-5,7,10(19)-triène-1,3,22-triol,
le (5*Z*,7*E*)-(1*S*,3*R*,22*S*)-25-(1-oxooctyl)-26,27-cyclo-24a-homo-9,10-sécocholesta-5,7,10(19)-triène-1,3,22-triol,
le (5*Z*,7*E*)-(1*S*,3*R*,22*R*)-25-(1-oxooctyl)-26,27-cyclo-24a-homo-9,10-sécocholesta-5,7,10(19)-triène-1,3,22-triol,
le (5Z,7E)-(1*S*,3*R*,22*S*)-25-(1-oxononyl)-26,27-cyclo-24a-homo-9,10-sécocholesta-5,7,10(19)-triène-1,3,22-triol,
le (5Z,7E)-(1*S*,3*R*,22*R*)-25-(1-oxononyl)-26,27-cyclo-24a-homo-9,10-sécocholesta-5,7,10(19)-triène-1,3,22-triol,
le (5Z,7E)-(1*S*,3*R*,22*S*)-25-(1-oxodécyl)-26,27-cyclo-24a-homo-9,10-sécocholesta-5,7,10(19)-triène-1,3,22-triol,
le (5Z,7E)-(1*S*,3*R*,22*R*)-25-(1-oxodécyl)-26,27-cyclo-24a-homo-9,10-sécocholesta-5,7,10(19)-triène-1,3,22-triol,
le (5Z,7E,22E)-(1*S*,3*R*,24*R*)-25-acétyl-26,27-cyclo-24a,24b-dihomo-9,10-sécocholesta-5,7,10(19),22-tétraène-1,3,24-triol,

le (5Z,7E,22E) - (1*S*,3*R*,24*S*) -25-acétyl-26,27-cyclo-24a,24b-dihomo-9,10-sécocholesta-5,7,10(19),22-tétraène-1,3,24-triol,
le (5Z,7E,22E)-(1*S*,3*R*,24*R*)-25-(1-oxopropyl)-26,27-cyclo-24a,24b-dihomo-9,10-sécocholesta-5,7,10(19), 22-tétraène-1,3,24-triol,
le (5*Z*,7*E*,22*E*)-(1*S*,3*R*,24*S*)-25-(1-oxopropyl)-26,27-cyclo-24a,24b-dihomo-9,10-sécocholesta-5,7,10(19), 22-tétraène-1,3,24-triol,
le (5*Z*,7*E*,22*E*)-(1*S*,3*R*,24*R*)-25-(1-oxobutyl)-26,27-cyclo-24a,24b-dihomo-9,10-sécocholesta-5,7,10(19),22-tétraène-1,3,24-triol,
le (5Z,7E,22E)-(1*S*,3*R*,24*S*)-25-(1-oxobutyl)-26,27-cyclo-24a,24b-dihomo-9,10-sécocholesta-5,7,10 (19), 22-tétraène-1,3,24-triol,
le (5Z,7E,22E)-(1*S*,3*R*,24*R*)-25-(1-oxopentyl)-26,27-cyclo-24a,24b-dihomo-9,10-sécocholesta-5,7,10(19), 22-tétraène-1,3,24-triol,
le (5Z,7E,22E)-(1*S*,3*R*,24*S*)-25-(1-oxopentyl)-26,27-cyclo-24a,24b-dihomo-9,10-sécocholesta-5,7,10(19), 22-tétraène-1,3,24-triol,
le (5Z,7E,22E)-(1*S*,3*R*,24*R*)-25-(1-oxohexyl)-26,27-cyclo-24a,24b-dihomo-9,10-sécocholesta-5,7,10(19), 22-tétraène-1,3,24-triol,
le (5Z,7E,22E)-(1*S*,3*R*,24*S*)-25-(1-oxohexyl)-26,27-cyclo-24a,24b-dihomo-9,10-sécocholesta-5,7,10(19), 22-tétraène-1,3,24-triol,
le (5Z,7E,22E)-(1*S*,3*R*,24*R*)-25-(1-oxoheptyl)-26,27-cyclo-24a,24b-dihomo-9,10-sécocholesta-5,7,10(19), 22-tétraène-1,3,24-triol,
le (5Z,7E,22E)-(1*S*,3*R*,24*S*)-25-(1-oxoheptyl)-26,27-cyclo-24a,24b-dihomo-9,10-sécocholesta-5,7,10(19), 22-tétraène-1,3,24-triol,
le (5Z,7E,22E)-(1*S*,3*R*,24*R*)-25-(1-oxooctyl)-26,27-cyclo-24a,24b-dihomo-9,10-sécocholesta-5,7,10(19), 22-tétraène-1,3,24-triol,
le (5Z,7E,22E)-(1*S*,3*R*,24*S*)-25-(1-oxooctyl)-26,27-cyclo-24a,24b-dihomo-9,10-sécocholesta-5,7,10(19), 22-tétraène-1,3,24-triol,
le (5Z,7*E*,22*E*)-(1*S*,3*R*,24*R*)-25-(1-oxononyl)-26,27-cyclo-24a,24b-dihomo-9,10-sécocholesta-5,7,10(19), 22-tétraêne-1,3,24-triol,
le (5*Z*,7*E*,22*E*)-(1*S*,3*R*,24*S*)-25-(1-oxononyl)-26,27-cyclo-24a,24b-dihomo-9,10-sécocholesta-5,7,10(19), 22-tétraène-1,3,24-triol,
le (5Z,7E,22E)-(1*S*,3*R*,24*R*)-25-(1-oxodécyl)-26,27-cyclo-24a,24b-dihomo-9,10-sécocholesta-5,7,10(19), 22-tétraène-1,3,24-triol,
le (5Z,7E,22E)-(1*S*,3*R*,24*S*)-25-(1-oxodécyl)-26,27-cyclo-24a,24b-dihomo-9,10-sécocholesta-5,7,10(19), 22-tétraène-1,3,24-triol,
le (5Z,7E,22E)-(1*S*,3*R*,24*R*)-25-acétyl-24-méthoxy-26,27-cyclo-24a,24b-dihomo-9,10-sécocholesta-5,7,10 (19),22-tétraène-1,3-diol,
le (5Z,7E,22E)-(1*S*,3*R*,24*S*)-25-acétyl-24-méthoxy-26,27-cyclo-24a,24b-dihomo-9,10-sécocholesta-5,7,10 (19),22-tétraène-1,3-diol,
le (5Z,7E,22E)-(1*S*,3*R*,24*R*)-24-méthoxy-25-(1-oxopropyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxopropyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxobutyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxobutyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxopentyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxopentyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxohexyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxohexyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxoheptyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxoheptyl)-26,27-cyclo-24a,24b-dihomo-9,10-sécocholesta-5,7,10(19),22-tétraène-1,3-diol,

le (5Z,7E,22E)-(1*S*,3*R*,24*R*)-24-méthoxy-25- (1-oxooctyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxooctyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxononyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxononyl)-26,27-cyclo-24a,24b-dihomo-9,10-sécocholesta-5,7,10(19),22-tétraène-1,3-diol,
le (5Z,7E,22E)-(1*S*,3*R*,24*R*)-24-méthoxy-25-(1-oxodécyl)-26,27-cyclo-24a,24b-dihomo-9,10-sécocholesta-5,7,10(19),22-tétraène-1,3-diol,
le (5Z,7E,22E)-(1*S*,3*R*,24*S*)-24-méthoxy-25-(1-oxodécyl)-26,27-cyclo-24a,24b-dihomo-9,10-sécocholesta-5,7,10(19),22-tétraène-1,3-diol.

**5.** Procédé de préparation des dérivés de la vitamine D de formule générale I, dans lequel un composé de formule générale II

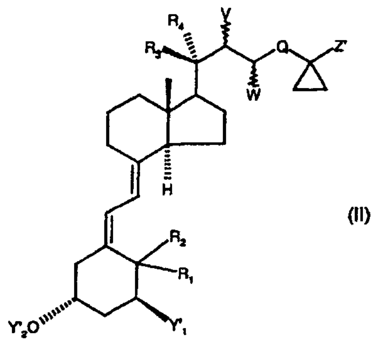

(II)

dans laquelle $Y'_1$ représente un atome d'hydrogène, un atome d'halogène ou un groupe hydroxy protégé et $Y'_2$ représente un groupe protecteur d'hydroxy, est transformé en le composé de formule générale I par clivage simultané ou successif des groupes protecteurs d'hydroxy et de céto et éventuellement par estérification partielle ou totale des groupes hydroxy libres.

**6.** Utilisation des dérivés de la vitamine D de formule générale I pour la fabrication de médicaments.

**7.** Utilisation des dérivés de la vitamine D de formule générale I selon la revendication 6 pour la fabrication de médicaments destinés à la thérapie de maladies hyperprolifératives de la peau, du prurit, de maladies tumorales, de précancéroses, de troubles du système immunitaire, de maladies inflammatoires, de la polyarthrite rhumatoïde, de l'asthme, de maladies auto-immunes, de la sclérose en plaques, du diabète sucré, du SIDA ainsi que de réactions de rejet dans le cas de graisses autologues, hallogéniques ou xénogéniques.

**8.** Utilisation selon la revendication 6, **caractérisée en ce que** le médicament est en outre combiné avec d'autres substances actives immunosuppressives telles que la cyclosporine A, FK 506, la rapamycine et des anticorps anti-CD 4.

**9.** Utilisation des dérivés de la vitamine D de formule générale I selon la revendication 6 pour la fabrication de médicaments destinés à la thérapie de cicatrisation de la peau atrophique ou de blessures, la thérapie de l'hyperparathyroïdisme secondaire, de l'ostéodystrophie rénale ainsi que de l'ostéoporose sénile et post-ménopausal, du diabète sucré de type II et à la thérapie de maladies dégénératives du système nerveux périphérique et central ainsi que la régulation de la pousse des cheveux.

**10.** Utilisation de dérivés de la vitamine D de formule générale I selon la revendication 6, qui antagonisent l'action du calcitriol dans des cellules HL 60, pour la thérapie d'hypercalcémies ou de maladies granulomateuses, d'hypercalcémies paranéoplastiques, d'une hypercalcémie dans le cas de l'hyper-parathyroïdisme, pour la contraception masculine et féminine ou en tant qu'immunostimulants, également dans le cas de l'hirsutisme, pour la thérapie et la prophylaxie de l'artériosclérose et pour la thérapie de maladies inflammatoires.

**11.** Intermédiaires de formule générale XII dans le cadre de la préparation des dérivés de la vitamine D de formule générale I selon la revendication 1 :

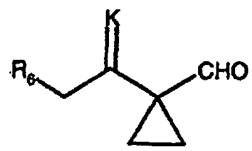

dans laquelle
$R_6$ représente un groupe alkyle linéaire ou ramifié ayant jusqu'à 11 atomes de carbone et K représente un groupe protecteur de céto.

**12.** Intermédiaire selon la revendication 11, **caractérisé en ce que** K représente un 1,3-dioxolane, un 1,3-dioxane, un 5,5-diméthyl-1,3-dioxane ou un dialcoxycétal.